# FASCICULE DE BREVET EUROPEEN

(11) **EP 2 976 330 B1**
(45) Date de publication et mention de la délivrance du brevet: **09.02.2022**
(21) Numéro de dépôt: 14710593.6
(22) Date de dépôt: 18.03.2014
(51) Int. Cl.: C07D 233/58, C07D 403/04, G01T 1/02

(54) **NOUVELLE FAMILLE DE MOLECULES DISCRIMINANTES POUR LES RAYONNEMENTS NEUTRON ET GAMMA ET LIQUIDES IONIQUES**
NEUE FAMILIE VON MOLEKÜLEN ZUR UNTERSCHEIDUNG VON NEUTRONEN UND GAMMASTRAHLUNGEN UND IONISCHEN FLÜSSIGKEITEN
NEW FAMILY OF DISCRIMINATING MOLECULES FOR NEUTRON AND GAMMA RAYS AND IONIC LIQUIDS

(30) Priorité: 18.03.2013 FR 1352400
(43) Date de publication de la demande: 27.01.2016
(73) Titulaire: Centre National de la Recherche Scientifique (C.N.R.S.), 75016 Paris (FR); Université de Strasbourg, 67000 Strasbourg (FR)
(72) Inventeur: DOUCE, Laurent, F-67100 Strasbourg (FR); STUTTGE, Louise, F-67000 Strasbourg (FR); BOUAJILA, Ezeddine, F-69100 Villeurbanne (FR); FOUCHET, Julien, F-67100 Strasbourg (FR); HEINRICH, Benoît, F-67100 Strasbourg (FR)
(74) Mandataire: Lavoix
(86) Numéro de dépôt international: PCT/EP2014/055428
(87) Numéro de publication internationale: WO 2014/147078

(56) Documents cités:
- WO-A2-2010/004228
- US-A1- 2006 024 522
- US-A1- 2011 105 761
- DIRK MEYER ET AL: "1,2,4-Triazole-Based Tunable Aryl/Alkyl Ionic Liquids", THE JOURNAL OF ORGANIC CHEMISTRY, vol. 76, no. 1, 7 janvier 2011 (2011-01-07), pages 305-308, XP055084341, ISSN: 0022-3263, DOI: 10.1021/jo101784v
- XIAOHONG CHENG ET AL: "Self-Assembly of Imidazolium-Based Rodlike Ionic Liquid Crystals: Transition from Lamellar to Micellar Organization", CHEMISTRY - A EUROPEAN JOURNAL, vol. 16, no. 15, 19 avril 2010 (2010-04-19), pages 4588-4601, XP055084345, ISSN: 0947-6539, DOI: 10.1002/chem.200903210
- ENRIQUE DIEZ-BARRA ET AL: "Reactivity of tetracyanoethylene oxide toward heteroaromatic compounds. Synthesis and structure of heterocyclic dicyanomethylides", THE JOURNAL OF ORGANIC CHEMISTRY, vol. 47, no. 23, 1 novembre 1982 (1982-11-01), pages 4409-4412, XP055084353, ISSN: 0022-3263, DOI: 10.1021/jo00144a004
- LIJUAN SHI ET AL: "Aggregation Behavior of Long-Chain N -Aryl Imidazolium Bromide in Aqueous Solution", LANGMUIR, vol. 27, no. 5, 1 mars 2011 (2011-03-01), pages 1618-1625, XP055084359, ISSN: 0743-7463, DOI: 10.1021/la104719v
- HIROTO YOSHIDA ET AL: "Facile Synthesis of N -Alkyl- N ' -arylimidazolium Salts via Addition of Imidazoles to Arynes", ORGANIC LETTERS, vol. 4, no. 16, 1 août 2002 (2002-08-01), pages 2767-2769, XP055084386, ISSN: 1523-7060, DOI: 10.1021/ol0262845
- G. MICHAEL BLACKBURN ET AL: "Bonding of benzo[a]pyrene to nitrogen heterocycles by anodic oxidation", JOURNAL OF THE CHEMICAL SOCIETY, CHEMICAL COMMUNICATIONS, no. 2, 1 janvier 1974 (1974-01-01), page 67, XP055084390, ISSN: 0022-4936, DOI: 10.1039/c39740000067
- BJÖRN WIEGMANN ET AL: "A New Framework of a Heteroleptic Iridium(III)-Carbene Complex as a Triplet Emitting Material", ORGANOMETALLICS, vol. 31, no. 15, 13 août 2012 (2012-08-13) , pages 5223-5226, XP055084394, ISSN: 0276-7333, DOI: 10.1021/om300458f
- MICHAEL BLACKBURN G ET AL: "Intramolecular fluorescence quenching of anthracene by heterocyclic ligands", JOURNAL OF THE CHEMICAL SOCIETY, PERKIN TRANSACTIONS 2, CHEMICAL SOCIETY. LETCHWORTH, GB, 1 janvier 1976 (1976-01-01), pages 1452-1456, XP008119029, ISSN: 1472-779X
- ANUPAM MIDYA ET AL: "A new class of solid state ionic conductors for application in all solid state dye sensitized solar cells", CHEMICAL COMMUNICATIONS, vol. 46, no. 12, 1 janvier 2010 (2010-01-01), page 2091, XP055084412, ISSN: 1359-7345, DOI: 10.1039/B924497B

## Description

La présente invention concerne une nouvelle famille de molécules discriminantes pour les rayonnements neutron et gamma notamment et leur procédé de préparation. Ces molécules peuvent notamment être utilisées pour la détection des rayonnements nucléaires, en recherche fondamentale ou bien dans les domaines de la dosimétrie et de l'imagerie médicale.

Comme l'explique la demande WO 2010/004228 (US 2011-0303850, EP 09784508), la production de paires électron-trou par ionisation constitue la première étape, l'une des plus rapides, de la relaxation de l'énergie initialement déposée par un rayonnement dans un milieu. Dans la matière organique excitée, les paires formées sont généralement constituées d'un électron quasi-libre dans la bande de conduction, géminé (électriquement apparié) à un trou beaucoup moins mobile dans la bande de valence.

La discrimination repose sur le fait que la quantité d'énergie, dE, déposée dans un milieu par unité de distance parcourue, dx, par un rayonnement incident, est différente selon que ce rayonnement soit formé de photons ou bien de particules chargées. Dans le cas d'une excitation par des photons gammas issus, par exemple, d'une source radioactive, la quantité dE/dx est relativement faible, ce qui implique de faibles densités d'ionisation, et donc une production de paires électron-trou, relativement faible. Dans le cas d'une excitation par des neutrons, il se produit différentes réactions nucléaires, dont la plus importante est la réaction (n,p) au cours de laquelle un proton (p) est éjecté d'un noyau par collision élastique avec un neutron (n) incident. Ce proton va naturellement ralentir dans le milieu par excitation et ionisation de ce dernier, produisant des paires électron-trou géminées de même nature que celles produites avec un rayonnement gamma, mais avec une densité d'ionisation bien plus forte.

La recombinaison différée des paires produites étant à l'origine de l'émission de fluorescence, cette dernière sera plus intense lors d'une excitation par des neutrons (indirectement par des protons) que par des photons gamma. La comparaison des intensités de fluorescence émises lors des deux types d'excitation, photons et neutrons, permet donc la discrimination.

La discrimination des rayonnements neutron et gamma est généralement réalisée selon trois types de méthodes :
- par mesure de temps de vol des particules gamma et des neutrons ;
- par l'utilisation de détecteurs à base de semi-conducteurs ; ou
- par l'utilisation de molécules scintillantes diluées au sein de matériaux plastiques transparents.

La méthode qui consiste à faire des mesures de temps de vol est particulièrement complexe de mise en œuvre sur les plans technique et opératoire, notamment en raison de la difficulté d'accéder à une bonne référence temporelle. Elle nécessite, en effet, une technicité importante, tant dans la fabrication matérielle de l'expérience que dans sa conduite. Cette technique, qui donne cependant des résultats très précis, reste essentiellement dédiée aux laboratoires de recherche et aux applications en milieu peu hostile.

La méthode qui consiste à utiliser comme partie active d'un détecteur un matériau semi-conducteur reste limitée aux fortes fluences. Dans ce domaine d'application, la principale limitation à l'utilisation de matériaux conducteurs réside toujours dans le fait que la mesure se fait sur la quantité de charges produites ou bien le courant généré. Dans tous les cas, les mesures restent peu sensibles car la limite de détection d'un courant se situe aux environs du picoampère et ne correspond donc qu'à la production d'environ 10 millions de porteurs de charges mobiles chaque seconde. On compense généralement la faible sensibilité de cette méthode par l'augmentation du volume sensible du détecteur, mais se posent alors de nouveaux problèmes, liés à la diminution globale de la conduction de l'ensemble ainsi qu'à la dégradation de la réponse temporelle du détecteur.

Les mesures basées sur la scintillation de molécules diluées sont de loin les plus utilisées.

Ainsi, lorsque le milieu irradié contient des molécules luminescentes, la recombinaison géminée des paires électron-trou résultant de l'ionisation primaire conduit, avec un rendement défini, à une émission de fluorescence différée dont l'intensité sera proportionnelle à la densité des ionisations produites par le rayonnement sur son parcours. Cette intensité est plus élevée lors du passage d'un proton que lors du passage d'un photon en raison d'une perte d'énergie par unité de parcours, dE/dx, plus importante. Ainsi, par une mesure de fluorescence différée, on peut discriminer entre la fluorescence résultant du passage d'un proton [produit par un neutron lors d'une réaction (n,p)] et celle résultant du passage d'un photon. La comparaison des intensités des deux signaux de fluorescence émis permet d'effectuer la discrimination. La difficulté de la mesure résulte essentiellement dans le fait que la fluorescence n'est émise que durant un temps très court, de quelques centaines de nanosecondes, après le passage du rayonnement primaire, ce qui implique une instrumentation spécifique, fonctionnant avec une résolution temporelle nanoseconde. L'intensité de fluorescence étant globalement faible, on ne peut la détecter qu'à l'aide de photomultiplicateurs. Le spectre d'émission de la fluorescence est indépendant de la nature de l'excitation et ne dépend que de la nature du fluorophore choisi. La sensibilité de détection est bien supérieure à celle que l'on peut attendre d'une mesure de courant car il suffit que quelques milliers porteurs de charges libres soient produits dans les milieux pour que la détection devienne possible.

Les molécules scintillantes diluées sont généralement des oxazoles ou des oxadiazoles, voire du para-terphényle ou de l'anthracène. Toutes ces molécules ont des propriétés de luminescence nanoseconde dans un domaine spectral s'étendant de 300 à 400 nm.

Cette méthode, basée sur la mesure de fluorescence de recombinaison, après interception des charges produites dans le milieu par les molécules scintillantes, est de loin la plus rapide (nanoseconde) et la plus sensible. En théorie, la production d'une seule charge libre dans le milieu suffit à produire une fluorescence détectable et donc une mesure. La méthode serait donc 10 millions fois plus sensible que celle basée sur la conduction. Mais, dans la pratique actuelle, le fait que la limite de dilution d'une molécule scintillante dans un plastique se situe vers 0.01 mole/L, réduit d'un facteur 10 à 100 la sensibilité. L'autre facteur de réduction de la sensibilité est donné par l'angle solide de détection de la lumière. On peut l'estimer de 100 à 1000. L'utilisation d'un photomultiplicateur ayant un rendement de détection typique de 20 % réduit donc la sensibilité d'un facteur 5 environ. Globalement, on peut estimer que les techniques de fluorescence sont environ 1000 fois plus sensibles que les techniques électriques et que la principale limitation provient de la dilution des molécules fluorescentes. L'utilisation de matériaux fluorescents aromatiques dilués dans un liquide (benzène, xylène) ou utilisés soit à l'état solide pur (p-terphényle, anthracène) est possible.

Les scintillateurs actuellement utilisés pour la détection des rayonnements neutrons sont essentiellement de trois types :
- Scintillateurs liquides (NE213, BC501, BC501A) : mélanges de xylène et de naphtalène auxquels des additifs sont ajoutés.
- Scintillateurs cristallins : cristaux organiques et inorganiques (anthracène, stilbène, fluorophores dopés au cérium) (voir WO 20152/142365). Ce type de scintillateur est difficile et coûteux à produire.
- Plastiques scintillants. Ces scintillateurs sont peu coûteux à fabriquer.

Les scintillateurs liquides et solides permettent la détection et la discrimination des neutrons et des rayons gamma alors que les plastiques scintillants peuvent uniquement détecter les deux rayonnements sans les discriminer sauf par la technique du temps de vol. Par ailleurs ces derniers peuvent se déformer et s'opacifier avec le temps (la détection et la discrimination donnent de meilleurs résultats dans un matériau transparent). Cependant tous ces scintillateurs présentent un problème majeur au niveau de la sécurité : ils sont inflammables voire explosifs dans le cas des liquides, toxiques et nocifs pour l'environnement. L'enjeu est donc de proposer des matériaux, permettant de discriminer les neutrons et les rayons gamma, qui satisfassent les normes d'hygiène et de sécurité afin d'envisager leur utilisation dans des endroits tels que les centrales nucléaires, les hôpitaux, les aéroports, ... où de tels scintillateurs sont proscrits actuellement. De plus, les nouveaux scintillateurs doivent pouvoir être placés sous vide, pour éviter que l'air interfère avec certains rayonnements, ce qui n'est pas envisageable aujourd'hui.

La demande WO 2010/004228 décrit une méthode permettant de préparer des matériaux solides, stables thermiquement (200 °C) et sans tension de vapeur mesurable, pouvant discriminer les rayonnements neutrons et gamma. Des oxazole/imidazolium ont été synthétisés et donnent de bons résultats en discrimination. Cependant leur synthèse requiert sept à huit étapes et leur transparence est difficile à obtenir.

### Buts de l'invention

Ainsi l'invention a pour but de résoudre les problèmes techniques cités ci-dessus, tout en procurant des composés simples à synthétiser.

L'invention a aussi pour but de fournir de nouvelles molécules permettant d'améliorer la détection des rayonnements gamma, X, neutron et/ou proton.

La présente invention a également pour but de fournir de nouvelles molécules permettant d'améliorer la discrimination des rayonnements proton/gamma, proton/X, neutron/gamma, neutron/X, alpha/gamma, alpha/X.

La présente invention a encore pour but de fournir des instruments de radiodétection, de dosimétrie industrielle ou médicale.

La présente invention a notamment pour but de simplifier la préparation des composés synthétisés précédemment selon la méthode décrite dans la demande WO 2010/004228.

### Description de l'invention

Les inventeurs ont découvert une nouvelle famille de molécules (ou composés) discriminantes. La méthode de synthèse permet d'obtenir des imidazolium en deux ou trois étapes à partir des produits commerciaux. Cette méthode de synthèse est universelle car un grand nombre d'aryles ou groupes aromatiques peuvent être couplés.

Par ailleurs les propriétés de ces nouvelles molécules sont encore meilleures du point de vue de la détection et/ou de la discrimination des rayons précités que celles décrites dans le brevet WO 2010/004228.

Cette nouvelle famille de molécules fluorescentes permet notamment la discrimination en temps réel des rayonnements neutron et gamma, de manière améliorée.

Contre toute attente, les composés ainsi obtenus présentent d'une part, des propriétés de fluorescence rapides (nanoseconde) et d'émission dans l'ultraviolet (400 nm), et d'autre part les propriétés des liquides ioniques. Lorsque ces molécules possèdent au moins un groupe fluorophore, cela leur confère des rendements de fluorescence importants, semblables aux scintillateurs liquides, mais avec de nombreuses propriétés supplémentaires :
- tension de vapeur non mesurable permettant une utilisation sous vide poussé ;
- bonne stabilité thermique (jusqu'à 200°C) ;
- caractère ininflammable ;
- faible toxicité ;
- état liquide ou solide.

Outre ces propriétés, la réalisation de molécules à façon permet d'ajuster l'efficacité d'interaction initiale avec le rayonnement afin d'augmenter la perte d'énergie (dE/dx) et donc la quantité de lumière émise. Ceci peut être obtenu, par exemple, par l'adjonction d'un atome lourd (détection des photons), ou par l'augmentation du nombre d'atomes d'hydrogène (détection des neutrons). Une meilleure sensibilité de détection est obtenue, en milieux purs et dilués, dans la mesure où l'on contrôle mieux la construction de la molécule et les paramètres d'interaction. Les molécules luminescentes de l'invention peuvent donc être utilisées dans des scintillateurs.

Selon la présente invention, le terme « fluorophore » désigne un groupe fluorescent, capable d'absorber de l'énergie à une longueur d'onde spécifique et de réémettre de l'énergie à une longueur d'onde différente mais également spécifique. La quantité et la longueur d'onde de l'énergie réémise dépendent à la fois du fluorophore et de l'environnement chimique du fluorophore. L'invention est définie selon les revendications.

Les inventeurs décrivent un composé imidazolium de formule (I) : 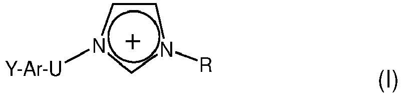 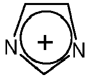 représente le cation imidazolium,
« Y-Ar-U » est un groupe comprenant au moins un atome de carbone sp³ ;
« Y » nécessairement présent, représente un groupe « alkyle » ou O-« alkyle », « akyle » étant défini comme un radical hydrocarboné saturé, comprenant éventuellement une ou plusieurs insaturations, en chaîne linéaire ou ramifié, de 1 à 30 atomes de carbone, de préférence de 5 à 20 atomes de carbone, éventuellement substitué, ;
« Ar » est éventuellement présent et défini comme un groupe aromatique, éventuellement substitué,
« U » est éventuellement présent et défini comme un radical hydrocarboné saturé, comprenant éventuellement une ou plusieurs insaturations, en chaîne linéaire ou ramifié, de 1 à 30 atomes de carbone, de préférence de 5 à 20 atomes de carbone, éventuellement substitué ;
« R » représentant un groupe aromatique fluorophore, comprenant au moins un atome de carbone sp², éventuellement substitué,
dans lequel le groupe imidazolium est lié directement au groupe aromatique « R » par une liaison covalente entre un atome d'azote du groupe imidazolium et un atome de carbone sp² du groupe aromatique,
et dans lequel le groupe imidazolium est lié directement au substituant « Y-Ar-U- » par une liaison covalente entre l'autre atome d'azote du noyau imidazolium et un atome de carbone sp³ dudit groupe « Y-Ar-U- ».

Les inventeurs ont notamment découvert que les molécules de l'invention définies selon la revendication 1 présentent des propriétés de luminescence. En présence d'un groupe « R » luminescent ces composés permettent une application très avantageuse en détection et discrimination de rayonnements, notamment neutrons et gamma.

Le groupe « R » est un groupe aromatique fluorophore, absorbant de l'énergie à une longueur d'onde spécifique et réémettant de l'énergie à une longueur d'onde différente mais également spécifique. Le groupe « R » est fluorophore avant son couplage avec la partie imidazolium/imidazole des composés de l'invention.

Avantageusement, le groupe « R » comprend au moins un groupe ou consiste d'un groupe choisi parmi : phényle, éventuellement substitué, naphtalène, éventuellement substitué, fluorène, éventuellement substitué, méthylcarbazole, éventuellement substitué, et anthracène, éventuellement substitué, pyrène, éventuellement substitué, tétracène, éventuellement substitué, une protéine fluorescente, par exemple GFP (vert), YFP (jaune) et RFP (rouge), des dérivés de xanthene comme : fluorescéine, rhodamine, Oregon green, éosine, et chlorure d'acide de sulforhodamine 101 (Texas red) ; la cyanine ou ses dérivés comme: indocarbocyanine, oxacarbocyanine, thiacarbocyanine, et merocyanine ; des dérivés de naphtalène comme : chlorure de 5-(dimethylamino)naphthalene-1-sulfonyl (dansyl) et 1-[6-(Dimethylamino)-2-naphthalenyl]-1-propanone (prodan), un dérivé de coumarine, un dérivé d'oxadiazole, comme : pyridyloxazole, nitrobenzoxadiazole et benzoxadiazole, un dérivé de pyrène, comme : cascade blue^{®}, un dérivé d'oxazine comme : 9-diethylamino-5-benzo[α]phenoxazinone (Nile red), un dérivé de benzophenoxazine, notamment de un dérivé de benzo[a]phenoxazine (Nile blue), un dérivé de benzo[b]phenoxazine, (9-dimethylamino-10-methyl-benzo[a]phenoxazin-5-ylidene)ammonium chloride (cresyl violet), 5,9-Bis(ethylamino)-10-methylbenzo[a]phenoxazin-7-ium (oxazine 170) ; un dérivé d'acridin, comme : proflavin, N,N,N',N'-Tetramethylacridine-3,6-diamine (acridine orange), 3,6-diamino-2,7-dimethylacridine (acridine yellow) ; un dérivé d'arylmethin, comme : auramine, chlorure de Tris(4-(dimethylamino)phenyl)methylium (crystal violet), 4-[(4-dimethylaminophenyl)phenyl-methyl]-N,N-dimethylaniline (malachite green) ; un dérivé de tétrapyrrol, comme : porphine, phtalocyanine, bilirubin, un 4,4-difluoro-4-bora-3a,4a-diaza-s-indacene (BODIPY), BODIPY FI, BODIPY TMR, BODIPY 581/591, BODIPY 630/650, BODIPY 650/665. Selon une variante, « R » est un groupe polyaromatique, notamment un groupe polyaromatique hydrocarboné.

Tels qu'on les utilise dans toute la description de l'invention, les termes suivants, sauf mention contraire, doivent être compris comme ayant les significations suivantes.

Le terme « halogénoalkyle » désigne un radical alkyle substitué par un ou plusieurs atomes d'halogène. Les radicaux halogénoalkyle incluent les radicaux perhalogénoalkyle et notamment les radicaux perfluoroalkyle de formule CnF₂ₙ₊₁.

Le terme « halogène » désigne un atome de chlore, brome, iode ou fluor.

Le terme « cycloalkyle » signifie un système de cycle non aromatique mono- ou multicyclique de 3 à 10 atomes de carbone, de préférence de 5 à 7 atomes de carbone. A titre d'exemple de cycloalkyle monocyclique, on peut citer notamment le cyclopentyle, le cyclohexyle, le cycloheptyle, et similaires. A titre d'exemple de groupe cycloalkyle multicyclique, on peut citer notamment la 1-décaline, le norbornyle, ou l'adamant-(1 ou 2-)yle.

Le terme « alcényle » désigne un groupe hydrocarboné aliphatique qui contient une double liaison carbone-carbone et qui peut être linéaire ou ramifié ayant de 2 à 6 atomes de carbone dans la chaîne. Ramifié signifie qu'un ou plusieurs groupes alkyle inférieurs, tels que le méthyle, l'éthyle ou le propyle, sont liés à une chaîne alcényle linéaire. Comme exemple de groupe alcényle, on peut citer notamment l'éthényle, le propényle, le *n-*butényle, l'*i*-butényle, le 3-méthylbut-2-ényle, ou le *n*-pentényle.

Le terme « alcynyle » désigne un groupe hydrocarboné aliphatique qui contient une triple liaison carbone-carbone et qui peut être linéaire ou ramifié ayant 2 à 6 atomes de carbone dans la chaîne, de préférence 2 à 4 atomes de carbone. Ramifié signifie qu'un ou plusieurs groupes alkyle inférieurs, tels que le méthyle, l'éthyle ou le propyle, sont liés à une chaîne alcynyle linéaire. Comme exemple de groupes alcynyles, on peut citer notamment l'éthynyle, le propynyle, le *n*-butynyle, le 2-butynyle, le 3-méthylbutynyle, et le *n*-pentynyle.

Le terme « aryle » désigne un système cyclique monocyclique ou multicyclique aromatique de 5 à 20 atomes de carbone. Comme exemple de groupes aryle, on peut citer notamment le phényle, éventuellement substitué, naphtalène, éventuellement substitué, fluorène, éventuellement substitué, méthylcarbazole, éventuellement substitué, et anthracène, éventuellement substitué, pyrène, éventuellement substitué, tétracène, éventuellement substitué, bodipy, thiophène et polythiophene.

Le terme « substitué » désigne une substitution d'un atome d'hydrogène par un atome d'halogène ou par un groupe alkyle, halogénoalkyle, cycloalkyle, alcényle, alcynyle, aryle, un groupe hétérocyclique, comme un hétérocycloalkyle, un hétéroaryle.

Le terme « aralkyle » désigne un groupe aryl-alkyl-, dans lequel l'aryle et l'alkyle sont tels que décrits dans le présent document. Comme exemple de groupes aralkyle, on peut citer notamment le benzyle, le 2-phénéthyle et le naphtlèneméthyle.

Le terme « groupe hétérocyclique » désigne un groupe carbocyclique substitué ou non substitué, mono- ou multicyclique dans lequel la partie cyclique comprend au moins un hétéroatome tel que O, N, S ou B. L'azote et le soufre peuvent être éventuellement oxydés, et l'azote peut être éventuellement substitué dans les cycles aromatiques. Les groupes hétérocycliques comprennent les groupes hétéroaryles et les groupes hétérocycloalkyles.

Le terme « hétérocycloalkyle » désigne un groupe cycloalkyle dans lequel un ou plusieurs atomes de carbone de cycle sont substitués par au moins un atome choisi parmi O, N, ou S. A titre d'exemple de groupe hétérocycloalkyle, on peut citer notamment les groupes pyrrolidinyle, pyrrolinyle, imidazolidinyle imidazolinyle, pirazolidinyle, pirazolinyle, pyrazalinyle, pipéridyle, pipérazinyle, morpholinyle, thiomorpholinyle, tétrahydrofuranyle, dithiolyle, oxathiolyle, oxadiazolyle, oxathiazolyle, pyranyle, oxazinyle, oxathiazinyle, et oxadiazinyle.

Le terme « hétéroaryle » ou « hétéroaromatique » désigne un groupe contenant de 5 à 10 atomes de carbone dans lequel au moins un carbone de cycle est remplacé par au moins un atome choisi parmi -O-, -N-, -S-, ou B. Comme exemple de groupe hétéroaryle, on peut citer notamment pyrrolyle, furanyle, thiényle, pirazolyle, imidazolyle, thiazolyle, isothiazolyle, isoxazolyle, oxazolyle, oxathiolyle, oxadiazolyle, triazolyle, oxatriazolyle, furazanyle, tétrazolyle, pyridyle, pyrazinyle, pyrimidinyle, pyridazinyle, triazinyle, indolyle, isoindolyle, indazolyle, benzofuranyle, isobenzofuranyle, purinyle, quinazolinyle, quinolyle, isoquinolyle, benzoimidazolyle, benzothiazolyle, benzothiophényle, thianaphthényle, benzoxazolyle, benzisoxazolyle, cinnolinyle, phthalazinyle, naphthyridinyle, et quinoxalinyle. Sont également compris dans la définition des groupes "hétéroaryle" les systèmes de cycles fusionnés incluant notamment les systèmes cycliques dans lequel le cycle aromatique est fusionné avec un cycle hétérocycloalkyle. Comme exemple de tels systèmes de cycles fusionnés, on peut citer notamment le phthalamide, l'anhydride phthalique, l'indoline, l'isoindoline, et la tetrahydroisoquinoline.

Le terme « hétéroarylalkyle » désigne un groupe aryl-hétéroaryl-, dans lequel l'hétéroaryle et l'alkyle sont tels que décrits dans le présent document.

Les termes « alkylène, cycloalkylène, hétérocycloalkylène, arylène, hétéroarylène » désignent respectivement des groupes bivalents alkyle, cycloalkyle, hétérocycloalkyle aryle et hétéroaryle, ces groupes étant tels que définis ci-dessus.

A⁻ est un anion choisi parmi un halogénure, P(R⁴)⁶⁻, B(R⁴)⁴⁻, SCN⁻, (R⁵SO₂)₂N⁻, R⁵OSO₃, R⁵SO₃⁻, carborane, carbonate (CO₃²⁻), hydrogénocarbonate (HCO₃⁻), alcoolate (R⁴O⁻), carboxylate (R⁴COO⁻), amidure (NH₂⁻), phosphate (PO₄⁻), SiF₆⁻, SbF₆⁻, I₃⁻, nitrate (NO₃⁻), oxyde d'halogénure, silicate, sulfate (SO₄⁻), sulfonate (R⁴SO₃⁻), cyanure (CN⁻), carbanions, ou métallate ;
où :
R⁴ est, à chaque occurrence, un groupe indépendamment choisi parmi un atome d'halogène, un groupe alkyle en C₁-C₆, un groupe aryle en C₆-C₁₀, un groupe aralkyle ;
R⁵ est, à chaque occurrence, un groupe indépendamment choisi parmi alkyle en C₁-C₂₀, halogénoalkyle en C₁-C₂₀, aryle en C₆-C₁₀, aralkyle.

Le terme "métallate" désigne un complexe anionique contenant un métal, notamment un métal de transition complexé par plusieurs ligands, par exemple un chalcogène tel que l'oxygène ou un groupe cyanure. De préférence, l'anion métallate est un groupe cyanométallate ou oxométallate.

Le terme "carborane" désigne une molécule constituée d'atomes de bore, de carbone et d'hydrogène et portant une charge négative. A titre d'exemple, on peut citer CB₁₁H₁₂⁻.

Le terme "oxyde d'halogénure" désigne des oxydes de formule HalOₓ⁻ où Hal représente Br, Cl ou I et x est un entier de 1 à 4. A titre d'exemple, on peut citer ClO₄⁻, IO₃⁻ .Le terme "carbanion" désigne un composé comprenant un atome de carbone porteur d'une charge négative. A titre d'exemple, on peut citer (CF₃SO₂)₃C⁻.

Plus préférentiellement, A⁻ est choisi parmi Cl⁻, Br⁻, I⁻, PF₆⁻, BF₄⁻, (CF₃SO₂)₂N⁻, C₁₂H₂₅OSO₃⁻, C₁₆H₃₃OSO₃⁻, CF₃SO₃⁻.Selon une variante, A⁻ est PF₆⁻. Selon une variante, A⁻ est BF₄⁻.

Selon un autre objet, l'invention concerne un matériau, notamment un matériau plastique transparent comprenant ou constitué d'un composé défini selon les revendications.

L'invention concerne également un matériau solide ou liquide fluorophore comprenant ou constitué d'un composé fluorophore défini selon les revendications. Avantageusement, le matériau solide ou liquide fluorophore est un matériau plastique transparent.

L'invention concerne selon un second aspect l'utilisation de ces molécules pour leurs propriétés de fluorophore. Ainsi l'invention concerne l'utilisation d'un composé fluorophore tel que défini selon les revendications ou d'un matériau tel que défini selon les revendications, pour la détection des rayonnements gamma, X, neutron, proton.

Selon une variante, l'invention concerne l'utilisation pour la discrimination des rayonnements proton/gamma, proton/X, neutron/gamma, neutron/X, alpha/gamma, alpha/X.

L'invention concerne selon un autre aspect l'utilisation d'un composé fluorophore tel que défini selon les revendications ou d'un matériau tel que défini selon les revendications, pour la fabrication d'un instrument de radiodétection, de dosimétrie industrielle ou médicale.

La présente invention concerne selon un troisième aspect également un procédé de synthèse permettant de préparer des composés ioniques selon l'invention avec le groupement désiré.

En particulier, l'invention concerne un procédé de préparation d'un composé comprenant un groupe imidazolium lié directement à un groupe aromatique par une liaison covalente entre un atome d'azote du groupe imidazolium et un atome de carbone dudit groupe aromatique, ledit procédé comprenant une réaction de couplage d'un groupe aromatique à un groupe imidazole par création d'une liaison covalente entre un atome de carbone sp² du groupe aromatique et un atome d'azote du groupe imidazole, en présence d'une zéolite NaY comportant du cuivre (II) et une base, de préférence par couplage de type Ullmann.

Les inventeurs décrivent aussi la préparation d'un composé de formule (I), ledit procédé comprenant :
(i) une réaction de couplage d'un groupe aromatique « R » à un groupe imidazole par création d'une liaison covalente entre un atome de carbone sp² du groupe aromatique et un atome d'azote du groupe imidazole, en présence d'une zéolite NaY comportant du cuivre (II) et une base, de préférence par couplage de type Ullmann ; et
(ii) une réaction de couplage d'un groupe « Y-Ar-U » comprenant au moins un atome de carbone sp³ avec un groupe imidazole par création d'une liaison covalente entre l'atome de carbone sp³ du groupe « Y-Ar-U » et un atome d'azote du groupe imidazole, de préférence par substitution nucléophile, de préférence en utilisant un groupe « Y-Ar-U » réactif halogéné.

Selon une variante, l'étape (i) correspond à la réaction suivante : 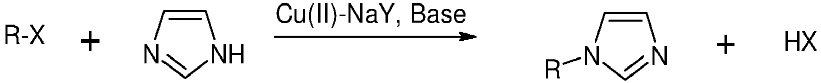 où X est un atome d'halogène, de préférence la réaction étant réalisée en présence de radiations micro-ondes.

Selon un mode de ralisation particulier, l'étape (ii) correspond à la réaction suivante : 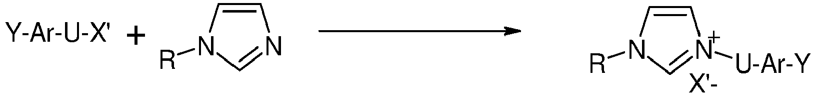 où X'- est un atome d'halogène et « - » la charge négative de cet atome.

De préférence, le composé 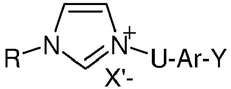 est mis à réagir avec un sel de l'anion A⁻ désiré pour obtenir par metathèse anionique le composé

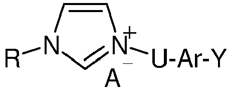

De préférence le sel de l'anion A⁻ est un sel de lithium, de sodium ou de potassium.

Le catalyseur est simple à préparer : la zéolite NaY est mise en présence de sulfate de cuivre dissout dans de l'eau et agitée pendant 24 heures. La zéolite imprégnée de cuivre (II) est récupérée par filtration, séchée à l'étuve (100 °C) puis calcinée à 550 °C pendant 4 heures. On peut notamment faire référence à la publication M. L. Kantam, B. P. C. Rao, B. M. Choudrary, R. S. Reddy, Synlett, 2006, 14, 2195-2198. doi : 10.1055/s-2006-949615.

Lors du couplage de type Ullmann (J. Hassan, M. Sevignon, C. Gozzi, E. Schulz, M. Lemaire, « Aryl-Aryl Bond Formation One Century after the Discovery of the Ullmann Reaction », Chemical Reviews, vol. 102, 2002, p. 1359-1470), les réactifs sont chauffés par exemple dans un tube scellé pendant 72 heures à 180 °C à l'aide d'un bain de sable. Pour récupérer le brut de réaction, il suffit de reprendre dans un solvant organique comme le dichlorométhane et de filtrer afin d'éliminer la zéolite et la base (carbonate de potassium). Le dérivé imidazole ainsi obtenu est purifié par exemple par chromatographie sur colonne.

Bien que n'utilisant pas de solvant ni d'atmosphère inerte, il est nécessaire de chauffer le mélange réactionnel nécessaire pour le couplage de l'imidazole et de l'aryle, par exemple en chauffant à une température de 180 °C pendant 72 heures.

Avantageusement, la réaction est réalisée en présence de radiations micro-ondes. Par cette méthode les inventeurs ont pu diminuer très sensiblement le temps de réaction. On peut passer d'un temps de réaction de 72 heures (en tube scellé chauffé avec un bain de sable) à moins de 3 heures et cela pour des rendements identiques.

Les micro-ondes sont des ondes électromagnétiques qui ont une longueur d'onde comprise entre 30 cm (1 GHz) et 1 mm (300 GHz).

La base est choisie de préférence parmi un carbonate de potassium ou de césium, un phosphate de potassium, un phosphate de césium, LiOH, NaOH, ou KOH.

Avantageusement, le procédé selon la présente invention ne requiert pas de solvant. Selon l'art antérieur les procédés de synthèse requièrent l'utilisation d'un solvant (diméthylformamide, diméthylsulfoxyde, toluène, ...), ce qu'évite la présente invention, selon un mode de réalisation. L'invention couvre toutefois selon une variante l'utilisation d'un solvant.

La présente invention permet d'éviter l'utilisation de ligands aminés (carbène, phénanthroline, L-proline ...).

Ce procédé permet d'éviter l'utilisation de solvants, d'atmosphère inerte et de ligands chers, et permet de préparer en seulement deux ou trois étapes les composés de l'invention. Par ailleurs, le procédé selon l'invention permet d'utiliser des molécules commercialement disponibles.

De manière très avantageuse, l'utilisation de radiations micro-ondes lors du couplage de type Ullmann permet de diminuer significativement le temps de réaction. Le temps de réaction peut ainsi passer de 72 à moins de 3 heures.

La première étape de synthèse consiste à réaliser le couplage de type Ullmann, entre un imidazole, c'est-à-dire une molécule comprenant un groupe imidazole, et une molécule porteuse d'un groupe aromatique halogéné, c'est-à-dire porteuse d'un atome d'halogène sur un atome de carbone, atome d'halogène qui va être remplacé par l'imidazole.

La seconde étape consiste à faire réagir l'imidazole ainsi obtenu avec une molécule définie par « Y-Ar-U » pour créer une liaison covalente entre l'imidazole et un atome de carbone sp³ de cette molécule.

Selon une variante cette seconde étape consiste à réaliser une *N*-alkylation, c'est-à-dire à faire réagir l'imidazole avec un radical alkyle pour créer une liaison *N*-alkyle entre le radical alkyle et l'atome d'azote de l'imidazole (atome d'azote non porteur du groupe aromatique greffé par couplage de Ullmann).

Une éventuelle troisième étape consiste à échanger l'anion halogénure afin d'obtenir un composé fluorophorelorsque la molécule comprenant le groupe aromatique est fluorophore. Cette troisième étape peut être réalisée en milieu biphasique.

On peut utiliser le sel de potassium, de lithium, ou de sodium de l'anion désiré.

Eventuellement, ledit procédé peut également comprendre l'étape consistant à isoler le produit obtenu.

Le procédé de l'invention, est particulièrement intéressant car extrapolable à l'échelle industrielle du fait du faible nombre d'étapes de synthèse, des réactifs utilisés, qui sont commerciaux, ainsi que de l'appareillage pour les réactions, utilisable à plus grande échelle pour des réactions industrielles.

Il a été observé que les propriétés de luminescence sont conservées pour tous les composés purs à l'état solide ou liquide. Ceci permet donc une utilisation avantageuse de matériaux à l'état solide ou liquide.

Ainsi, l'invention concerne également des matériaux solides ou liquides comprenant ou constitués des composés de l'invention.

Les composés de l'invention peuvent remplacer les liquides organiques scintillants. Ainsi, l'invention couvre notamment l'utilisation des composés de l'invention, solides ou liquides, pour la détection et/ou l'identification des neutrons rapides et/ou lents. On entend par « neutrons rapides » des neutrons dont l'énergie cinétique est supérieure à 100 keV. On entend par « neutrons lents » des neutrons dont l'énergie cinétique est inférieure à 1 keV.

Les analyses thermogravimétriques ont montré que, les composés de l'invention sont stables jusqu'à 200°C, ce qui permet d'envisager qu'ils satisfont à toutes règles d'hygiène et sécurité.

L'invention couvre en particulier des dispositifs comprenant les composés de l'invention, adaptés pour une utilisation dans des centrales nucléaires, des hôpitaux, des aéroports, ou lieux similaires.

Les composés de l'invention ne présentant pas de tension de vapeur mesurable peuvent être utilisés sous vide.

Parmi les applications des composés de l'invention, on peut citer la dosimétrie industrielle (radioprotection, dosimétrie) et médicale, l'imagerie médicale et des animaux, l'électronique moléculaire, notamment pour la préparation de diodes électroluminescentes ou de lasers organiques, en biologie, notamment par couplage ou avec des marqueurs en biologie (diagnostic médical) ou en électronique moléculaire. En particulier, il est fait référence à la transfection de siRNA (Mesomorphic Imidazolium salts : New vectors for efficient siRNA transfection, J. Am. Chem. Soc. 2009, 131, 13338-13346).

Les applications spécifiques des composés de l'invention dépendent de la molécule considérée et varient en fonction de la nature de l'anion, du cation (imidazole de départ) et du groupe aromatique, qui doit notamment servir de fluorophore.

Il est envisagé que l'utilisation de composés de l'invention permette d'abaisser le seuil de détection et la sensibilité des installations existantes dans les domaines comme la sécurité nucléaire (radioprotection, prolifération des déchets ou d'armement nucléaire, réacteur nucléaire) et la recherche fondamentale.

Pour caractériser un produit et vérifier sa pureté, des méthodes d'analyses classiques telles que les spectroscopies de résonance nucléaire magnétique (RMN), infrarouge (IR), ultraviolet/visible (UV/Visible) et les analyses élémentaires existent. Des techniques spécifiques ont été développées pour identifier et caractériser les mésophases : la microscopie à lumière polarisée (POM, acronyme anglais signifiant Polarized Optical Microscopy), les analyses thermiques (analyse thermogravimétrique et calorimétrie différentielle à balayage), la diffraction des rayons X de la mésophase (DRX de la mésophase) et la dilatométrie.

Ces techniques ont été utilisées pour caractériser les composés synthétisés.

De manière avantageuse, les composés de l'invention peuvent être utilisés purs, c'est-à-dire sous forme d'un matériau essentiellement constitué d'au moins un composé de l'invention. Une forte concentration permet avantageusement d'augmenter d'autant plus l'efficacité de détection de rayonnements. Les composés décrits précédemment (NE213 ou BC501) ne permettaient pas l'utilisation de fortes concentrations car leur pouvoir de détection des rayonnements diminuait à partir d'une certaine concentration. Ainsi, l'invention représente un perfectionnement significatif à cet égard. L'invention couvre également l'utilisation des composés de l'invention sous forme diluée, comme charge dans des matrices polymères en tant que constituants de matériaux composites. La concentration des composés de l'invention peut être de 1 à 80% en masse dans un polymère ou dans un solvant.

### FIGURES

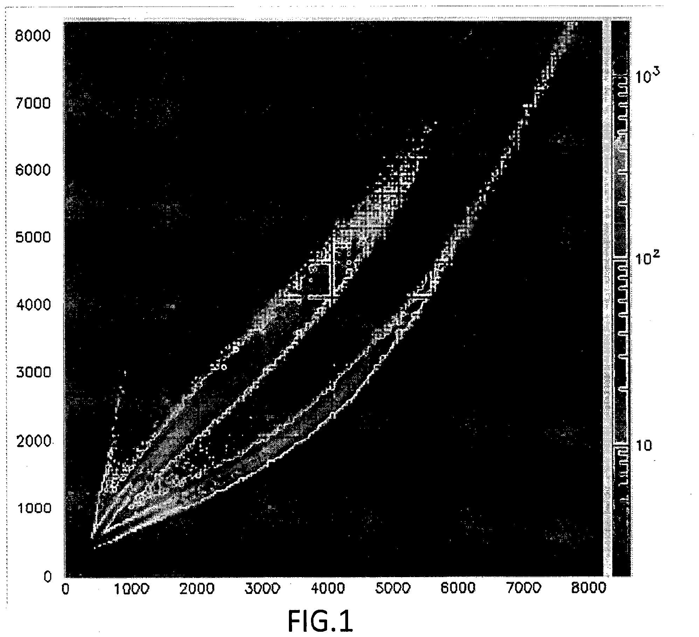

Figure 1 : Discrimination neutrons/gamma du NE213 via une étude de la forme de l'impulsion lumineuse (méthode PSD). Cette caractérisation est utilisée dans l'analyse en forme des signaux délivrés par un détecteur tel que DEMON constitué de NE213 et irradié par une source d'AmBe : l'intégration de la charge du signal total (Qtot) contre celle de la partie lente (Qlent) permet de discriminer les neutrons des rayons gamma. Il convient de préciser que le neutron étant une particule neutre, il ne peut pas être détecté directement mais doit d'abord être converti en une particule chargée (un proton par exemple), par réaction nucléaire et c'est la particule secondaire qui interagit avec le matériau de détection.

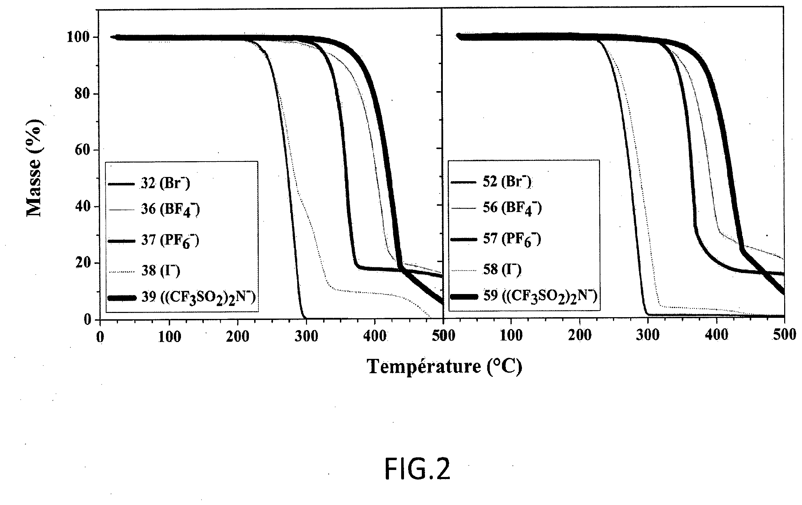

Figure 2 : Analyses thermogravimétriques des composés 32, 36 à 39, 52 et 56 à 59. Note : concernant l'axe des abscisses (température) : le 500 du graphique de gauche correspond au 0 du graphique de droite.
Figure 3 : Analyses thermogravimétriques des composés 33, 40 à 43, 53 et 60 à 63. Note : concernant l'axe des abscisses (température) : le 500 du graphique de gauche correspond au 0 du graphique de droite.
Figure 4 : Analyses thermogravimétriques des composés 34, 44 à 47, 54 et 64 à 67. Note : concernant l'axe des abscisses (température) : le 500 du graphique de gauche correspond au 0 du graphique de droite.
Figure 5 : Cliché de diffraction de rayons X du composé 45 enregistré à l'état amorphe (T = 20 °C).
Figure 6 : Analyses thermogravimétriques des composés 35, 48 à 51, 55 et 68 à 71. Note concernant l'axe des abscisses (température) : le 500 du graphique de gauche correspond au 0 du graphique de droite.
Figure 7 : Spectres de luminescence des composés 52, 53, 54, 58 et 68.
Figure 8 : Photos du matériau 60 à l'état solide (gauche), éclairé avec une lampe UV à l'état liquide avec un début de cristallisation (milieu) et solide (droite).
Figure 9 : L'image de gauche montre clairement deux composantes correspondant aux neutrons et aux rayons gamma lors d'une irradiation par une source d'²⁴¹Am⁹Be. Sur l'image de droite, seule la composante correspondant aux rayons gamma apparaît lorsque le composé est irradié par des sources de ¹³⁷Cs et de ²²Na.

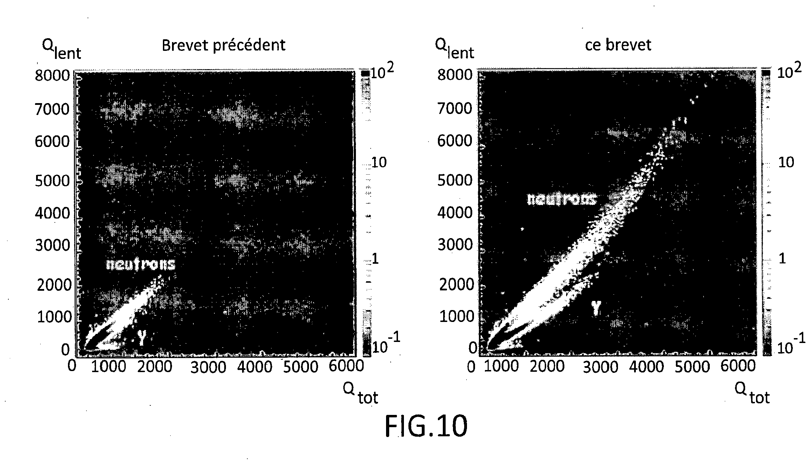

Figure 10 : L'image de droite (référencée « ce brevet ») correspond au composé 60 et l'image de gauche au composé oxazole imidazolium (demande de brevet WO 2010/004228, référencée « Brevet précédent »). Les graphiques représentent une comparaison des deux composantes correspondant aux neutrons et aux rayons gamma lors d'une irradiation par une source d'²⁴¹Am⁹Be mesurées dans les mêmes conditions. Sur l'image de droite (composé 60), les 2 composantes (neutron -gamma) sont plus intenses et mieux séparées que sur l'image de gauche (référencée « Brevet précédent » - demande de brevet WO 2010/004228). Cela met en évidence une meilleure discrimination et détection des rayonnements neutron-gamma.

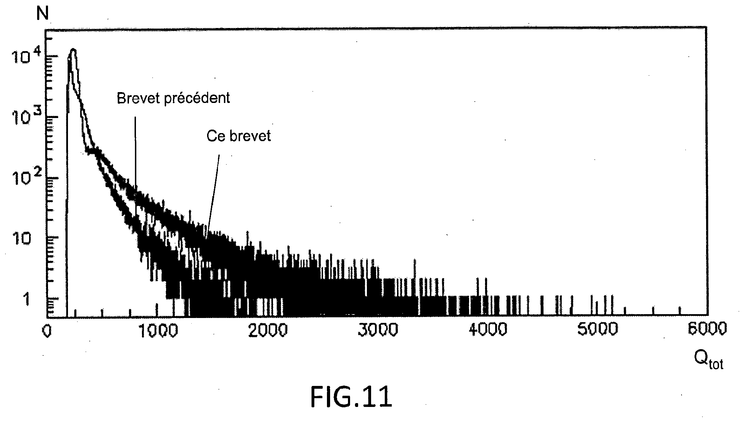

Figure 11 : Le graphique représente la projection de la charge totale (Qtot) comparée entre le composé 60 et le composé oxazole imidazolium du brevet précédent (« Brevet précédent » - demande de bevet WO 2010/004228). La charge totale du composé 60 atteint des valeurs bien plus élevées que celle du composé de la demande de brevet précédente.

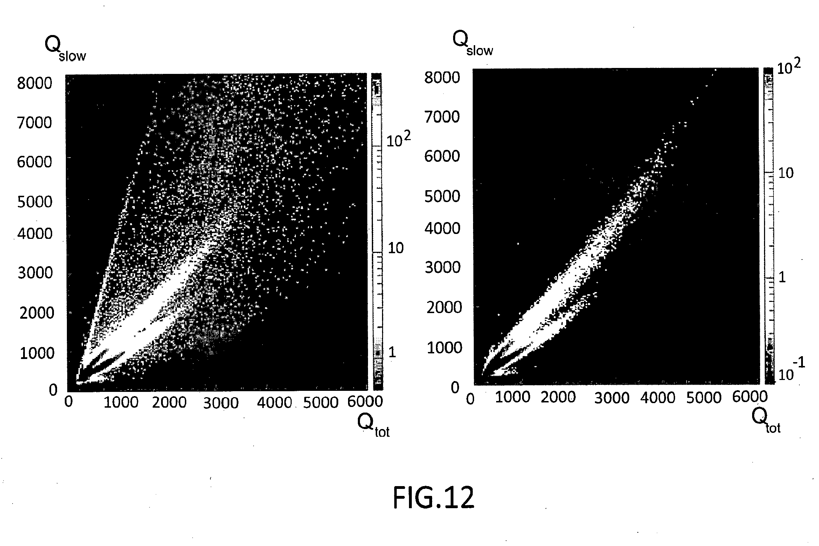

Figure 12 : Les deux images représentent la charge lente (Qslow) en fonction de la charge totale (Qtot) du composé 60 irradié par une source AmBe avec environ un an d'intervalle.

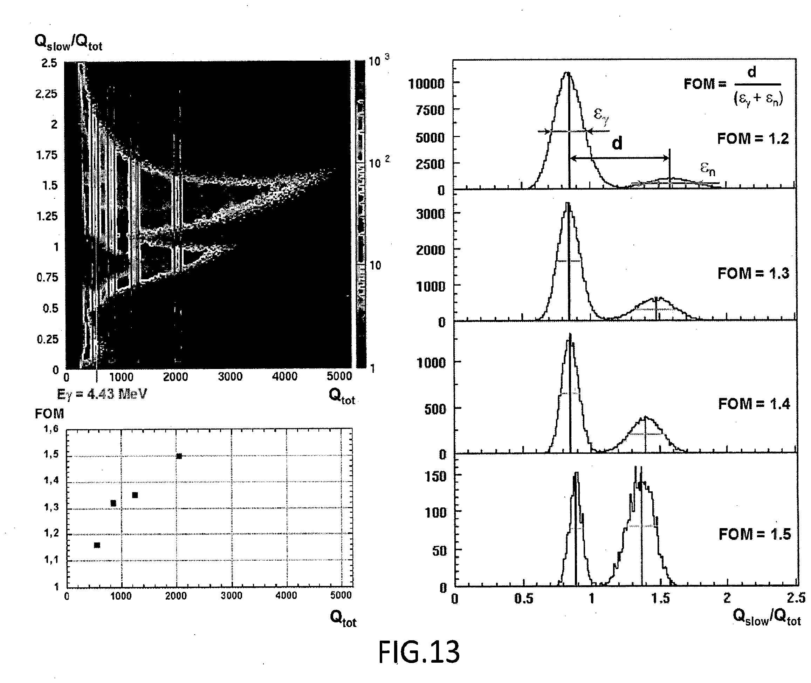

Figure 13 : Les graphiques montrent le pouvoir de discrimination du composé 60 irrigué par une source AmBe. Sur la partie de gauche les spectres bidimensionnels montrent le ratio entre la charge lente et la charge totale (R=Qslow/Qtot) en fonction de la charge totale (Qtot). Quatre tranches, indiquées sur la figure en haut à gauche, ont été sélectionnées à partir de la figure 13 en haut à gauche, et les ratios sont projetés sur la partie droite de la figure 13. La figure en bas à gauche représente l'évolution du facteur de mérite (FOM ou « Figure of Merit ») avec une énergie déposée croissante. Ces valeurs sont comparables aux valeurs obtenues avec un scintillateur plastique développé par Zaitseva et al. (Zaitseva N., B.L. Rupert, I. Pawelczak, A. Glenn, H.P. Martinez, L. Carman, M. Faust, N. Cherepy, and S. Payne. 2012. Plastic Scintillators with Efficient Neutron/Gamma Pulse Shape Discrimination. Nuclear Instruments and Methods in Physics Research A, 668 88-93.)

### EXEMPLES

Les exemples suivants illustrent l'invention, sans toutefois la limiter. Les produits de départ utilisés sont des produits connus ou préparés selon des modes opératoires connus.

Les pourcentages sont exprimés en poids, sauf mention contraire. Tous les composés des exemples 1, 3 et 4 ne font pas partie de l'invention.

### Synthèse d'imidazolium luminescents : applications de discrimination de rayons neutrons/gamma

Il a été réalisé un couplage de type Ullmann, sans solvant, entre un halogénure d'un groupe fluorophore et un imidazole pour préparer des composés de l'invention aux propriétés luminescentes. Ces matériaux ou scintillateurs émettent de la lumière suite à l'absorption d'un rayonnement ionisant. La scintillation est un phénomène de fluorescence où la désexcitation entraîne une émission de photons (le plus souvent dans le visible). Les fluorophores suivants ont été utilisés : le naphtalène, le fluorène, le 9-méthylcarbazole et l'anthracène. La réaction de couplage a été faite entre l'imidazole et le fluorophore bromé (1-bromonaphtalène, 2-bromofluorène, 3-bromo-9-méthylcarbazole, 9-bromoanthracène) afin d'obtenir les composés **28** à **31** (Schéma 1 et tableau 1). (voir publication M. B. Ponce, F. M. Cabrerizo, S. M. Bonesi, R. Erra-Balsells, Helvetica Chimica Acta, 2006, 89, 1123-1139. doi : 10.1002/hlca.200690110)

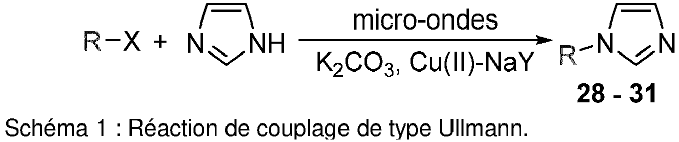

Les couplages ont été réalisés à l'aide d'un four à micro-ondes à 180 °C pendant 140 minutes en présence d'une base (carbonate de potassium) et de Cu(II)-NaY (catalyseur, zéolite imprégnée de cuivre).

Ces conditions ont permis de préparer rapidement les composés **28** à **30** avec des rendements corrects. Le dérivé imidazole **31** a également pu être obtenu rapidement mais avec un rendement faible (30%). Pour récupérer le brut de réaction, il suffit de reprendre au dichlorométhane et de filtrer afin d'éliminer la zéolite et le carbonate de potassium. Le dérivé imidazole ainsi obtenu est purifié par chromatographie sur colonne.

**Tableau 1 : Rendement de la synthèse des composés 28 à 31. Temps de réaction : 140 minutes.**

| **Aryle utilisé** | **Température de réaction** | **Imidazole obtenu** | **Rendement** |
|---|---|---|---|
| 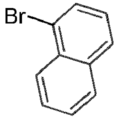 | 180 °C | 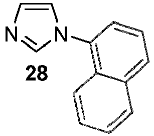 | 67% |
| 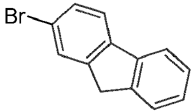 | 180 °C | 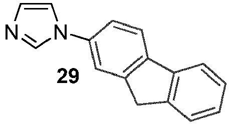 | 62% |
| 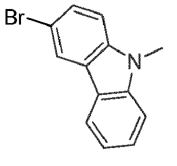 | 180 °C | 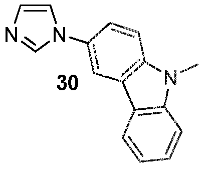 | 52% |
| 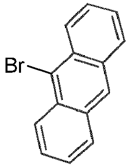 | 180 °C | 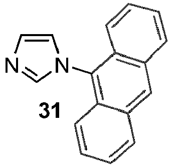 | 30% |

Les dérivés imidazole, **28** à **31**, ont ensuite été alkylés, sans solvant, en présence d'un bromoalcane (1-bromooctane et 1-bromododécane) en chauffant à 110 °C afin d'obtenir les imidazolium correspondants (schéma 2). Durant cette étape d'alkylation, le mélange réactionnel a tendance à prendre en masse. Il a été choisi deux bromoalcanes avec une longueur de chaîne différente (le 1-bromooctane et le 1-bromododécane) afin d'avoir des matériaux avec des températures de fusion différentes (l'anion et la longueur de chaîne ont, tous deux, une influence sur les températures de fusion). Enfin l'anion bromure a été échangé par métathèse anionique en milieu biphasique (dichlorométhane/eau) en utilisant le sel de lithium ou de potassium de l'anion désiré afin d'obtenir les composés **32** à **71** après purification (chromatographie sur colonne).

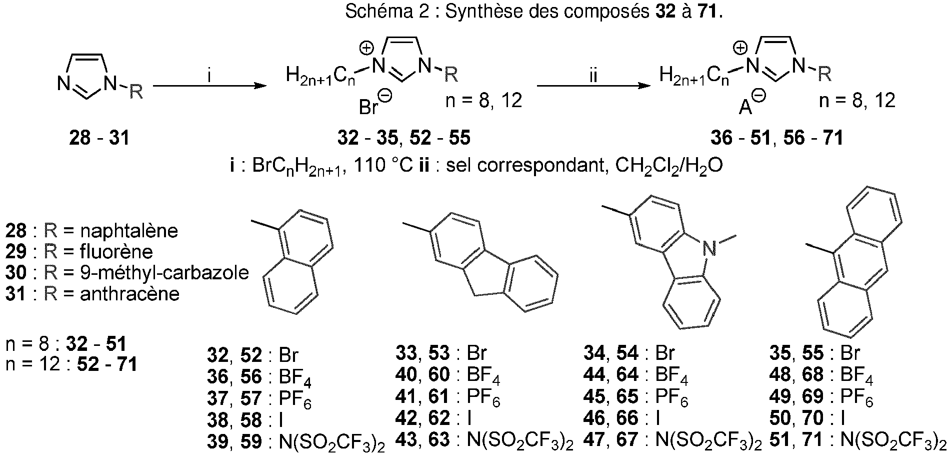

A⁻ est choisi parmi Br⁻, I⁻, PF₆⁻, BF₄⁻, (CF₃SO₂)₂N⁻, comme indiqué ci-dessus.

Les imidazoliums possédant une chaîne à huit carbones sont numérotés de **32** à **51** et ceux possédant une chaîne à douze carbones sont numérotés de **52** à **71**. Tous les imidazoles (**28** à **31**) et les imidazoliums (**32** à **71**) ont été caractérisés par spectroscopie RMN (¹H, ¹³C{¹H}), IR, UV/Visible et analyse élémentaire (C, H, N). Des observations au POM et des mesures thermiques (ATG, DSC) ont été réalisées sur les composés **32** à **71**. Dans un souci de clarté, les résultats d'analyses seront regroupés et présentés par type de fluorophore. Les mesures de luminescence et de discrimination sont également présentées dans des parties différentes à la suite des caractérisations.

Sur le même principe les composés **72** et **73** ont été préparés, avec n =6, R représente un fluorène, et A⁻ représente respectivement Br⁻ et BF₄⁻.

### Exemple 1 - Imidazolium comportant une unité naphtalène (composés n°32, 36 à 39, 52 et 56 à 59).

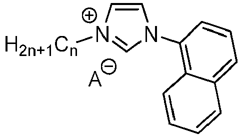

### 1.1 Caractérisations

Les tableaux 2 et 3 regroupent les rendements des synthèses, le déplacement chimique de l'hydrogène N-CH-N de l'imidazolium ainsi que les bandes d'absorption caractéristiques des anions tétrafluoroborate, hexafluorophosphate et bis(trifluorométhylsulfonyl)amidure et les bandes d'absorption en UV/Visible pour les imidazolium contenant une unité naphtalène. Les imidazoliums **32** et **36** à **39** possèdent une chaîne alkyle à huit carbones et les imidazoliums **52** et **56** à **59** ont une chaîne alkyle à douze carbones.

Toutes les analyses élémentaires donnent de bons résultats attestant la pureté des composés synthétisés, hormis l'imidazolium **38**.

**Tableau 2 : N-alkylation et métathèse anionique des composés 32, 36 à 39, 52 et 56 à 59.**

| **Composé** | | **Rendement** | **Déplacement (δ, en ppm / anion)** | | **Bande IR ^{a}** |
|---|---|---|---|---|---|
| **32** | | 82 % | 10,69 / Br | | |
| **36** | | 92 % | 9,11 / BF₄- | | 1053 cm⁻¹ |
| **37** | | 86 % | 8,78 / PF₆⁻ | | 843 cm⁻¹ |
| **38** | | 94 % | 10,26 / I⁻ | | |
| **39** | | 87 % | 9,01 / (CF₃SO₂)₂N⁻ | | 1350 cm⁻¹ |
| | | | | | 1140 cm⁻¹ |
| **52** | | 86 % | 10,68 / Br⁻ | | |
| **56** | | 53 % | 9,10 / BF₄⁻ | | 1053 cm⁻¹ |
| **57** | | 71 % | 8,77 / PF₆⁻ | | 820 cm⁻¹ |
| **58** | | 76 % | 10,43 / I⁻ | | |
| **59** | | 92 % | 9,01 / (CF₃SO₂)₂N⁻ | | 1350 cm⁻¹ 1196 cm⁻¹ |
| ^{a} bande de vibration caractéristique des anions BF₄⁻, PF₆⁻ et (CF₃SO₂)₂N⁻. | | | | | |

**Tableau 3 : Spectroscopie UV/Visible des imidazolium 32, 36 à 39, 52 et 56 à 59.**

| **Composé** | | | **Bande UV/Visible ^{a}** | |
|---|---|---|---|---|
| **32** | | | 283 nm (7200) 228 nm (37300) | |
| **36** | | | 282 nm (7200) 228 nm (33000) | |
| **37** | | | 282 nm (6900) 228 nm (31300) | |
| **38** | 364 nm(500) | | 283 nm (8500) 228 nm (41400) | |
| **39** | | | 283 nm (7600) 228 nm (33000) | |
| **52** | | | 283 nm (7100) 228 nm (35700) | |
| **56** | | | 283 nm (7100) 229 nm (26700) | |
| **57** | | | 283 nm (7100) 229 nm (27300) | |
| **58** | | | 283 nm (8100) 230 nm (25300) | |
| **59** | | | 283 nm (7400) 228 nm (33800) | |
| ^{a} bande d'absorption en UV/Visible : λₘₐₓ (ε, L.mol⁻¹.cm⁻¹). | | | | |

### 1.2 Analyses thermiques

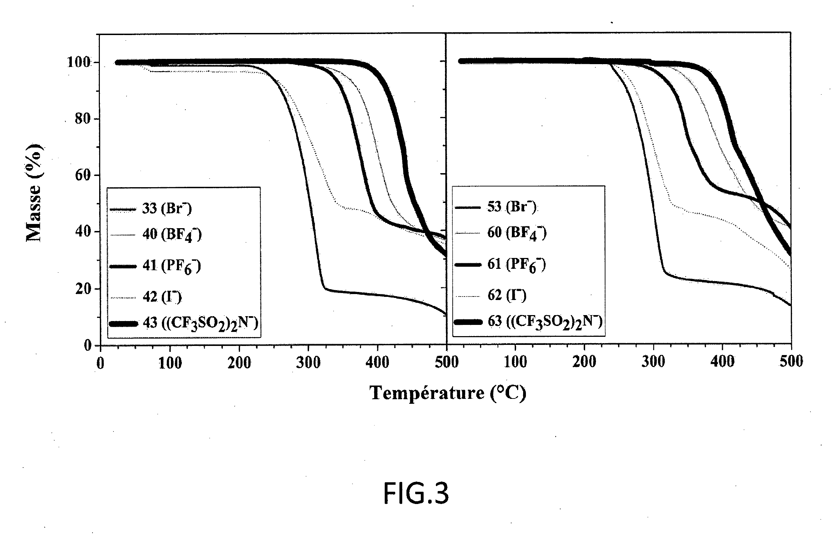

Des mesures thermogravimétriques ont été réalisées afin de déterminer la stabilité des imidazoliums comportant un fluorophore naphtalène (figure 3).

Les imidazoliums (**32**, **36** à **39**, **52** et **56** à **59**) ne présentent pas de propriété mésomorphe (observation au POM), ce qui a été confirmé par DSC. Les températures de transition et les enthalpies correspondantes ont été déterminées par DSC. Excepté les composés **32** et **52**, les autres imidazoliums sont des liquides ioniques (température de fusion inférieure à 100 °C). Les composés **36**, **39**, **56** et **59** sont liquides à température ambiante, leurs températures de fusion n'ont pas pu être déterminées (transition solide amorphe/liquide isotrope). Il est observé une vitrification pour tous les imidazoliums (excepté pour l'imidazolium **57**) qui en refroidissant ne cristallisent pas mais se solidifient dans un état amorphe.

**Tableau 4 : Enthalpies et températures de transition des composés 32, 36 à 39, 52 et 56 à 59.**

| **Composé** | **Chauffage** | **T (°C)** | **H (kJ.mol⁻¹)** | **Refroidissement** | **T (°C)** | **H (kJ.mol⁻¹)** |
|---|---|---|---|---|---|---|
| **32**, Br⁻ | Cr - Iso | 138,0 | 25,88 | vitrification : Tg = 11,6 °C | | |
| **36**, BF₄⁻ | Liquide à TA | | | vitrification : Tg = - 29,7 °C | | |
| **37**, PF₆⁻ | Cr - Iso | 79,4 | 23,17 | vitrification : Tg non mesurable | | |
| **38**, I⁻ | Cr - Iso | 91,7 | 16,96 | vitrification : Tg = - 3,5 °C | | |
| **39**, (CF₃SO₂)₂N⁻ | Liquide à TA | | | vitrification : Tg = - 52,4 °C | | |
| **52**, Br⁻ | Cr - Iso | 112,0 | 36,70 | vitrification : Tg = 8,2 °C | | |
| **56**, BF₄⁻ | Liquide à TA | | | vitrification : Tg = 4,1 °C | | |
| **57**, PF₆⁻ | Cr - Iso | 45,8 | 25,79 | Iso - Cr | cristallisation partielle | |
| **58**, I⁻ | Cr - Iso | 63,3 | 24,85 | vitrification : Tg = - 2,3 °C | | |
| **59**, (CF₃SO₂)₂N⁻ | Liquide à TA | | | vitrification : Tg = - 54,7 °C | | |
| Légende : Cr : Cristal ; Iso : liquide isotrope ; T : température ; H : enthalpie ; Tg : transition vitreuse ; TA : température ambiante. | | | | | | |

Les imidazoliums avec une chaîne en C₁₂ ont une température de fusion inférieure (d'environ 30 °C) à ceux avec une chaîne en C₈. Les températures de fusion les plus basses sont obtenues avec une longueur de chaînes de douze carbones.

### Exemple 2 - Imidazolium comportant une unité fluorène (33, 40 à 43, 53 et 60 à 63).

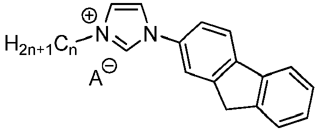

### 2.1 Caractérisations

Les tableaux 5 et 6 regroupent les rendements des synthèses, le déplacement chimique de l'hydrogène N-CH-N de l'imidazolium ainsi que les bandes d'absorption caractéristiques des anions tétrafluoroborate, hexafluorophosphate et bis(trifluorométhylsulfonyl)amidure et les bandes d'absorption en UV/Visible pour les imidazoliums contenant une unité naphtalène. Les imidazoliums **33** et **40** à **43** possèdent une chaîne alkyle à huit carbones et les imidazoliums **53** et **60** à **63** ont une chaîne alkyle à douze carbones.

**Tableau 5 : N-alkylation et métathèse anionique des composés 33, 40 à 43, 53 et 60 à 63.**

| **Composé** | **Rendement** | **Déplacement (δ, en ppm / anion)** | **Bande IR ^{a}** |
|---|---|---|---|
| **33** | 88 % | 11,29 / Br | |
| **40** | 75 % | 9,35 / BF₄- | 1030 cm⁻¹ |
| **41** | 85 % | 9,04 / PF₆⁻ | 820 cm⁻¹ |
| **42** | 89 % | 10,80 / I⁻ | |
| **43** | 89 % | 9,24 / (CF₃SO₂)₂N⁻ | 1343 cm⁻¹ |
| | | | 1130 cm⁻¹ |
| **53** | 86 % | 11,29 / Br | |
| **60** | 84 % | 9,34 / BF₄⁻ | 1067 cm⁻¹ |
| **61** | 68 % | 8,98 / PF₆⁻ | 828 cm⁻¹ |
| **62** | 62 % | 10,82 / I⁻ | |
| **63** | 89 % | 9,26 / (CF₃SO₂)₂N⁻ | 1349 cm⁻¹ |
| | | | 1121 cm⁻¹ |
| ^{a} bande de vibration caractéristique des anions BF₄⁻, PF₆⁻ et (CF₃SO₂)₂N⁻. | | | |

**Tableau 6 : Spectroscopie UV/Visible des imidazolium 33, 40 à 43, 53 et 60 à 63.**

| **Composé** | | **Bande UV/Visible ^{a}** | | |
|---|---|---|---|---|
| **33** | | 304 nm (19900) | 281 nm (22600) | 227 nm (12700) |
| **40** | | 304 nm (19300) | 281 nm (21900) | 227 nm (9300) |
| **41** | | 304 nm (19100) | 281 nm (21400) | 228 nm (11500) |
| **42** | 363 nm (1000) | 303 nm (21400) | 281 nm (23800) | 228 nm (21100) |
| **43** | | 304 nm (19900) | 281 nm (22600) | 227 nm (12700) |
| **53** | | 304 nm (19700) | 281 nm (22300) | 227 nm (15600) |
| **60** | | 304 nm (18800) | 280 nm (21500) | 229 nm (10200) |
| **61** | | 304 nm (18700) | 281 nm (20600) | 229 nm (10300) |
| **62** | | 304 nm (20400) | 282 nm (22500) | 230 nm (19300) |
| **63** | | 304 nm (19900) | 281 nm (21800) | 227 nm (13200) |
| ^{a} bande d'absorption en UV/Visible : λₘₐₓ (ε, L.mol⁻¹.cm⁻¹). | | | | |

### 2.2 Analyses thermiques

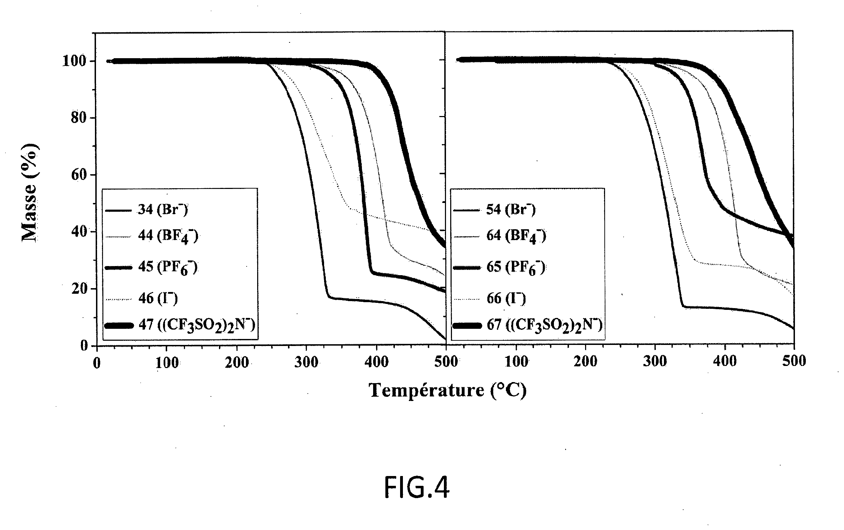

Des mesures thermogravimétriques ont été effectuées afin de déterminer la stabilité des composés qui possèdent une unité fluorène (figure 4). Les bromures et iodures d'imidazolium (**33**, **42**, **53** et **62**) ont une stabilité thermique inférieure (environ 240 °C) aux autres composés. Les composés les plus stables thermiquement sont ceux qui possèdent un anion bis(trifluorométhylsulfonyl)amidure : 387 °C pour l'imidazolium **43** et 358 °C pour l'imidazolium **63**.

Les imidazoliums comportant une unité fluorène (**33**, **40** à **43**, **53** et **60** à **63**) ne présentent pas de propriété mésomorphe (observation au POM), ce qui est confirmé par DSC. Les températures de transition et les enthalpies correspondantes ont été déterminées par DSC (tableau 7 et figure 3). La plupart des composés possèdent une seule phase cristalline. Les composés **53** et **62** présentent deux phases cristallines. Les produits **43** et **63** présentent également deux phases cristallines : la première (Cr₁) est visible uniquement à la première montée en température, la deuxième n'est observée que lors des chauffages suivants.

**Tableau 7 : Enthalpies et températures de transition des composés 33, 40 à 43, 53 et 60 à 63.**

| **Composé** | **Chauffage** | **T (°C)** | **H (kJ.mol⁻¹)** | **Refroidissement** | **T (°C)** | **H (kJ.mol⁻¹)** |
|---|---|---|---|---|---|---|
| **33**, Br⁻ | Cr - Iso | 196,9 | 27,25 | Iso - Cr | 177,9 | 23,09 |
| **40**, BF₄⁻ | Cr - Iso | 79,9 | 17,14 | vitrification : Tg = | 2,8 °C | |
| **41**, PF₆⁻ | Cr - Iso | 110,1 | 45,11 | Iso - Cr | 47,8 | 15,5 |
| **42**, I⁻ | Cr - Iso | 160,2 | 18,35 | Iso - Cr | 115,1 | 17,17 |
| **43**, (CF₃SO₂)₂N⁻ | Cr₁ - Iso | 80,6 | 27,97 | Iso - Cr₂ | 42,6 | 14,17 |
| | Cr₂ - Iso^{∗} | 49,3^{∗} | 12,52^{∗} | | | |
| **53**, Br⁻ | Cr₁ - Cr₂ | 61,8 | 18,17 | Iso - Cr₂ | 172,8 | 24,09 |
| | Cr₂ - Iso | 200,6 | 27,01 | Cr₂ - Cr₁ | 62,4 | 15,71 |
| **60**, BF₄⁻ | Cr - Iso | 79,4 | 18,70 | vitrification : Tg = | 2,0 °C | |
| **61**, PF₆⁻ | Cr - Iso | 111,7 | 30,54 | Iso - Cr | 97,6 | 34,16 |
| **62**, I⁻ | Cr₁ - Cr₂ | 69,8 | 10,47 | Iso - Cr₂ | 129,7 | 19,85 |
| | Cr₂ - Iso | 163,2 | 19,75 | Cr₂ - Cr₁ | 48,4 | 11,86 |
| **63**, (CF₃SO₂)₂N⁻ | Cr₁ - Iso | 79,3 | 40,13 | Iso - Cr₂ | 45,1 | 11,77 |
| | Cr₂ - Iso^{∗} | 68,7^{∗} | 33,12^{∗} | | | |
| Légende : Cr : Cristal ; Cr₁ : cristal 1 ; Cr₂ : cristal 2 ; Iso : liquide isotrope ; T : température ; H : enthalpie ; | | | | | | |
| Tg : transition vitreuse. ^{∗} 2^{ème} montée en température. | | | | | | |

Les températures de fusion (passage à l'isotrope) sont sensiblement les mêmes pour les imidazoliums avec la chaîne alkyle à huit carbones (**33**, **40** à **43**) et ceux possédant une chaîne à douze carbones (**53**, **60** à **63**).

Le bromure de 1-(9H-fluorèn-2-yl)-3-Hexyl-1H-imidazol-3-ium (**72**) a été préparé :

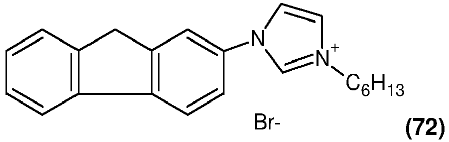

Le tétrafluoroborate de 1-(9H-fluorèn-2-yl)-3-Hexyl-1H-imidazol-3-ium (**73**) a été préparé :

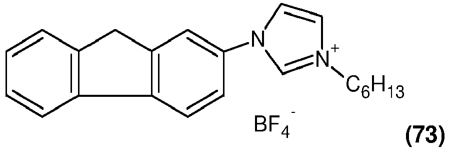

Les composés **72** et **73** ont été caractérisés par spectroscopie RMN et mesure de leur point de fusion. Ces composés présentent des propriétés de discrimination des rayons.

### Exemple 3 -Imidazoliums comportant une unité méthylcarbazole (34, 44 à 47, 54 et 64 à 67)

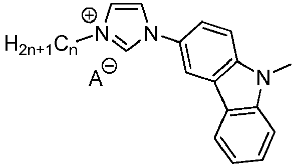

### 3.1 Caractérisations

Les tableaux 8 et 9 regroupent les rendements des synthèses, le déplacement chimique de l'hydrogène N-CH-N de l'imidazolium ainsi que les bandes d'absorption caractéristiques des anions tétrafluoroborate, hexafluorophosphate et bis(trifluorométhylsulfonyl)amidure et les bandes d'absorption en UV/Visible pour les imidazoliums contenant une unité méthylcarbazole. Les imidazoliums **34** et **44** à **47** possèdent une chaîne alkyle à huit carbones et les imidazoliums **54** et **64** à **67** ont une chaîne alkyle à douze carbones

**Tableau 8 : N-alkylation et métathèse anionique des composés 34, 44 à 47, 54 et 64 à 67.**

| **Composé** | **Rendement** | **Déplacement (δ, en ppm / anion)** | **Bande IR ^{a}** |
|---|---|---|---|
| **34** | 85 % | 11,12 / Br | |
| **44** | 85 % | 9,30 / BF₄- | 1024 cm⁻¹ |
| **45** | 95 % | 8,93 / PF₆⁻ | 847 cm⁻¹ |
| **46** | 72 % | 10,70 / I⁻ | |
| **47** | 94 % | 9,15 / (CF₃SO₂)₂N⁻ | 1354 cm⁻¹ |
| | | | 1134 cm⁻¹ |
| **54** | 77 % | 11,15 / Br | |
| **64** | 65 % | 9,32 / BF₄⁻ | 1056 cm⁻¹ |
| **65** | 72 % | 8,95 / PF₆⁻ | 816 cm⁻¹ |
| **66** | 56 % | 10,77 / I⁻ | |
| **67** | 90% | 9,16 / (CF₃SO₂)₂N⁻ | 1350 cm⁻¹ |
| | | | 1197 cm⁻¹ |
| ^{a} bande de vibration caractéristique des anions BF₄⁻, PF₆⁻ et (CF₃SO₂)₂N⁻. | | | |

Le tableau 9 regroupe les analyses de spectroscopie UV/Visible des imidazoliums avec une unité méthylcarbazole.

**Tableau 9 : Spectroscopie UV/Visible des imidazolium 34, 44 à 47, 54 et 64 à 67.**

| **Composé Bande UV/Visible ^{a}** | | | | | | | | | | |
|---|---|---|---|---|---|---|---|---|---|---|
| **34** | 350 (3100) | nm | 335 (4100) | nm | 279 (31700) | nm | 239 (31300) | nm | | |
| **44** | 350 (2700) | nm | 334 (3700) | nm | 278 (29500) | nm | 239 (29200) | nm | | |
| **45** | 350 (2700) | nm | 335 (3800) | nm | 280 (28700) | nm | 239 (29100) | nm | | |
| **46** | 352 (3300) | nm | 335 (4200) | nm | 281 (31500) | nm | 239 (41700) | nm | | |
| **47** | 350 (2800) | nm | 335 (4000) | nm | 280 (28700) | nm | 239 (29500) | nm | | |
| **54** | 352 (3000) | nm | 335 (4000) | nm | 280 (31900) | nm | 239 (31700) | nm | 229 (28600) | nm |
| **64** | 351 (2500) | nm | 334 (3500) | nm | 279 (29600) | nm | 239 (29700) | nm | | |
| **65** | 350 | nm | 334 | nm | 280 | nm | 238 | nm | | |
| | (2200) | | (3200) | | (28600) | | (29300) | | | |
| **66** | 352 (3700) | nm | 336 (4500) | nm | 281 (32200) | nm | 239 (42900) | nm | | |
| **67** | 351 (3000) | nm | 337 (4000) | nm | 281 (28100) | nm | 238 (28900) | nm | | |
| ^{a} bande d'absorption en UV/Visible : λₘₐₓ (ε, L.mol⁻¹.cm⁻¹). | | | | | | | | | | |

### 3.2 Analyses thermiques

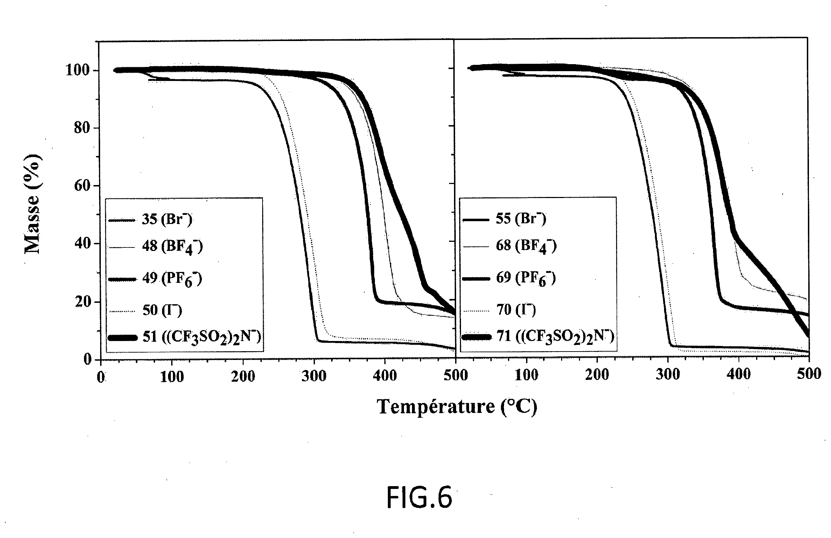

La figure 6 regroupe les analyses thermogravimétriques des composés **34**, **44** à **47**, **54** et **64** à **67**. Les sels d'imidazolium comportant un anion bromure (**34** et **54**) et iodure (**46** et **66**) ont une stabilité thermique inférieure (environ 250 °C) aux autres imidazoliums de la série. Les plus stables sont ceux possédant un anion bis(trifluorométhylsulfonyl)amidure (385 °C pour le composé **47** et 355 °C pour le composé **67**).

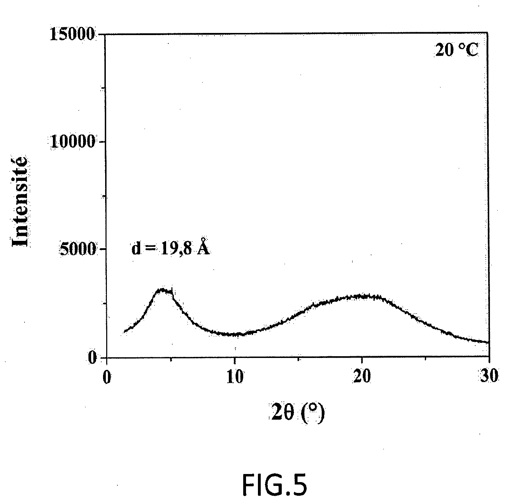

Les imidazoliums comportant une unité méthylcarbazole (**34**, **44** à **47**, **54** et **64** à **67**) ne présentent pas de propriété mésomorphe (observation au POM), confirmé par DSC. Les températures de transition et les enthalpies correspondantes ont été déterminées par DSC (tableau 10 et figure 4). Hormis le composé **67** qui est liquide à température ambiante, les autres composés sont des solides. Avec une température inférieure à 100 °C, les imidazolium **44**, **47**, **64**, **65**, et **67** sont des liquides ioniques. Certains composés présentent plusieurs phases cristallines, notamment l'iodure d'imidazolium **66** qui a trois phases cristallines. La température de fusion du composé **45** n'a pu être déterminée car il se révèle être amorphe à température ambiante, ce qui a été confirmé par une observation au POM et un cliché DRX (figure 5). Les thermogrammes (DSC) du composé **45** suggèrent une transition solide amorphe/liquide isotrope (ou l'inverse lors du refroidissement) sans passer par une étape de cristallisation. Le composé **67**, liquide à température ambiante, se vitrifie lors du refroidissement ; quand il est chauffé, il passe directement d'un état amorphe à l'état liquide, ce qui ne rend pas possible la détermination de sa température de fusion.

**Tableau 10 : Enthalpies et températures de transition des composés 34, 44 à 47, 54 et 64 à 67.**

| **Composé** | **Chauffage** | **T (°C)** | **H (kJ.mol⁻¹)** | **Refroidissement** | **T (°C)** | **H (kJ.mol⁻¹)** |
|---|---|---|---|---|---|---|
| **34**, Br⁻ | Cr - Iso | 171,6 | 11,95 | Iso - Cr | cristallisation partielle | |
| **44**, BF₄⁻ | Cr - Iso | 75,1 | 19,65 | vitrification : Tg = 12,6 °C | | |
| **45**, PF₆⁻ | amorphe à TA ^{∗} | | | vitrification : Tg = 1,7 °C | | |
| **46**, I**⁻** | Cr₁ - Cr₂ | 111,3 | 0,87 | cristallisation partielle | | |
| | Cr₂ - Iso | 137,6 | 23,16 | | | |
| **47**, | Cr₁ - Cr₂ | 38,7 | 3,59 | vitrification : Tg non mesurable | | |
| (CF₃SO₂)₂N | ⁻ Cr₂ - Iso | 57,4 | 27,86 | | | |
| **54**, Br | Cr₁ - Cr₂ | 111,9 | 24,28 | Iso - Cr₂ | 164,9 | 27,15 |
| | Cr₂ - Iso | 186,1 | 28,39 | Cr₂ - Cr₁ | 97,2 | 12,95 |
| **64**, BF₄⁻ | Cr₁ - Cr₂ | 61,4 | 1,10 | vitrification : Tg = 14,0 °C | | |
| | Cr₂ - Iso | 79,9 | 21,40 | | | |
| **65**, PF₆⁻ | Cr - Iso | 85,8 | 24,17 | vitrification : Tg = | 16,1 °C | |
| **66**, I⁻ | Cr₁ - Cr₂ | 129,7 | 9,11 | Iso - Cr | 105,8 | 41,18 |
| | Cr₂ - Cr₃ | 137,2 | 4,81 | | | |
| | Cr₃ - Iso | 140,8 | 9,57 | | | |
| **67**, (CF₃SO₂)₂N | liquide à TA | | | vitrification : Tg = - | 26,1 °C | |
| Légende : Cr : Cristal ; Cr₁ : cristal 1 ; Cr₂ : cristal 2 ; Cr₃ : cristal 3 ; Iso : liquide isotrope ; T : température ; H : enthalpie ; | | | | | | |
| Tg : transition vitreuse ; TA : température ambiante. ^{∗} déterminé par POM et DRX de la mésophase. | | | | | | |

Dans l'ensemble les températures de fusion différent peu suivant la longueur de la chaîne alkyle.

### 3.3 Diffraction de rayons X

Une observation au POM a révélé que le composé **45** est amorphe à température ambiante. Le cliché DRX confirme cet état amorphe de l'imidazolium **45**

### Exemple 4 - Imidazoliums comportant une unité anthracène (35, 48 à 51, 55 et 68 à 71)

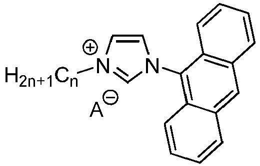

### 4.1 Caractérisations

Les tableaux 11 et 12 regroupent les rendements des synthèses, le déplacement chimique de l'hydrogène N-CH-N de l'imidazolium ainsi que les bandes d'absorption caractéristiques des anions tétrafluoroborate, hexafluorophosphate et bis(trifluorométhylsulfonyl)amidure et les bandes d'absorption en UV/Visible pour les imidazoliums contenant une unité anthracène. Les imidazoliums **35** et **48** à **51** possèdent une chaîne alkyle à huit carbones et les imidazoliums **55** et **68** à **71** ont une chaîne alkyle à douze carbones. L'analyse élémentaire du composé **51** révèle la présence de traces du bromure d'imidazolium (**35**) qui n'ont pas été détectées par chromatographie sur couche mince ni par RMN (¹H, ¹³C{¹H}).

**Tableau 11 : N-alkylation et métathèse anionique des composés 35, 48 à 51, 55 et 68 à 71.**

| **Composé** | **Rendement** | **Déplacement (δ, en ppm / anion)** | **Bande IR ^{a}** |
|---|---|---|---|
| **35** | 93 % | 10,41 / Br⁻ | |
| **48** | 75 % | 9,02 / BF₄- | 1071 cm⁻¹ |
| **49** | 63 % | 8,69 / PF₆⁻ | 878 cm⁻¹ |
| **50** | 71 % | 10,09 / I⁻ | |
| **51** | 65 % | 8,90 / (CF₃SO₂)₂N⁻ | 1354 cm⁻¹ |
| | | | 1134 cm⁻¹ |
| **55** | 95 % | 10,43 / Br⁻ | |
| **68** | 88 % | 9,02 / BF₄⁻ | 1140 cm⁻¹ |
| **69** | 91 % | 8,69 / PF₆⁻ | 845 cm⁻¹ |
| **70** | 91 % | 10,05 / I⁻ | |
| **71** | 91 % | 8,90 / (CF₃SO₂)₂N⁻ | 1350 cm⁻¹ |
| | | | 1138 cm⁻¹ |
| ^{a} bande de vibration caractéristique des anions BF₄⁻, PF₆⁻ et (CF₃SO₂)₂N⁻. | | | |

**Tableau 12 : Spectroscopie UV/Visible des imidazolium 35, 48 à 51, 55 et 68 à 71.**

| **Composé** | **Bande UV/Visible ^{a}** | | | | |
|---|---|---|---|---|---|
| **35** | 387 nm (6000) | 370 nm (6900) | 354 nm (5300) | | 254 nm (149700) |
| **48** | 387 nm (6600) | 370 nm (8200) | 353 nm (6200) | 305 nm (1500) | 254 nm (163500) |
| **49** | 387 nm (5300) | 369 nm (7000) | 352 nm (5400) | | 254 nm (163600) |
| **50** | 387 nm (6100) | 370 nm (7700) | 353 nm (6000) | 322 nm (2100) | 254 nm (159100) |
| **51** | 387 nm (5900) | 370 nm (7700) | 351 nm (5900) | | 254 nm (165600) |
| **55** | 387 nm (6500) | 369 nm (7500) | 352 nm (6000) | | 255 nm (161300) |
| **68** | 387 nm (5400) | 371 nm (6700) | 351 nm (5300) | 308 nm (8500) | 254 nm (185400) |
| **69** | 387 nm (5500) | 370 nm (6800) | 354 nm (5100) | | 254 nm (160900) |
| **70** | 387 nm (6700) | 369 nm (8300) | 354 nm (6700) | 289 nm (3500) | 254 nm (165900) |
| **71** | 387 nm (4900) | 370 nm (6000) | 354 nm (4900) | | 254 nm (156800) |
| ^{a} bande d'absorption en UV/Visible : λₘₐₓ (ε, L.mol⁻¹.cm⁻¹). | | | | | |

### 4.2 Analyses thermiques

Les analyses thermogravimétriques ont été effectuées et montrent que les halogénures d'imidazolium (**35**, **50**, **55** et **70**) sont les moins stables thermiquement (environ 220 °C) de la série (figure 6). Les plus stables (environ 310 °C) sont ceux possédant un anion bis(trifluorométhylsulfonyl)amidure (**51** et **71**).

Le tableau 13 et la figure 10 regroupent les températures de transition ainsi que les enthalpies correspondantes. Seul l'imidazolium **71** est liquide à température ambiante ; sa température de fusion n'a pas pu être déterminée par DSC (transition solide amorphe/liquide isotrope). Tous les autres sont des solides à températures ambiantes et ont une température de fusion qui se situe entre 74 et 134 °C suivant l'anion.

**Tableau 13 : Enthalpies et températures de transition des composés 35, 48 à 51, 55 et 68 à 71.**

| **Composé** | **Chauffage** | **T (°C)** | **H (kJ.mol⁻¹)** | **Refroidissement** | **T (°C)** | **H (kJ.mol⁻¹)** |
|---|---|---|---|---|---|---|
| **35**, Br | Cr - Iso | 92,2 | 24,56 | vitrification : Tg non mesurable | | |
| **48**, BF₄⁻ | Cr - Iso | 100,8 | 21,39 | vitrification : Tg = 7,1 °C | | |
| **49**, PF₆⁻ | Cr - Iso | 114,9 | 23,72 | vitrification : Tg = 10,9 °C | | |
| **50**, I⁻ | Cr - Iso | 74,3 | 21,30 | vitrification : Tg non mesurable | | |
| **51**, (CF₃SO₂)₂N⁻ | Cr₁ - Cr₂ | 71,7 | 1,55 | vitrification : Tg = 14,7 °C | | |
| | Cr₂ - Iso | 108,6 | 20,46 | | | |
| **55**, Br⁻ | Cr₁ - Cr₂ | 109,3 | 22,52 | Iso - Cr₁ | 75,3 | 12,54 |
| | Cr₂ - Iso | 114,3 | 9,45 | | | |
| **68**, BF₄⁻ | Cr₁ - Cr₂ | 77,8 | 22,97 | vitrification : Tg = 14,0 °C | | |
| | Cr₂ - Iso | 100,7 | 2,85 | | | |
| **69**, PF₆⁻ | Cr - Iso | 99,1 | 13,95 | vitrification : Tg = | 6,0 °C | |
| **70**, I⁻ | Cr₁ - Cr₂ | 79,4 | 8,62 | vitrification : Tg = 22,9 °C | | |
| | Cr₂ - Iso | 133,6 | 12,51 | | | |
| **71**, (CF₃SO₂)₂N⁻ | liquide à TA | | | vitrification : Tg = - 35,3 °C | | |
| Légende : Cr : Cristal ; Cr₁ : cristal 1 ; Cr₂ : cristal 2 ; Iso : liquide isotrope ; T : température ; H : enthalpie ; Tg : transition vitreuse ; TA : température ambiante. | | | | | | |

La présence d'une chaîne à huit carbones ne permet pas d'abaisser sensiblement la température de fusion des composés (**35**, **48** à **51**). D'ailleurs le seul composé liquide à température ambiante est un imidazolium possédant une chaîne alkyle à douze carbones.

### Conclusions sur les exemples 1 à 4 - Imidazolium/fluorophores

Les imidazoliums comportant un fluorophore (naphtalène, fluorène, méthylcarbazole et anthracène) ont été synthétisés avec, dans l'ensemble, de bons rendements. Tous ces composés ont été caractérisés par les méthodes usuelles. Les analyses thermiques révèlent que tous les imidazoliums ont une bonne stabilité thermique. La spectroscopie UV/Visible a permis de déterminer les bandes d'absorption des différents sels d'imidazolium afin de pouvoir réaliser des mesures de luminescence.

### Exemple 5 - Luminescence et discrimination de rayons neutrons et gamma

### 5.1 Caractérisation

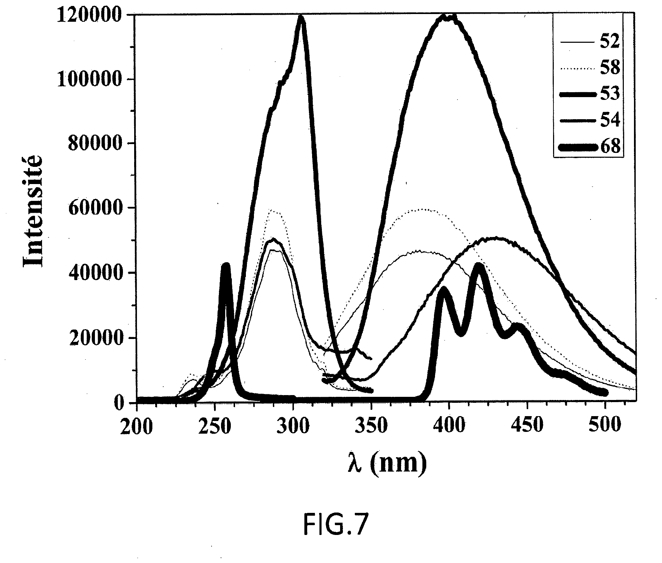

Dans le but de caractériser les propriétés de luminescence, les spectres d'excitation et d'émission des composés **52**, **58** (imidazolium/naphtalène), **53** (imidazolium/fluorène), **54** (imidazolium/méthylcarbazole) et **68** (imidazolium/anthracène) ont été enregistrés (tableau 14 et figure 7).

**Tableau 14 : Analyses de spectroscopie UV/Visible et de luminescence 52, 53, 54 et 58.**

| **Composé** | **UV/Visible** | | | | | | **Luminescence Excitation (nm)** | **Emission (nm)** |
|---|---|---|---|---|---|---|---|---|
| **52** | *λₘₐₓ (nm)* | 228 | 283 | | | | 289 | 384 |
| | *ε(L.mol⁻¹.cm⁻¹)* | 3570 0 | 7100 | | | | | |
| **58** | *λₘₐₓ (nm)* | 230 | 283 | | | | 289 | 384 |
| | *ε(L.mol⁻¹.cm⁻¹)* | 2530 0 | 8100 | | | | | |
| **53** | *λₘₐₓ (nm)* | 227 | 281 | 304 | | | 306 | 401 |
| | *ε(L.mol⁻¹.cm⁻¹)* | 1560 0 | 22300 | 19700 | | | | |
| **54** | *λₘₐₓ (nm)* | 229 | 239 | 280 | 335 | 352 | 288 | 429 |
| | *ε(L.mol⁻¹.cm⁻¹)* | 2860 0 | 31700 | 31900 | 4000 | 3000 | | |
| **68** | *λₘₐₓ (nm)* | 254 | 308 | 351 | 371 | 387 | 258 | 397, 419, 444 |
| | *ε(L.mol⁻¹.cm⁻¹)* | 1854 00 | 8500 | 5300 | 6700 | 5400 | | |

Les spectres de luminescence ont été réalisés dans du dichlorométhane et à faible concentration : 2,45.10⁻⁶ (**52**), 2,50.10⁻⁶ (**58**), 2,52.10⁻⁷ (**53**), 2,52.10⁻⁷ (**54**) et 2,88.10⁻⁷ mol.L⁻¹ (**68**). Tous ces imidazoliums absorbent dans l'ultraviolet entre 258 (**68**) et 288 nm (**54**) et réémettent dans le violet (visible) entre 384 (**52** et **58**) et 444 nm (**68**). Le composé **68** présente trois bandes d'émission lors qu'il est irradié à 258 nm alors que les autres imidazoliums n'ont qu'une seule bande d'émission lors de leur irradiation.

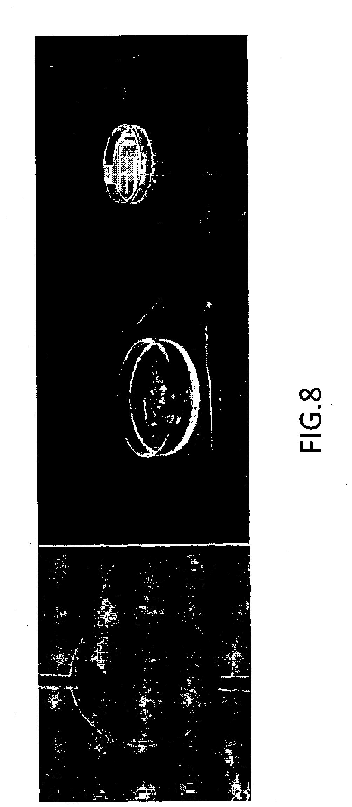

Les composés comportant un fluorène, faciles à préparer en grande quantité (rendement du couplage de type Ullmann supérieur à 50 %), sont de bons candidats pour préparer un scintillateur. Les matériaux comportant un anion tétrafluoroborate donnent de meilleurs résultats en discrimination que les autres anions. Un échantillon d'une dizaine de grammes du composé **60** a été préparé et disposé dans une boîte de pétri. La mise en forme de ce matériau a consisté à y faire fondre puis laisser cristalliser à température ambiante le composé. L'imidazolium est utilisé pur, sans ajout d'autres composés solide, liquide ou de solvant. Le matériau garde ses propriétés de luminescence à l'état liquide et solide (figure 8).

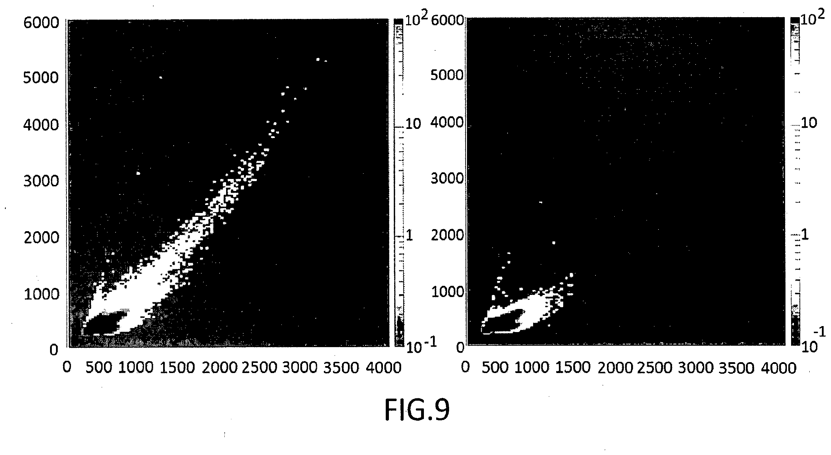

Les résultats préliminaires montrent que l'imidazolium **60** permet de détecter et de discriminer les neutrons des rayonnements gamma (figure 9). Cette propriété de discrimination a été mise en évidence en irradiant le matériau avec une source d'²⁴¹Am⁹Be qui émet des neutrons et des rayons gamma ainsi que des sources émettant uniquement des rayons gamma (¹³⁷Cs, ²²Na, ⁶⁰Co). L'analyse de la forme du signal a été effectuée et on distingue très nettement deux composantes correspondant aux neutrons et aux rayonnements gamma.

### 5.2 Conclusion

Il a été préparé des imidazoliums comportant un fluorophore (naphtalène, fluorène, méthylecarbazole, anthracène) afin d'obtenir des matériaux ayant des propriétés de luminescence et de discrimination de rayonnements neutrons/gamma. Tous les composés ont été caractérisés et leur luminescence a été mesurée cependant aucun de ces matériaux ne présente de propriétés cristal liquide. Les mesures préliminaires de détection effectuées sur l'imidazolium comportant une unité fluorène mettent en évidence ses propriétés de discrimination des rayonnements gamma/neutrons.

Les couplages d'aromatiques homo- et hétérocycliques possédant des propriétés de luminescence ont été étudiés. Des fluorophores (naphtalène, fluorène, méthylcarbazole, anthracène) ont été couplés aux imidazoliums et permettent de préparer des composés présentant des applications en détection et discrimination de rayonnements ionisants. Les mesures préliminaires de détection effectuées sur ces composés comportant un fluorène mettent en évidence leurs propriétés de discrimination des rayonnements neutrons/gamma. En discrimination, la transparence du scintillateur est un paramètre important car celle-ci est basée sur la mesure des luminescences des matériaux : plus il sera transparent, meilleure sera sa réponse en détection. Ce paramètre reste à ajuster pour ce type de matériaux. Toutefois, l'ingénierie cristalline (four à miroir, ...) ou les techniques de fusion de zone devraient permettre d'obtenir la transparence des composés.

### Exemple 6 - Propriétés de transparence

La transparence des échantillons a été évaluée du point de vue de la transmission de la lumière directe, qui représente les performances minimales du matériau, quelles que soient les caractéristiques du détecteur auquel il sera intégré, en particulier, l'angle solide de collecte du photomultiplicateur. La mesure a été réalisée avec un microscope optique et une caméra digitale, en variant le temps d'exposition entre une première configuration où l'échantillon (lamelle de produit, récipient avec cristallisat) est intercalé dans le chemin optique et une seconde configuration sans produit (avec une lamelle de verre de même épaisseur ou un récipient avec la même quantité d'eau). Le rapport des temps pour un même comptage est assimilé à la transmission.

Les résultats sont les suivants :
Composé demande WO 2010/004228 (oxazole, imidazolium, PF6) : 0.07%
Composé 60 : 0.16%

Le composé 60 est environ 2,3 (=16/7) fois plus transparent que le composé oxazole, imidazolium, PF6 (brevet WO 2010/004228) dans les mêmes conditions de préparation (chauffage et cristallisation).

### Exemple 7 - Partie expérimentale

Tous les réactifs et les solvants sont commerciaux et utilisés, sauf indications contraires, sans purification supplémentaire.

Les synthèses au four à micro-ondes ont été effectuées à l'aide d'un four à micro-ondes pour synthèse Monowave 300 (Anton Paar).

Les séparations par chromatographie ont été réalisées avec de la silice Merck Si 60 (40-63 mm) ou avec de l'oxyde d'aluminium Merck 90 standardisé. Les chromatographies flash ont été effectuées à l'aide d'un appareil Combi*Flash* Companion (Teledyne ISCO, Serlabo Technologies). Les chromatographies sur couche mince ont été réalisées avec des feuilles d'aluminium recouvertes de silice Merck Si 60 F254.

Les spectres de résonance magnétiques nucléaires du proton et du carbone ont été enregistrés à l'aide d'un spectromètre Brucker Avance 300. Les déplacements chimiques (δ) sont exprimés en ppm et les abréviations s, s large, d, dd, t, t large, qua, qui, m et m large sont utilisées pour désigner respectivement la multiplicité des signaux : singulet, singulet large, doublet, doublet de doublets, triplet, triplet large, quadruplet, quintuplet, multiplet et multiplet large. Les constantes de couplages, J, sont exprimées en Hz. Sauf indications contraires, les spectres sont enregistrés à 300 MHz pour le proton et à 75 MHz pour le carbone dans du chloroforme deutéré à 25 °C.

Les spectres infrarouges ont été enregistrés avec un spectrophotomètre IR Digital FTS 3000 (pastille KBr). Les fréquences (νₘₐₓ) sont exprimées en cm⁻¹.

Les spectres UV/Visibles ont été enregistrés à l'aide d'un spectrophotomètre U-3000 et le solvant utilisé, sauf indications contraires, est le dichlorométhane. Les longueurs d'onde (λₘₐₓ) sont exprimées en nm et les coefficients d'absorption molaire (ε) en L.mol⁻¹.cm⁻¹. Les spectres de luminescence ont été enregistrés avec un spectrophotomètre Photon Technology International (Motor Driver 5020, Lamp Power Supply 220B, Photomultiplier Détection System 814, Felix software).

Les analyses élémentaires ont été effectuées par le service analytique de l'Institut Charles Sadron et par le service analytique de l'Université de Strasbourg (Strasbourg, France). Les observations des phases et des mésophases ont été réalisées à l'aide d'un microscope à lumière polarisée Leitz Orthoplan équipée d'une platine chauffante Mettler FP82 pilotée par une unité de commande FP80.

Les analyses thermogravimétriques ont été effectuées à l'aide d'un appareil SDT Q600 (10 °C.min⁻¹).

Les températures de transition et les enthalpies correspondantes ont été mesurées par calorimétrie différentielle à balayage avec un appareil DSC Q1000 (TA Instruments) (5 °C.min⁻¹, 2 °C.min⁻¹).

Les mesures de diffraction des rayons X en températures ont été réalisées en transmission sur des échantillons en poudre contenus dans des capillaires de Lindeman ou des cellules scellées, avec un faisceau CuK_{α1}, issu d'un générateur à tube scellé muni d'un monochromateur à lame de quartz focalisant, et un détecteur courbe Inel CPS120, la température étant contrôlée avec une précision de 0.03 °C.

### 7.1 Préparation des composés 28 à 71.

### Procédures pour la synthèse des composés 28 à 71.

### Prodécure générale pour le couplage de type Ullmann (formation de la liaison C-N).

Un mélange d'halogénure d'aryle, d'imidazole, de carbonate de potassium et de Cu(II)-NaY a été chauffé à l'aide d'un four à micro-ondes à 180 °C pendant 140 minutes dans un tube fermé. Le brut de réaction a été repris au dichlorométhane et filtré pour éliminer le catalyseur. Le filtrat est purifié par chromatographie flash (gel de silice, acétate d'éthyle/cyclohexane : 30/70) ou par chromatographie sur colonne (gel de silice, acétate d'éthyle) pour obtenir le produit correspondant pur.

### 1-(naphtalèn-1-yl)-2H-imidazole (28).

La procédure générale avec du 1-bromo-naphtalène (2,534 g ; 12.24 mmol), de l'imidazole (1,088 g ; 15,98 mmol), du carbonate de potassium (1,902 g ; 13,76 mmol) et du Cu(II)-NaY (1,227 g) permet d'obtenir **28** avec un rendement de 67 % (1,584 g ; 8,16 mmol). RMN ¹H (300 MHz, CDCl₃) : δ = 7,31 (s, 1H, C*H* naphtalène ou imidazole) ; 7,34 (s, 1H, C*H* naphtalène ou imidazole) ; 7,49 (d, 1H, J = 6,57 Hz, C*H* naphtalène ou imidazole) ; 7,55-7,64 (m, 4H, C*H* naphtalène ou imidazole) ; 7,92 (s, 1H, N-C*H*-N), 7,99 (t, 2H, J = 7.44 Hz, C*H* naphtalène ou imidazole). RMN ¹³C (75 MHz, CDCl₃) : δ = 121,61 ; 122,27 ; 123,59 ; 125,13 ; 126,91; 127,54 ; 128,25 ; 129,18 (*C*H naphtalène ou imidazole) ; 129,49 (*C* quaternaire); 129,53 (*C*H naphtalène ou imidazole) ; 134,06 ; 134,16 (*C* quaternaire) ; 138,34 (*C*H naphtalène ou imidazole). νₘₐₓ/cm⁻¹ 3056 (C-H aromatique) ; 1595 (C=C aromatique) ; 1279 (C-N). UV/Vis (CH₂Cl₂) : λₘₐₓ (ε L mol⁻¹ cm⁻¹) = 282 nm (7600) ; 231 nm (15900). Analyse élémentaire pour C₁₃H₁₀N₂._{0,04}H₂O ; Calculée : C 80,09 ; H 5,21 ; N 14,37 %. Trouvée : C 80,09 ; H 5,33 ; N 14,32 %.

### 1-(9H-fluorèn-2-yl)-2H-imidazole (29)

La procédure générale avec du 2-bromo-9H-fluorène (1,972 g ; 8,05 mmol), de l'imidazole (0,951 g ; 13,97 mmol), du carbonate de potassium (1,496 g ; 10,82 mmol) et du Cu(II)-NaY (0,924 g) permet d'obtenir **29** avec un rendement de 62 % (1,151 g ; 4,96 mmol). RMN ¹H (300 MHz, CDCl₃) : δ = 3,99 (s, 2H, C*H*₂ fluorène) ; 7,24 (s, 1H, C*H* fluorène ou imidazole) ; 7,35-7,45 (m, 4H, C*H* fluorène ou imidazole) ; 7,58-7,60 (m, 2H, C*H* fluorène ou imidazole) ; 7,91-7,92 (m, 3H, C*H* fluorène ou imidazole). RMN ¹³C (75 MHz, CDCl₃) : δ = 36,87 (*C*H₂ fluorène) ; 118,31 ; 119,95 ; 120,33 ; 120,67 ; 125,01 ; 126,97 ; 127,12 ; 130,28 (CH fluorène ou imidazole) ; 135,71 (*C* quaternaire) ; 135,93 (fluorène ou imidazole) ; 140,38 ; 141,07 ; 143,10; 144,83 (*C* quaternaire). vₘₐₓ/cm⁻¹ 3051 (C-H aromatique) ; 2917 (C-H aliphatique) ; 1587 (C=C aromatique) ; 1259 (C-N). UV/Vis (CH₂Cl₂) : λₘₐₓ (ε L.mol⁻¹.cm⁻¹) = 278 nm (24400) ; 218 nm (9500). Analyse élémentaire pour C₁₆H₁₂N_{2.0,35}H₂O ; Calculée : C 82,35 ; H 5,23 ; N 12,00 %. Trouvée : C 82,36 ; H 5,57 ; N 11,57 %.

### 1-(9-méthyl-carbazol-3-yl)-2H-imidazole (30)

La procédure générale avec du 3-bromo-9-méthyl-carbazole (0,647 g ; 2,49 mmol), de l'imidazole (0,349 g ; 5,13 mmol), du carbonate de potassium (0,691 g ; 5,00 mmol) et du Cu(II)-NaY (0,324 g) permet d'obtenir **30** avec un rendement de 52 % (0,321 g ; 1,30 mmol). RMN ¹H (300 MHz, CDCl₃) : δ = 3,92 (s, 3H, C*H*₃ carbazole) ; 7,25 (s, 1H, C*H* carbazole ou imidazole) ; 7,30-7,37 (m, 2H, C*H* carbazole ou imidazole) ; 7,45-7,59 (m, 4H, C*H* carbazole ou imidazole) ; 7,91 (s, 1H, N-CH-N) ; 8,08-8,13 (m, 2H, C*H* carbazole ou imidazole). RMN ¹³C (75 MHz, CDCl₃) : δ = 29,12 (*C*H₃ carbazole) ; 108,79 ; 109,03 ; 113,90 ; 119,33 ; 120,07 ; 120,42 (CH carbazole ou imidazole) ; 122,06; 123,17 (C quaternaire); 126,57 (*C*H carbazole ou imidazole) ; 129,55 (C quaternaire); 129,90 ; 136,31 (*C*H carbazole ou imidazole) ; 139,81 ; 141,64 (*C* quaternaire). vₘₐₓ/cm⁻¹ 3113 (C-H aromatique) ; 2928 (C-H aliphatique) ; 1503 (C=C aromatique) ; 1228 (C-N). UV/Vis (CH₂Cl₂) : λₘₐₓ (ε L.mol⁻¹.cm⁻¹) = 352 nm (3200) ; 340 nm (3300) ; 299 nm (13300) ; 270 nm (31600) ; 242 nm (38800). Analyse élémentaire pour C₁₆H₁₃N_{3.0,12}H₂O ; Calculée : C 77,04 ; H 5,35 ; N 16,84 %. Trouvée : C 77,04 ; H 5,50 ; N 16,54 %.

### 1-(anthracèn-9-yl)-2H-imidazole (31).

La procédure générale avec du 9-bromoanthracène (0,506 g ; 1,97 mmol), de l'imidazole (0,328 g ; 4,81 mmol), du potassium carbonate (0,482 g ; 3,49 mmol) et du Cu(II)-NaY (0,286 g) permet d'obtenir **31** avec un rendement de 30 % (0,143 g ; 0,59 mmol). RMN ¹H (300 MHz, CDCl₃) : δ = 7,30 (s, 1H, C*H* anthracène ou imidazole); 7,44-7,57 (m, 7H, C*H* anthracène ou imidazole) ; 7,83 (s, 1H, C*H* anthracène ou imidazole) ; 8,10 et 8,12 (dd, 2H, J = 1,92 et 6,84 Hz, C*H* anthracène ou imidazole) ; 8,63 (s, 1H, N-C*H-*N)*.* RMN ¹³C (75 MHz, CDCl₃) : δ= 122,29 ; 122,61 ; 125,79 ; 127,47 ; 128,25 ; 128,36 (*C*H anthracène ou imidazole); 128,67 (*C* quaternaire) ; 129,61 (*C*H anthracène ou imidazole) ; 131,12 (C quaternaire) ; 139,50 (*C*H anthracène ou imidazole). vₘₐₓ/cm⁻¹ 3053 (C-H aromatique) ; 2923 et 2851 (C-H aliphatique) ; 1487 (C=C aromatique). UV/Vis (CH₂Cl₂) : λₘₐₓ (εL.mol⁻¹.cm⁻¹) = 385 nm (8300) ; 366 nm (8300) ; 348 nm (5300) ; 332 nm (2500) ; 255 nm (168100). Analyse élémentaire pour C₁₇H₁₂N_{2.0,06}H₂O ; Calculée : C 83,21 ; H 4,98 ; N 11,42 %. Trouvée : C 83,22 ; H 5,12 ; N 11,24 %.

### Imidazoliums 32 à 51 avec une chaîne carbonée en C₈.

### Prodécure générale pour la synthèse des imidazoliums (N-alkylation).

Un mélange de dérivé imidazole et de 1-bromooctane a été chauffé à l'aide d'un four à micro-ondes pendant 140 minutes à 110°C dans un tube fermé. Le sel d'imidazolium correspondant a été purifié par chromatographie flash (gel de silice, dichlorométhane à dichlorométhane/méthanol : 95/5) ou par chromatographie sur colonne (gel de silice, dichlorométhane/méthanol : 95/5).

### Bromure de 1-(napthalèn-1-yl)-3-octyl-2H-imidazol-3-ium (32).

La procédure générale avec **28** (2,676 g ; 13,78 mmol) et du 1-bromooctane (11,34 g ; 58,70 mmol) permet d'obtenir **32** avec un rendement de 82 % (4,385 g ; 11,32 mmol). RMN ¹H (300 MHz, CDCl₃) : δ = 0,89 (t, 3H, J = 6.60 Hz, C*H*₃ chaîne aliphatique) ; 1,30-1,46 (m large, 10H, C*H₂* chaîne aliphatique et N-CH₂-CH₂-C*H₂*) ; 2,07 (qui, 2H, J = 7,41 Hz, N-CH₂-C*H₂*) ; 4,79 (t, 2H, J = 7,68 Hz, N-C*H₂*) ; 7,49-7,67 (m, 6H, C*H* naphtalène ou imidazolium) ; 7,92 et 7,94 (dd, 1H, J = 1,08 et 7,41 Hz, C*H* naphtalène ou imidazolium) ; 8,01-8,11 (m, 2H, C*H* naphtalène ou imidazolium) ; 10,69 (s, 1H, N-C*H*-N)*.* RMN ¹³C (75 MHz, CDCl₃) : δ = 13,72 (C-*C*H₃ chaîne aliphatique); 22,23 ; 25,93 ; 28,67 ; 28,71 ; 30,12 ; 31,33 (*C*H₂ chaîne aliphatique); 50,16 (N-*C*H₂) ; 120,34 ; 123,17 ; 123,87; 124,34; 124,94 ; 127,27 (CH naphtalène ou imidazolium); 127,36 (C quaternaire); 128,43; 128,50 (*C*H naphtalène ou imidazolium) ; 130,32 (*C* quaternaire) ; 131,19 (*C*H naphtalène ou imidazolium) ; 133,79 (*C* quaternaire) ; 137,27 (CH naphtalène ou imidazolium). vₘₐₓ/cm⁻¹ 3059 (C-H aromatique) ; 2924 et 2851 (C-H aliphatique) ; 1545 (C=C aromatique). UV/Vis (CH₂Cl₂) : λₘₐₓ (εL.mol⁻¹.cm⁻¹) = 283 nm (7200); 228 nm (37300). Analyse élémentaire pour C₂₁H₂₇BrN₂ ; Calculée : C 65,11 ; H 7,03 ; N 7,23 %. Trouvée : C 64,95 ; H 7,01 ; N 7,33 %.

### Bromure de 1-(9H-fluorèn-2-yl)-3-octyl-2H-imidazol-3-ium (33).

La procédure general avec **29** (2,896 g ; 12,47 mmol) et du 1-bromooctane (25,09 g ; 129,93 mmol) permet d'obtenir **33** avec un rendement de 88 % (4,642 g ; 10,91 mmol). RMN ¹H (300 MHz, CDCl₃) : δ = 0,88 (t, 3H, J = 6.30 Hz, C*H*₃ chaîne aliphatique) ; 1.27-1.44 (m large, 10H, C*H₂* chaîne aliphatique et N-CH₂-CH₂-C*H₂*) ; 2,02 (qui, 2H, J = 7,68 Hz, N-CH₂-C*H₂*) ; 4,04 (s, 2H, C*H₂* fluorène) ; 4,64 (t, 2H, J = 7,41 Hz, N-C*H₂*) ; 7,37-7,46 (m, 3H, C*H* fluorène ou imidazolium) ; 7,59-7,61 (m, 2H, C*H* fluorène ou imidazolium) ; 7,73 et 7,75 (dd, 1H, J = 2,19 et 8,22 Hz, C*H* fluorène ou imidazolium) ; 7,83 (d, 1H, J = 6,87 Hz, C*H* fluorène ou imidazolium); 7,94 (d, 1H, J = 8,22 Hz, C*H* fluorène ou imidazolium) ; 8,05 (s, 1H, C*H* fluorène ou imidazolium) ; 11,29 (s, 1H, N-CH-N). RMN ¹³C (75 MHz, CDCl₃) : δ = 13,51 (C-*C*H₃ chaîne aliphatique) ; 22,00 ; 25,74 ; 28,47 ; 28,49 ; 29,91 ; 31,12 (*C*H₂ chaîne aliphatique) ; 36,43 (*C*H₂ fluorène) ; 49,83 (N-*C*H₂) ; 118,04 ; 119,93 ; 120,00 ; 120,54 ; 120,79 ; 122,83 ; 124,59 ; 126,57; 127,31 (CH fluorène ou imidazolium) ; 132,18 (*C* quaternaire) ; 134,74 (*C*H fluorène ou imidazolium) ; 139,05 ; 142,85 ; 142,97 ; 144,76 (*C* quaternaire). vₘₐₓ/cm⁻¹ 3038 (C-H aromatique) ; 2923 et 2854 (C-H aliphatique) ; 1556 (C=C aromatique). UV/Vis (CH₂Cl₂) : λₘₐₓ (ε L.mol⁻¹.cm⁻¹) = 304 nm (19900) ; 281 nm (22600) ; 227 nm (12700). Analyse élémentaire pour C₂₄H₂₉BrN₂ ; Calculée : C 67,76 ; H 6,87 ; N 6,59 %. Trouvée : C 67,52 ; H 6,93 ; N 6,31 %.

### Bromure 1-(9-méthyl-carbazol-3-yl)-3-octyl-2H-imidazol-3-ium (34).

La procédure générale avec **30** (1,554 g ; 6,28 mmol) et du 1-bromooctane (13,09 g ; 67,78 mmol) permet d'obtenir **34** avec un rendement de 85 % (2,357 g ; 5,35 mmol). RMN ¹H (300 MHz, CDCl₃) : δ = 0,88 (t, 3H, J = 6,33 Hz, C*H*₃ chaîne aliphatique) ; 1,27-1,40 (m large, 10H, C*H₂* chaîne aliphatique et N-CH₂-CH₂-C*H₂*) ; 2,02 (qui, 2H, J = 7,68 Hz, N-CH₂-C*H₂*) ; 3,87 (s, 3H, C*H₃* carbazole) ; 4,61 (t, 2H, J = 7,50 Hz, N-C*H₂*) ; 7,30-7,62 (m, 5H, C*H* carbazole ou imidazolium) ; 7.68 (s large, 1H, C*H* carbazole ou imidazolium) ; 7,86 (d, 1H, J = 9,06 Hz ; C*H* carbazole ou imidazolium) ; 8,20 (d, 1H, J = 7,95 Hz, C*H* carbazole ou imidazolium) ; 8,47 (d, 1H, J = 2,19 Hz, C*H* carbazole ou imidazolium) ; 11,12 (s, 1H, N-C*H*-N)*.* RMN ¹³C (75 MHz, CDCl₃) :δ= 13,79 (C-*C*H₃ chaîne aliphatique) ; 22,31 ; 26,05 ; 28,75 ; 28,79 (*C*H₂ chaîne aliphatique) ; 29,01 (*C*H₃ carbazole) ; 30,17 ; 31,43 (*C*H₂ chaîne aliphatique); 49,97 (N-*C*H₂) ; 108,65; 109,08; 113,46; 118,29; 119,30 ; 120,91 ; 121,19 (*C*H carbazole ou imidazolium) ; 121,61 (*C* quaternaire) ; 122,43 (*C*H carbazole ou imidazolium) ; 122,75 ; 125,87 (*C* quaternaire) ; 126,70 ; 134,79 (*C*H carbazole ou imidazolium); 140,13 ; 141,29 (*C* quaternaire). vₘₐₓ/cm⁻¹ 3069 (C-H aromatique) ; 2922 et 2853 (C-H aliphatique) ; 1560 (C=C aromatique). UV/Vis (CH₂Cl₂) : λₘₐₓ (ε L.mol⁻¹.cm⁻¹) = 350 nm (3100) ; 335 nm (4100) ; 279 nm (31700) ; 239 nm (31300). Analyse élémentaire pour C₂₄H₃₀BrN_{3.0,11}H₂O ; Calculée : C 65,16 ; H 6,89 ; N 9,50 %. Trouvée : C 65,15 ; H 6,83 ; N 9,47 %.

### Bromure 1-(anthracèn-9-yl)-3-octyl-2H-imidazol-3-ium (35).

La procédure générale avec **31** (0,473 g ; 1,94 mmol) et du 1-bromooctane (3,487 g ; 18,06 mmol) permet d'obtenir **35** avec un rendement de 93 % (0,790 g ; 1,81 mmol). RMN ¹H (300 MHz, CDCl₃) : δ = 0,91 (t, 3H, J = 6,57 Hz, C*H*₃ chaîne aliphatique) ; 1,31-1,53 (m large, 10H, C*H₂* chaîne aliphatique et N-CH₂-CH₂-C*H₂*) ; 2,13 (qui, 2H, J = 7,41 Hz, N-CH₂-C*H₂*) ; 4,97 (t, 2H, J = 7,41 Hz, N-C*H₂*) ; 7,36-7,39 (m, 2H, C*H* anthracène ou imidazolium) ; 7,47 (t, 1H, J = 1,65 Hz, C*H* anthracène ou imidazolium), 7,58-7,67 (m, 4H, C*H* anthracène ou imidazolium); 7,92 (t, 1H, J = 1,62 Hz, C*H* anthracène ou imidazolium); 8,14-8,17 (m, 2H, C*H* anthracène ou imidazolium); 8,77 (s, 1H, C*H* anthracène ou imidazolium) ; 10,41 (s, 1H, N-C*H*-N)*.* RMN ¹³C (75 MHz, CDCl₃) : δ = 13,88 (C-*C*H₃ chaîne aliphatique); 22,40 ; 26,03 ; 28,82 ; 28,90 ; 30,37 ; 31,47 (*C*H₂ chaîne aliphatique) ; 50,50 (N-*C*H₂) ; 120,27 ; 123,95 (*C*H anthracène ou imidazolium) ; 124,51 (*C* quaternaire) ; 125,12 ; 126,11 (*C*H anthracène ou imidazolium) ; 127,44 (*C* quaternaire) ; 128,67 ; 129,07 (*C*H anthracène ou imidazolium) ; 130,71 (*C* quaternaire) ; 130,93 ; 138,58 (CH anthracène ou imidazolium). vₘₐₓ/cm⁻¹ 3042 (C-H aromatique), 2918 et 2852 (C-H aliphatique) ; 1449 (C=C aromatique). UV/Vis (CH₂Cl₂) : λₘₐₓ (ε L.mol⁻¹.cm⁻¹) = 387 nm (6000) ; 370 nm (6900) ; 354 nm (5300) ; 254 nm (149700). Analyse élémentaire pour C₂₅H₂₉BrN_{3.0,9}H₂O ; Calculée : C 66,19 ; H 6,84 ; N 6,18 %. Trouvée : C 66,19 ; H 6,77 ; N 6,19 %.

### Procédure générale pour la metatheses anionique - échange d'anion.

Un mélange de bromure d'imidazolium dissout dans du dichlorométhane et un mélange du sel correspondant dans de l'eau sont agités ensemble pendant 48 heures. La phase organique est extraite avec du dichlorométhane, séchée sur du chlorure de calcium puis filtrée. Le filtrat est ensuite purifié par chromatographie flash (gel de silice, dichlorométhane à dichlorométhane/méthanol: 95/5) pour obtenir un produit pur.

### Tétrafluoroborate de 1-(naphthalèn-1-yl)-3-octyl-2H-imidazol-3-ium (36).

La procédure general avec **32** (0,826 g ; 2,13 mmol) et du tétrafluoroborate de potassium (0,589 g ; 4,68 mmol) permet d'obtenir **36** avec un rendement de 92 % (0,771 g ; 1,96 mmol). RMN ¹H (300 MHz, CDCl₃) : δ = 0,89 (t, 3H, J = 7,14 Hz, C*H*₃ chaîne aliphatique) ; 1,28-1,40 (m large, 10H, C*H₂* chaîne aliphatique et N-CH₂-CH₂-C*H₂*) ; 2,02 (qui, 2H, J = 7,68 Hz, N-CH₂-C*H₂*) ; 4,49 (t, 2H, J = 7,68 Hz, N-C*H₂*) ; 7,47-7,52 (m, 2H, C*H* naphtalène ou imidazolium) ; 7,57-7,67 (m, 4H, C*H* naphtalène ou imidazolium) ; 7,79 et 7,82 (dd, 1H, J = 1,08 et 7,41 Hz, C*H* naphtalène ou imidazolium) ; 8,01-8,04 (m, 1H, C*H* naphtalène ou imidazolium) ; 8,10 (d, 1H, J = 8,52 Hz, C*H* naphtalène ou imidazolium) ; 9,11 (s, 1H, N-C*H*-N)*.* RMN ¹³C (75 MHz, CDCl₃) : δ = 13,94 (C-*C*H₃ chaîne aliphatique) ; 22,47 ; 26,12 ; 28,80 ; 28,92 ; 29,95 ; 31,56 (*C*H₂ chaîne aliphatique) ; 50,47 (N-*C*H₂) ; 120,51 ; 123,05 ; 124,38; 124,54; 125,17; 127,50 (*C*H naphtalène ou imidazolium); 127,53 (*C* quaternaire) ; 128,67 ; 128,70 (*C*H naphtalène ou imidazolium) ; 130,51 (*C* quaternaire) ; 131,46 (CH naphtalène ou imidazolium) ; 133,99 (C quaternaire) ; 136,41 (CH naphtalène ou imidazolium). vₘₐₓ/cm⁻¹ 3055 (C-H aromatique) ; 1423 (C=C aromatique) ; 1053 (BF₄⁻). UV/Vis (CH₂Cl₂) : λₘₐₓ (ε L.mol⁻¹.cm⁻¹) = 282 nm (7200) ; 228 nm (33000). Analyse élémentaire pour C₂₁H₂₇BF₄N_{2.0,12}H₂O ; Calculée : C 63,63 ; H 6,93 ; N 7,07 %. Trouvée : C 63,62 ; H 6,85 ; N 7,15 %.

### Hexafluorophosphate de 1-(naphtalèn-1-yl)-3-octyl-2H-imidazol-3-ium (37).

La procédure générale avec **32** (0,829 g ; 2,14 mmol) et de l'hexafluorophosphate de potassium (0,498 g ; 2,70 mmol) permet d'obtenir **37** avec un rendement de 86 % (0,829 g ; 1,83 mmol). RMN ¹H (300 MHz, CDCl₃) : δ = 0,89 (t, 3H, J = 7,11 Hz, C*H*₃ chaîne aliphatique) ; 1,28-1,39 (m large, 10H, C*H₂* chaîne aliphatique et N-CH₂-CH₂-C*H₂*) ; 2,01 (qui, 2H, J = 7,68 Hz, N-CH₂-C*H₂*) ; 4,42 (t, 2H, J = 7,68 Hz, N-C*H₂*) ; 7,45-7,51 (m, 2H, C*H* naphtalène ou imidazolium) ; 7,56-7,68 (m, 4H, C*H* naphtalène ou imidazolium) ; 7,73 et 7,76 (dd, 1H, J = 1,11 et 7,41 Hz, C*H* naphtalène ou imidazolium) ; 7,99-8,03 (m, 1H, C*H* naphtalène ou imidazolium); 8,10 (d, 1H, J = 8,25 Hz, C*H* naphtalène ou imidazolium) ; 8,78 (s, 1H, N-CH-N). RMN ¹³C (75 MHz, CDCl₃) : δ= 13,96 (C-*C*H₃ chaîne aliphatique) ; 22,50 ; 26,15 ; 28,79 ; 28,93 ; 29,77 ; 31,58 (*C*H₂ chaîne aliphatique) ; 50,59 (N-*C*H₂) ; 120,51 ; 122,91; 124,47; 124,55; 125,18 ; 127,61 (*C*H naphtalène ou imidazolium) ; 127,61 (*C* quaternaire) ; 128,69 ; 128,86 (*C*H naphtalène ou imidazolium) ; 130,43 (*C* quaternaire); 131,63 (*C*H naphtalène ou imidazolium); 134,03 (*C* quaternaire) ; 135,89 (*C*H naphtalène ou imidazolium). vₘₐₓ/cm⁻¹ 3073 (C-H aromatique) ; 2928 et 2855 (C-H aliphatique) ; 1552 (C=C aromatique), 843 (PF₆⁻). UV/Vis (CH₂Cl₂): λₘₐₓ (ε L.mol⁻¹.cm⁻¹) = 282 nm (6900) ; 228 nm (31300). Analyse élémentaire pour C₂₁H₂₇FₑN₂P ; Calculée : C 55,75 ; H 6,02 ; N 6,19 %. Trouvée : C 55,73 ; H 6,07 ; N 5,82 %.

### Iodure de 1-(naphtalèn-1-yl)-3-octyl-2H-imidazol-3-ium (38).

La procédure général avec **32** (0,831 g ; 2,15 mmol) et de l'iodure de potassium (0,687 g ; 4,14 mmol) permet d'obtenir **38** avec un rendement de 94 % (0,873 g ; 2,01 mmol). RMN ¹H (300 MHz, CDCl₃) : δ= 0,89 (t, 3H, J = 7,14 Hz, C*H*₃ chaîne aliphatique) ; 1,29-1,51 (m large, 10H, C*H₂* chaîne aliphatique et N-CH₂-CH₂-C*H₂*) ; 2,08 (qui, 2H, J = 7,68 Hz, N-CH₂-C*H₂*) ; 4,73 (t, 2H, J = 7,68 Hz, N-C*H₂*) ; 7,51-7,55 (m, 2H, C*H* naphtalène ou imidazolium) ; 7,60-7,68 (m, 4H, C*H* naphtalène ou imidazolium) ; 7,99-8,05 (m, 2H, C*H* naphtalène ou imidazolium) ; 8,11 (d, 1H, J = 8,52 Hz, C*H* naphtalène ou imidazolium) ; 10,26 (s, 1H, N-C*H*-N)*.* RMN ¹³C (75 MHz, CDCl₃) : δ = 13,83 (C-*C*H₃ chaîne aliphatique) ; 22,34 ; 26,02 ; 28,76 ; 28,81 ; 30,08 ; 31,43 (*C*H₂ chaîne aliphatique) ; 50,57 (N-*C*H₂) ; 120,51 ; 123,26 ; 124,03 ; 124,65 ; 125,10 (*C*H naphtalène ou imidazolium) ; 127,34 (C quaternaire); 127,44; 128,63 ; 128,66 (*C*H naphtalène ou imidazolium); 130,28 (C quaternaire) ; 131,41 (*C*H naphtalène ou imidazolium) ; 133,90 (Cquaternaire) ; 136,80 (*C*H naphtalène ou imidazolium). vₘₐₓ/cm⁻¹ 3054 (C-H aromatique) ; 1422 (C=C aromatique). UV/Vis (CH₂Cl₂) : λₘₐₓ (ε L.mol⁻¹.cm⁻¹) = 364 nm (500) ; 283 nm (8500) ; 228 nm (41400). Analyse élémentaire pour C₂₁H₂₇I_{0.93}N_{2.0,07}Br ; Calculée : C 58,51 ; H 6,31 ; N 6,50 %. Trouvée : C 58,52 ; H 6,38 ; N 6,27 %.

### Bis(trifluorométhylsulfonyl)amidure de 1-(naphtalèn-1-yl)-3-octyl-2H-imidazol-3-ium (39).

La procédure générale avec **32** (0,833 g ; 2,15 mmol) et du bis(trifluorométhylsulfonyl)amidure de Ilithium (1,121 g ; 3,91 mmol) permet d'obtenir **39** avec un rendement de 87 % (1,103 g ; 1,88 mmol). RMN ¹H (300 MHz, CDCl₃) : δ = 0,90 (t, 3H, J = 6,57 Hz, C*H*₃ chaîne aliphatique); 1,27-1,42 (m large, 10H, C*H₂* chaîne aliphatique et N-CH₂-CH₂-C*H₂*) ; 2,04 (qui, 2H, J = 7,68 Hz, N-CH₂-C*H₂*) ; 4,46 (t, 2H, J = 7,68 Hz, N-C*H₂*) ; 7,44-7,47 (m, 1H, C*H* naphtalène ou imidazolium) ; 7,55-7,70 (m, 5H, C*H* naphtalène ou imidazolium) ; 7,75 et 7,77 (dd, 1H, J = 1,08 et 7,41 Hz, C*H* naphtalène ou imidazolium) ; 8,03-8,06 (m, 1H, C*H* naphtalène ou imidazolium) ; 8,13 (d, 1H, J = 8,22 Hz, C*H* naphtalène ou imidazolium) ; 9,01 (s, 1H, N-C*H*-N)*.* RMN ¹³C (75 MHz, CDCl₃) : δ = 13,91 (C-*C*H₃ chaîne aliphatique) ; 22,45 ; 26,07 ; 28,72 ; 28,87 ; 28,87 ; 29,87 ; 31,53 (*C*H₂ chaîne aliphatique) ; 50,65 (N-*C*H₂) ; 119,70 (qua, J = 319,65 Hz, (CF₃SO₂)₂N⁻) ; 120,32 ; 123,02 ; 124,54 ; 124,62 ; 125,12 (*C*H naphtalène ou imidazolium) ; 127,61 (*C* quaternaire); 127,63; 128,75; 128,86 (*C*H naphtalène ou imidazolium); 130,33 (C quaternaire) ; 131,73 (*C*H naphtalène ou imidazolium) ; 134,09 (C quaternaire) ; 136,09 (*C*H naphtalène ou imidazolium). vₘₐₓ/cm⁻¹ 3054 (C-H aromatique) ; 1422 (C=C aromatique) ; 1350 et 1140 ((CF₃SO₂)₂N⁻). UV/Vis (CH₂Cl₂) : λₘₐₓ (ε L.mol⁻¹.cm⁻¹) = 283 nm (7600) ; 228 nm (33000). Analyse élémentaire pour C₂₃H₂₇FₑN₃O₄S₂ ; Calculée : C 47,01 ; H 4,63 ; N 7,15 %. Trouvée : C 46,99 ; H 4,67 ; N 7,17 %.

### Tétrafluoroborate de 1-(9H-fluorèn-2-yl)-3-octyl-2H-imidazol-3-ium (40).

La procédure générale avec **33** (0,825 g ; 1,94 mmol) et du tétrafluoroborate de potassium (0,817 g ; 6,49 mmol) permet d'obtenir **40** avec un rendement de 75 % (0,631 g ; 1,46 mmol). RMN ¹H (300 MHz, CDCl₃) : δ = 0,88 (t, 3H, J = 6,30 Hz, C*H*₃ chaîne aliphatique) ; 1,27-1,35 (m large, 10H, C*H₂* chaîne aliphatique et N-CH₂-CH₂-C*H₂*) ; 1,95 (qui, 2H, J = 7,68 Hz, N-CH₂-C*H₂*) ; 3,99 (s, 2H, C*H₂* fluorène) ; 4,38 (t, 2H, J = 7,41 Hz, N-C*H₂*) ; 7,36-7,45 (m, 3H, C*H* fluorène ou imidazolium) ; 7,56-7,61 (m, 3H, C*H* fluorène ou imidazolium) ; 7,78-7,90 (m, 3H, C*H* fluorène ou imidazolium) ; 9.35 (s, 1H, N-C*H-*N)*.* RMN ¹³C (75 MHz, CDCl₃) : δ = 13,96 (C-*C*H₃ chaîne aliphatique) ; 22,50 ; 26,18 ; 28,86 ; 28,95 ; 30,07 ; 31,62 (*C*H₂ chaîne aliphatique) ; 36,85 (*C*H₂ fluorène) ; 50,45 (N-*C*H₂) ; 118,59 ; 120,41; 120,45 ; 121,03 ; 121,41 ; 123,07; 125,10 ; 127,08; 127,85 (*C*H fluorène ou imidazolium) ; 132,61 (*C* quaternaire) ; 133,94 (*C*H fluorène ou imidazolium) ; 139,51 ; 143,56 ; 145,44 (*C* quaternaire). vₘₐₓ/cm⁻¹ 3042 (C-H aromatique); 2926 et 2856 (C-H aliphatique) ; 1558 (C=C aromatique), 1030 (BF₄⁻). UV/Vis (CH₂Cl₂) : λₘₐₓ (ε L.mol⁻¹.cm⁻¹) = 304 nm (19300) ; 281 nm (21900) ; 227 nm (9300). Analyse élémentaire pour C₂₄H₂₉BF₄N₂ ; Calculée : C 66,68 ; H 6,76 ; N 6,48 %. Trouvée : C 66,47 ; H 6,71 ; N 6,51 %.

### Hexafluorophosphate 1-(9H-fluorèn-2-yl)-3-octyl-2H-imidazol-3-ium (41).

La procédure générale avec **33** (0,829 g ; 1,95 mmol) et de l'hexafluorophosphate de potassium (0,558 g ; 3,03 mmol) permet d'obtenir **41** avec un rendement de 85 % (0,812 g ; 1,66 mmol). RMN ¹H (300 MHz, CDCl₃) : δ = 0,82 (t, 3H, J = 6,87 Hz, C*H*₃ chaîne aliphatique) ; 1,21-1,31 (m large, 10H, C*H₂* chaîne aliphatique et N-CH₂-CH₂-C*H₂*) ; 1,92 (qui, 2H, J = 7,41 Hz, N-CH₂-C*H₂*) ; 3,94 (s, 2H, C*H₂* fluorène) ; 4,30 (t, 2H, J = 7,68 Hz, N-C*H₂*) ; 7,31-7,40 (m, 2H, C*H* fluorène ou imidazolium) ; 7,48-7,54 (m, 3H, C*H* fluorène ou imidazolium) ; 7,64 (t, 1H, J = 1,65 Hz, C*H* fluorène ou imidazolium) ; 7,75-7,78 (m, 2H, C*H* fluorène ou imidazolium) ; 7,86 (d, 1H, J = 8,25 Hz, C*H* fluorène ou imidazolium) ; 9,04 (s, 1H, N-C*H*-N)*.* RMN ¹³C (75 MHz, CD₂Cl₂) : δ= 14,19 (C-*C*H₃ chaîne aliphatique) ; 22,97 ; 26,60 ; 29,25 ; 29,37 ; 30,38 ; 32,05 (*C*H₂ chaîne aliphatique) ; 37,41 (*C*H₂ fluorène) ; 51,20 (N-*C*H₂) ; 119,58 ; 121,01 ; 121,40 ; 121,65 ; 122,36 ; 123,39 ; 125,66 ; 127,58 ; 128,51 (*C*H fluorène ou imidazolium); 133,04 (*C* quaternaire) ; 134,13 (*C*H fluorène ou imidazolium) ; 140,00 ; 144,26 ; 144,57 ; 146,20 (*C* quaternaire). vₘₐₓ/cm⁻¹ 3051 (C-H aromatique) ; 2926 et 2859 (C-H aliphatique) ; 1553 (C=C aromatique), 820 (PF₆₋) .UV/Vis (CH₂Cl₂) : λₘₐₓ (ε L.mol⁻¹.cm⁻¹) = 304 nm (19100) ; 281 nm (21400) ; 228 nm (11500). Analyse élémentaire C₂₄H₂₉F₆N₂P ; Calculée : C 58,77 ; H 5,96 ; N 5,71 %. Trouvée : C 58,58 ; H 5,95 ; N 5,74 %.

### Iodure de 1-(9H-fluorèn-2-yl)-3-octyl-2H-imidazol-3-ium (42).

La prodécure générale avec **33** (0,827 g ; 1,94 mmol) et de l'iodure de potassium (0,608 g ; 3,66 mmol) permet d'obtenir **42** avec un rendement de 82 % (0,745 g ; 1,58 mmol). RMN 1^{H} (300 MHz, CDCl₃) : δ = 0,88 (t, 3H, J = 6,60 Hz, C*H*₃ chaîne aliphatique) ; 1,27-1,44 (m large, 10H, C*H₂* chaîne aliphatique et N-CH₂-CH₂-C*H₂*) ; 2,02 (qui, 2H, J = 6,84 Hz, N-CH₂-C*H₂*) ; 4,03 (s, 2H, C*H₂* fluorène) ; 4,59 (t, 2H, J = 7,41 Hz, N-C*H₂*) ; 7,34-7,46 (m, 3H, C*H* fluorène ou imidazolium) ; 7,58-7,63 (m, 2H, C*H* fluorène ou imidazolium) ; 7,73 et 7,76 (dd, 1H, J = 2,19 et 7,89 Hz, C*H* fluorène ou imidazolium) ; 7,81 et 7,83 (dd, 1H, J = 1,65 et 6,03 Hz, C*H* fluorène ou imidazolium) ; 7,93 (d, 1H, J = 8,22 Hz, C*H* fluorène ou imidazolium) ; 8,06 (d, 1H, J = 1,92 Hz, C*H* fluorène ou imidazolium) ; 10,80 (s, 1H, N-C*H-*N)*.* RMN ¹³C (75 MHz, CDCl₃) : δ = 13,83 (C-*C*H₃ chaîne aliphatique) ; 22,35 ; 26,05 ; 28,77 ; 28,82 ; 30,15 ; 31,46 (*C*H₂ chaîne aliphatique); 36,82 (*C*H₂ fluorène) ; 50,41 (N-*C*H₂) ; 118,74 ; 120,36 ; 120,57 ; 120,96 ; 121,13 ; 123,10 ; 124,99 ; 126,95; 127,75 (*C*H fluorène ou imidazolium); 132,33 (*C* quaternaire) ; 134,55 (*C*H fluorène ou imidazolium); 139,37 ; 143,40 ; 143,43 ; 145,19 (*C* quaternaire). vₘₐₓ/cm⁻¹ 3067 (C-H aromatique) ; 2923 et 2854 (C-H aliphatique) ; 1556 (C=C aromaticque). UV/Vis (CH₂Cl₂) : λₘₐₓ (ε L.mol⁻¹.cm⁻¹) = 363 nm (1000) ; 303 nm (21400) ; 281 nm (23800) ; 228 nm (21100). Analyse élémentaire pour C₂₄H₂₉IN₂ ; Calculée : C 61,02 ; H 6,19 ; N 5,93 %. Trouvée : C 60,99 ; H 6,25 ; N 5,74 %.

### Bis(trifluorométhylsulfonyl)amidure de 1-(9H-fluorèn-2-yl)-3-octyl-2H-imidazol-3-ium (43).

La procédure générale avec **33** (0,870 g ; 2,05 mmol) et du bis(trifluorométhylsulfonyl)amidure de lithium (0,851 g ; 2,96 mmol) permet d'obtenir **43** avec un rendement de 89 % (1,141 g ; 1,82 mmol). RMN ¹H (300 MHz, CDCl₃) : δ = 0,89 (t, 3H, J = 6,33 Hz, C*H*₃ chaîne aliphatique); 1,29-1,38 (m large, 10H, C*H₂* chaîne aliphatique et N-CH₂-CH₂-C*H₂*) ; 1,97 (qui, 2H, J = 7,95 Hz, N-CH₂-C*H₂*) ; 4,03 (s, 2H, C*H₂* fluorène), 4,37 (t, 2H, J = 7,71 Hz, N-C*H₂*) ; 7,38-7,47 (m, 3H, C*H* fluorène ou imidazolium) ; 7,53-7,63 (m, 3H, C*H* fluorène ou imidazolium) ; 7,82-7,86 (m, 2H, C*H* fluorène ou imidazolium) ; 7,94 (d, 1H, J = 8,22 Hz, C*H* fluorène ou imidazolium) ; 9,24 (s, 1H, N-CH-N). RMN ¹³C (75 MHz, CDCl₃) : δ = 13,91 (C-*C*H₃ chaîne aliphatique) ; 22,46 ; 26,07 ; 28,75 ; 28,86 ; 30,01 ; 31,54 (*C*H₂ chaîne aliphatique) ; 36,84 (*C*H₂ fluorène) ; 50,60 (N-*C*H₂) ; 119,85 (qua, J = 319,10 Hz, (CF₃SO₂)₂N⁻) ; 118,82 ; 120,52 ; 120,67; 121,17 ; 121,79 ; 123,12 ; 125,18 ; 127,14 ; 128,01 (*C*H fluorène ou imidazolium) ; 132,49 (*C* quaternaire) ; 133,68 (CH fluorène ou imidazolium) ; 139,54 ; 143,65 ; 143,96 ; 145,61 (*C* quaternaire). vₘₐₓ/cm⁻¹ 3024 (C-H aromatique) ; 2931 et 2861 (C-H aliphatique) ; 1548 (C=C aromatique) ; 1343 et 1130 ((CF₃SO₂)₂N⁻). UV/Vis (CH₂Cl₂) : λₘₐₓ (ε L.mol⁻¹.cm⁻¹) = 303 nm (19700) ; 281 nm (21500) ; 227 nm (10400). Analyse élémentaire pour C₂₆H₂₉F₆N₃O₄S₂ ; Calculée : C 49,91 ; H 4,67 ; N 6,72 %. Trouvée : C 49,95 ; H 4,77 ; N 6,71 %.

### Tétrafluoroborate de 1-(9-méthyl-carbazol-3-yl)-3-octyl-2H-imidazol-3-ium (44).

La procédure générale avec **34** (0,459 g ; 1,04 mmol) et du tétrafluoroborate de potassium (0,255 g ; 2,03 mmol) permet d'obtenir **44** avec un rendement de 85 % (0,396 g ; 8,85 mmol). RMN ¹H (300 MHz, CDCl₃) : δ = 0,88 (t, 3H, J = 6,57 Hz, C*H*₃ chaîne aliphatique) ; 1,27-1 ;36 (m large, 10H, C*H₂* chaîne aliphatique et N-CH₂-CH₂-C*H₂*) ; 1,94 (qui, 2H, J = 7,68 Hz, N-CH₂-C*H₂*) ; 3,79 (s, 3H, C*H*₃ carbazole) ; 4,36 (t, 2H, J = 7,41 Hz, N-C*H₂*) ; 7,29-7,34 (m, 1H, C*H* carbazole ou imidazolium); 7,39-7,44 (m, 3H, C*H* carbazole ou imidazolium) ; 7,53-7,62 (m, 3H, C*H* carbazole ou imidazolium) ; 8,16 (d, 1H, J = 7,68 Hz, C*H* carbazole ou imidazolium) ; 8,25 (d, 1H, J = 2,22 Hz, C*H* carbazole ou imidazolium) ; 9,30 (s, 1H, N-C*H*-N). RMN ¹³C (75 MHz, CDCl₃) : δ= 13,96 (C-*C*H₃ chaîne aliphatique) ; 22,49 ; 26,17 ; 28,81 (*C*H₂ chaîne aliphatique) ; 28,84 (*C*H₃ carbazole) ; 28,95 ; 30,06 ; 31,60 (*C*H₂ chaîne aliphatique) ; 50,21 (N-*C*H₂) ; 108,80 ; 109,32 ; 113,28 ; 118,38; 119,53; 120,79; 121,55 (*C*H carbazole ou imidazolium); 121,65 (*C* quaternaire) ; 122,65 (*C*H carbazole ou imidazolium) ; 122,59 ; 125,93 (C quaternaire) ; 126,87 ; 133,59 (*C*H carbazole ou imidazolium) ; 140,37 ; 141,44 (*C* quaternaire). vₘₐₓ/cm⁻¹ 3053 (C-H aromatique) ; 2924 et 2856 (CH aliphatique) ; 1560 (C=C aromatique) ; 1024 (BF₄⁻). UV/Vis (CH₂Cl₂) : λₘₐₓ (ε L.mol⁻¹.cm⁻¹) = 350 nm (2700) ; 334 nm (3700) ; 278 nm (29500) ; 239 nm (29200). Analyse élémentaire pour C₂₄H₃₀BF₄N₃ ; Calculée : C 64,44 ; H, 6,76 ; N 9,39 %. Trouvée : C 64,41 ; H 6,87 ; N 8,91 %.

### Hexafluorophosphate 1-(9-méthyl-carbazol-3-yl)-3-octyl-2H-imidazol-3-ium (45).

La procédure générale avec **34** (0,456 g ; 1,04 mmol) et de l'hexafluorophosphate de potassium (0,383 g ; 2,08 mmol) permet d'obtenir **45** avec un rendement de 95 % (0,465 g ; 0,98 mmol). RMN ¹H (300 MHz, CDCl₃) : δ = 0,88 (t, 3H, J = 6,87 Hz, C*H*₃ chaîne aliphatique) ; 1,27-1,34 (m large, 10H, C*H₂* chaîne aliphatique et N-CH₂-CH₂-C*H₂*) ; 1,93 (qui, 2H, J = 6,84 Hz, N-CH₂-C*H₂*) ; 3,76 (s, 3H, C*H*₃ carbazole) ; 4,29 (t, 2H, J = 7,41 Hz, N-C*H₂*) ; 7,25-7,30 (m, 1H, C*H* carbazole ou imidazolium) ; 7,38-7,41 (m, 3H, C*H* carbazole ou imidazolium) ; 7,49-7,57 (m, 3H, C*H* carbazole ou imidazolium) ; 8,12 (d, 1H, J = 7,68 Hz, C*H* carbazole ou imidazolium) ; 8,18 (d, 1H, J = 2,19 Hz, C*H* carbazole ou imidazolium) ; 8,93 (s large, 1H, N-C*H*-N)*.* RMN ¹³C (75 MHz, CDCl₃) : δ= 13,97 (C-*C*H₃ chaîne aliphatique); 22,50 ; 26,16 ; 28,79 (*C*H₂ chaîne aliphatique); 28,81 (*C*H₃ carbazole) ; 28,94 ; 29,91 ; 31,60 (*C*H₂ chaîne aliphatique) ; 50,30 (N-*C*H₂) ; 108,86 ; 109,33 ; 113,39 ; 118,43 ; 119,59 ; 120,65 (*C*H carbazole ou imidazolium) ; 121,57 (*C* quaternaire); 121,67; 122,53 (*C*H carbazole ou imidazolium); 122,88; 125,82 (*C* quaternaire) ; 126,94 ; 133,20 ; 133,20 (*C*H carbazole ou imidazolium) ; 140,42 ; 141,45 (*C* quaternaire). vₘₐₓ/cm⁻¹ 3055 (C-H aromatique) ; 1423 (C=C aromatique) ; 847 (PF₆⁻). UV/Vis (CH₂Cl₂) : λₘₐₓ (ε L.mol⁻¹.cm⁻¹) = 350 nm (2700) ; 335 nm (3800) ; 280 nm (28700) ; 239 nm (29100). Analyse élémentaire pour C₂₄H₃₀F₆N₃P ; Calculée : C 57,03 ; H 5,98 ; N 8,31 %. Trouvée : C 57,02 ; H 5,94 ; N 8,37 %.

### Iodure de 1-(9-méthyl-carbazol-3-yl)-3-octyl-2H-imidazol-3-ium (46).

La procédure générale avec **34** (0,454 g ; 1,03 mmol) et de l'iodure de potassium (0,403 g ; 2,43 mmol) permet d'obtenir **46** avec un rendement de 72 % (0,363 g ; 0,74 mmol). RMN ¹H (300 MHz, CDCl₃) : δ = 0,89 (t, 3H, J = 6,87 Hz, C*H*₃ chaîne aliphatique) ; 1,28-1,44 (m large, 10H, C*H₂* chaîne aliphatique et N-CH₂-CH₂-C*H₂*) ; 2,02 (qui, 2H, J = 7,11 Hz, N-CH₂-C*H₂*) ; 3,87 (s, 3H, C*H*₃ carbazole) ; 4,57 (t, 2H, J = 7,41 Hz, N-C*H₂*) ; 7,31-7,60 (m, 4H, C*H* carbazole ou imidazolium) ; 7,68 (t, 1H, J = 1,92 Hz, C*H* carbazole ou imidazolium) ; 7,84 et 7,87 (dd, 1H, J = 2,19 et 8,79 Hz, C*H* carbazole ou imidazolium) ; 8,23 (d, 1H, J = 7,95 Hz, C*H* carbazole ou imidazolium) ; 8,49 (d, 1H, J = 2,19 Hz, C*H* carbazole ou imidazolium) ; 10,70 (s, 1H, N-C*H*-N)*.* RMN ¹³C (75 MHz, CDCl₃) : δ= 13,86 (C-*C*H₃ chaîne aliphatique) ; 22,38 ; 26,08 ; 28,79 ; 28,85 (*C*H2 chaîne aliphatique) ; 29,28 (*C*H₃ carbazole) ; 30,18; 31,49 (*C*H₂ chaîne aliphatique); 50,21 (N-*C*H₂) ; 108,78; 109,30 ; 113,78; 118,51 ; 119,47; 121,19 ; 121,37 (*C*H carbazole ou imidazolium); 121,65 (*C* quaternaire) ; 122.59 (*C*H carbazole ou imidazolium); 122,84; 125,79 (*C* quaternaire); 126,84; 134,24 (*C*H carbazole ou imidazolium); 140,28; 141,37 (*C* quaternaire). vₘₐₓ/cm⁻¹ 3049 (C-H aromatique) ; 2923 et 2853 (C-H aliphatique) ; 1556 (C=C aromatique). UV/Vis (CH₂Cl₂) : λₘₐₓ (ε L.mol⁻¹.cm⁻¹) = 352 nm (3300) ; 335 nm (4200) ; 281 nm (31500) ; 239 nm (41700). Analyse élémentaire pour C₂₄H₃₀IN₃ ; Calculée : C 59,14 ; H 6,20 ; N 8,62 %. Trouvée : C 59,20 ; H 6,25 ; N 8,37 %.

### Bis(trifluorométhylsulfonyl)amidure de 1-(9-méthyl-carbazol-3-yl)-3-octyl-2H-imidazol-3-ium (47).

La procédure générale avec **34** (0,455 g ; 1,03 mmol) et du bis(trifluorométhylsulfonyl)amidure de lithium (0,517 g ; 1,80 mmol) permet d'obtenir **47** avec un rendement de 94 % (0,621 g ; 0,97 mmol). RMN ¹H (300 MHz, CDCl₃) : δ = 0,89 (t, 3H, J = 6,87 Hz, C*H*₃ chaîne aliphatique) ; 1,29-1,38 (m large, 10H, C*H₂* chaîne aliphatique et N-CH₂-CH₂-C*H₂*) ; 1,98 (qui, 2H, J = 7,68 Hz, N-CH₂-C*H₂*) ; 3,90 (s, 3H, C*H*₃ carbazole) ; 4,37 (t, 2H, J = 7,68 Hz, N-C*H₂*) ; 7,30-7,38 (m, 1H, C*H* carbazole ou imidazolium) ; 7,43-7,65 (m, 6H, C*H* carbazole ou imidazolium) ; 8,15 (d, 1H, J = 7,95 Hz, C*H* carbazole ou imidazolium) ; 8,24 (d, 1H, J = 2,19 Hz, C*H* carbazole ou imidazolium) ; 9,15 (s, 1H, N-C*H*-N)*.* RMN ¹³C (75 MHz, CDCl₃) : δ= 13,93 (C-*C*H₃ chaîne aliphatique) ; 22,47 ; 26,10 ; 28,77 ; 28,89 (*C*H₂ chaîne aliphatique) ; 29,08 (*C*H₃ carbazole) ; 30,05 ; 31,57 (*C*H₂ chaîne aliphatique); 50,45 (N-*C*H₂) ; 109,01 ; 109,64; 113,78; 118,97; 119,82 (*C*H carbazole ou imidazolium); 119,91 (qua, J = 319,65 Hz, (CF₃SO₂)₂N⁻) ; 120,82 (*C*H carbazole ou imidazolium) ; 121,74 (*C* quaternaire) ; 122,14; 122,75 (*C*H carbazole ou imidazolium) ; 123,22 ; 125,93 (*C* quaternaire) ; 127,15 ; 133,48 (*C*H carbazole ou imidazolium); 140,82; 141,71 (*C* quaternaire). vₘₐₓ/cm⁻¹ 3082 (C-H aromatique) ; 2933 et 2859 (C-H aliphatique) ; 1553 (C=C aromatique) ; 1354 et 1134 ((CF₃SO₂)₂N⁻). UV/Vis (CH₂Cl₂) : λₘₐₓ (ε L.mol⁻¹.cm⁻¹) = 350 nm (2800) ; 335 nm (4000) ; 280 nm (28700) ; 239 nm (29500). Analyse élémentaire pour C₂₆H₃₀F₆N₄O₄S₂ ; Calculée : C 48,74 ; H, 4,72 ; N 8,75 %. Trouvée : C 48,58 ; H, 4,74 ; N 8,64 %.

### Tétrafluoroborate 1 -(anthracèn-9-yl)-3-octyl-2H-imidazol-3-ium (48).

La procédure générale avec **35** (0,166 g ; 0,38 mmol) et du tétrafluoroborate de potassium (0,195 g ; 1,55 mmol) permet d'obtenir **48** avec un rendement de 75 % (0,126 g ; 0,28 mmol). RMN ¹H (300 MHz, CDCl₃) : δ = 0,90 (t, 3H, J = 6,57 Hz, C*H*₃ chaîne aliphatique) ; 1,30-1,43 (m large, 10H, C*H₂* chaîne aliphatique et N-CH₂-CH₂-C*H₂*) ; 2,08 (qui, 2H, J = 7,41 Hz, N-CH₂-C*H₂*) ; 4,62 (t, 2H, J = 7,41 Hz, N-C*H₂*) ; 7,37 (d, 2H, J = 8,49 Hz, C*H* anthracène ou imidazolium); 7,49 (t, 1H, J = 1,65 Hz, C*H* anthracène ou imidazolium) ; 7,57-7,68 (m, 4H, C*H* anthracène ou imidazolium) ; 7,83 (t, 1H, J = 1,65 Hz, C*H* anthracène ou imidazolium) ; 8,13-8,16 (m, 2H, C*H* anthracène ou imidazolium) ; 8,75 (s, 1H, C*H* anthracène ou imidazolium) ; 9,02 (s, 1H, N-C*H*-N)*.* RMN ¹³C (75 MHz, CDCl₃) : δ= 14,00 (C-*C*H₃ chaîne aliphatique) ; 22,53 ; 26,14 ; 28,85 ; 29,01 ; 30,07 ; 31,59 (*C*H₂ chaîne aliphatique) ; 50,69 (N-*C*H₂) ; 120,30 ; 123,74 (*C*H anthracène ou imidazolium) ; 124,50 (*C* quaternaire) ; 125,50 ; 126,25 (*C*H anthracène ou imidazolium) ; 127,54 (*C* quaternaire) ; 128,73 ; 129,26 (*C*H anthracène ou imidazolium) ; 130,78 (*C* quaternaire) ; 131,10 ; 137,66 (*C*H anthracène ou imidazolium). vₘₐₓ/cm⁻¹ 3067 (C-H aromatique) ; 2927 et 2856 (C-H aliphatique) ; 1543 (C=C aromatique) ; 1071 (BF₄⁻). UV/Vis (CH₂Cl₂) : λₘₐₓ (ε L.mol⁻¹.cm⁻¹) = 387 nm (6600) ; 370 nm (8200) ; 353 nm (6200) ; 305 nm (1500) ; 254 nm (163500). Analyse élémentaire pour C₂₅H₂₉BF₄N₂ ; Calculée : C 67,58 ; H 6,58 ; N 6,30 %. Trouvée : C 67,44 ; H 6,59 ; N 6,34 %.

### Hexafluorophosphate 1-(anthracèn-9-yl)-3-octyl-2H-imidazol-3-ium (49).

La procédure générale avec **35** (0,165 g ; 0,38 mmol) et de l'hexafluorophosphate de potassium (0,171 g ; 0,93 mmol) permet d'obtenir **49** avec un rendement de 63 % (0,119 g ; 0,24 mmol). RMN ¹H (300 MHz, CDCl₃) : δ = 0,90 (t, 3H, J = 6,33 Hz, C*H*₃ chaîne aliphatique) ; 1,30-1,43 (3 large, 10H, C*H₂* chaîne aliphatique et N-CH₂-CH₂-C*H₂*) ; 2,07 (qui, 2H, J = 7,68 Hz, N-CH₂-C*H₂*) ; 4,56 (t, 2H, J = 7,68 Hz, N-C*H₂*) ; 7,37 (d, 2H, J = 8,76 Hz, C*H* anthracène ou imidazolium); 7,49 (t, 1H, J = 1,65 Hz, C*H* anthracène ou imidazolium) ; 7,57-7,68 (m, 4H, C*H* anthracène ou imidazolium) ; 7,80 (t, 1H, J = 1,65 Hz, C*H* anthracène ou imidazolium) ; 8,14 (d, 2H, J = 8,52 Hz, C*H* anthracène ou imidazolium) ; 8,69 (s, 1H, N-CH-N) ; 8,75 (s, 1H, C*H* anthracène ou imidazolium). RMN ¹³C (75 MHz, CDCl₃) : δ = 13,99 (C-*C*H₃ chaîne aliphatique) ; 22,53 ; 26,13 ; 28,79 ; 28,98 ; 29,81 ; 31,57 (*C*H₂ chaîne aliphatique) ; 50,75 (N-*C*H₂) ; 120,22 ; 123,64 (*C*H anthracène ou imidazolium) ; 124,29 (*C* quaternaire) ; 125,59 ; 126,28 (*C*H anthracène ou imidazolium) ; 127,50 (*C* quaternaire) ; 128,70 ; 129,37 (CH anthracène ou imidazolium) ; 130,73 (*C* quaternaire) ; 131,19 ; 136,89 (*C*H anthracène ou imidazolium). vₘₐₓ/cm⁻¹ 3063 (C-H aromatique) ; 2925 et 2857 (C-H aliphatique) ; 1548 (C=C aromatique) ; 878 (PF₆⁻). UV/Vis (CH₂Cl₂) : λₘₐₓ (ε L.mol⁻¹.cm⁻¹) = 387 nm (5300) ; 370 nm (7000) ; 352 nm (5400) ; 254 nm (163600). Analyse élémentaire pour C₂₅H₂₉F₆N₂P ; Calculée : C 59,76 ; H 5,82 ; N 5,58 %. Trouvée : C 59,93 ; H 5,92 ; N 5,49 %.

### Iodure de 1-(anthracèn-9-yl)-3-octyl-2H-imidazol-3-ium (50).

La procédure générale avec **35** (0,167 g ; 0,38 mmol) et de l'iodure de potassium (0,150 g ; 0,91 mmol) permet d'obtenir **50** avec un rendement de 71 % (0,131 g ; 0,27 mmol). RMN ¹H (300 MHz, CDCl₃) : δ = 0,90 (t, 3H, J = 6,30 Hz, C*H*₃ chaîne aliphatique) ; 1,31-1 ;50 (m large, 10H, C*H₂* chaîne aliphatique et N-CH₂-CH₂-C*H₂*) ; 2,14 (qui, 2H, J = 7,68 Hz, N-CH₂-C*H₂*) ; 4,93 (t, 2H, J = 7,41 Hz, N-C*H₂*) ; 7,42 (d, 2H, J = 8,52 Hz, C*H* anthracène ou imidazolium) ; 7,50 (t, 1H, J = 1,65 Hz, C*H* anthracène ou imidazolium) ; 7,59-7,69 (m, 4H, C*H* anthracène ou imidazolium); 7,94 (t, 1H, J = 1,65 Hz, C*H* anthracène ou imidazolium) ; 8,15-8,18 (m, 2H, C*H* anthracène ou imidazolium) ; 8,77 (s, 1H, C*H* anthracène ou imidazolium) ; 10,09 (s, 1H, N-C*H*-N)*.* RMN ¹³C (75 MHz, CDCl₃) : δ = 13,91 (C-*C*H₃ chaîne aliphatique) ; 22,42 ; 26,02 ; 28,83 ; 28,92 ; 30,31 ; 31,49 (*C*H₂ chaîne aliphatique) ; 50,85 (N-*C*H₂) ; 120,41 ; 124,18 (*C*H anthracène ou imidazolium) ; 124,33 (*C* quaternaire) ; 125,21 ; 126,20 (*C*H anthracène ou imidazolium) ; 127,42 (*C* quaternaire) ; 128,70 ; 129,26 (*C*H anthracène ou imidazolium) ; 130,73 (C quaternaire) ; 131,07 ; 137,89 (*C*H anthracène ou imidazolium). vₘₐₓ/cm⁻¹ 3051 (C-H aromatique) ; 2924 et 2852 (C-H aliphatique) ; 1543 (C=C aromatique). UV/Vis (CH₂Cl₂) : λₘₐₓ (ε L.mol⁻¹.cm⁻¹) = 387 nm (6100) ; 370 nm (7700) ; 353 nm (6000) ; 322 nm (2100) ; 254 nm (159100). Analyse élémentaire pour C₂₅H₂₉IN₂ ; Calculée : C 61,99 ; H 6,03 ; N 5,78 %. Trouvée : C 61,97 ; H 6,20 ; N 5,51 %.

### Bis(trifluorométhylsulfonyl)amideure de 1-(anthracèn-9-yl)-3-octyl-2H-imidazol-3-ium (51).

La procédure générale avec **35** (0,165 g ; 0,38 mmol) et du bis(trifluorométhylsulfonyl)amidure de lithium (0,192 g ; 0,67 mmol) permet d'obtenir **51** avec un rendement de 65 % (0,155 g ; 0,24 mmol). RMN ¹H (300 MHz, CDCl₃) : δ = 0,90 (t, 3H, J = 6,57 Hz, C*H*₃ chaîne aliphatique); 1,31-1,49 (m large, 10H, C*H₂* chaîne aliphatique et N-CH₂-CH₂-C*H₂*) ; 2,10 (qui, 2H, J = 7,14 Hz, N-CH₂-C*H₂*) ; 4,58 (t, 2H, J = 7,41 Hz, N-C*H₂*) ; 7,36 (d, 2H, J = 8,76 Hz, C*H* anthracène ou imidazolium) ; 7,54 (t, 1H, J = 1,65 Hz, C*H* anthracène ou imidazolium); 7,60-7,70 (m, 4H, C*H* anthracène ou imidazolium) ; 7,83 (t, 1H, J = 1,65 Hz, C*H* anthracène ou imidazolium) ; 8,16-8,19 (m, 2H, C*H* anthracène ou imidazolium) ; 8,78 (s, 1H, C*H* anthracène ou imidazolium) ; 8,90 (s, 1H, N-C*H*-N)*.* RMN ¹³C (75 MHz, CDCl₃) : δ = 13,98 (C-*C*H₃ chaîne aliphatique) ; 22,53 ; 26,14 ; 28,78 ; 28,98 ; 29,98 ; 31,56 (*C*H₂ chaîne aliphatique) ; 50,96 (N-*C*H₂) ; 119,69 (qua, J = 319,65 Hz, (*C*F₃SO₂)₂N⁻) ; 120,09 ; 123,68 (*C*H anthracène ou imidazolium) ; 124,27 (*C* quaternaire) ; 125,74 ; 126,40 (*C*H anthracène ou imidazolium) ; 127,63 (*C* quaternaire) ; 128,86 ; 129,50 (*C*H anthracène ou imidazolium) ; 130,89 (C quaternaire) ; 131,36 ; 137,42 (*C*H anthracène ou imidazolium). vₘₐₓ/cm⁻¹ 3073 (C-H aromatique) ; 2928 et 2858 (C-H aliphatique) ; 1549 (C=C aromatique) ; 1347 et 1138 (N(SO₂CF₃)₂⁻). UV/Vis (CH₂Cl₂) : λₘₐₓ (ε L.mol⁻¹.cm⁻¹) = 387 nm (5900) ; 370 nm (7700) ; 351 nm (5900) ; 254 nm (165600). Analyse élémentaire pour C₂₅H₂₉N₂.(N(SO2CF₃)₂)_{0,975}._{0,025}Br ; Calculée : C 51,16 ; H 4,62 ; N 6,59 %. Trouvée : C 51,18 ; H 4,77 ; N 6,40 %.

### Imidazolium 52 à 71 avec une chaîne carbonée en C₁₂.

### Prodécure générale pour la synthèse des imidazoliums (N-alkylation).

Un mélange de dérivé imidazole et de 1-bromododécane a été chauffé à 110 °C dans un ballon sous vide statique pendant 12 heures ou à l'aide d'un four à micro-ondes pendant 140 minutes dans un tube fermé. Le sel d'imidazolium correspondant a été purifié par chromatographie flash (gel de silice, dichlorométhane à dichlorométhane/méthanol : 95/5).

### Bromure de 3-dodécyl-1-(naphtalèn-1-yl)-2H-imidazol-3-ium (52).

La procédure générale avec **28** (1,288 g ; 6,63 mmol) et du 1-bromododécane (1,999 g ; 8,02 mmol) permet d'obtenir **52** avec un rendement de 86 % (2,516 g ; 5,67 mmol). RMN ¹H (300 MHz, CDCl₃) : δ = 0,89 (t, 3H, J = 6,33 Hz, C*H*₃ chaîne aliphatique) ; 1,27-1,50 (m large, 18H, C*H*₂ chaîne aliphatique et N-CH₂-CH₂-C*H₂*) ; 2,07 (qui, 2H, J = 7,11 Hz, N-CH₂-C*H₂*) ; 4,78 (t, 2H, J = 7,11 Hz, N-C*H₂*) ; 7,49-7,68 (m, 6H, C*H* naphtalène ou imidazolium) ; 7,93 (d, 1H, J = 7,41 Hz, C*H* naphtalène ou imidazolium) ; 8,01-8,04 (m, 1H, C*H* naphtalène ou imidazolium) ; 8,10 (d, 1H, J = 8,52 Hz, C*H* naphtalène ou imidazolium) ; 10,68 (s, 1H, N-C*H-*N)*.* RMN ¹³C (75 MHz, CDCl₃) : δ = 13,94 (C-*C*H₃ chaîne aliphatique) ; 22,50 ; 26,18 ; 28,95 ; 29,16 ; 29,28 ; 29,38 ; 29,44 ; 29,46 ; 30,29 ; 31,74 (*C*H₂ chaîne aliphatique) ; 50,48 (N-*C*H₂) ; 120,52 ; 123,00 (*C*H naphtalène ou imidazolium) ; 123,03 (*C* quaternaire) ; 123,94 ; 124,66 ; 125,21 ; 127,47 ; 128,65 ; 128,73 (CH naphtalène ou imidazolium) ; 130,52 (C quaternaire) ; 131,42 (CH naphtalène ou imidazolium) ; 134,03 (C quaternaire) ; 137,72 (CH naphtalène ou imidazolium). νₘₐₓ/cm⁻¹ 3059 (C-H aromatique); 2921 et 2849 (C-H aliphatique) ; 1510 (C=C aromatique). UV/Vis (CH₂Cl₂) : λₘₐₓ (ε L.mol⁻¹.cm⁻¹) = 283 nm (7100) ; 228 nm (35700). Analyse élémentaire pour C₂₅H₃₅BrN₂ ; Calculée : C 67,71 ; H 7,96 ; N 6,32 %. Trouvée : C 67,73 ; H 8,06 ; N 6,40 %.

### Bromure de 3-dodécyl-1-(9H-fluorèn-2-yl)-2H-imidazol-3-ium (53).

La procédure générale avec **29** (10,686 g ; 46,00 mmol) et du 1-bromododécane (44,979 g ; 180,47 mmol) permet d'obtenir **53** avec un rendement de 86 % (18,948 g ; 39,35 mmol). RMN ¹H (300 MHz, CDCl₃) : δ = 0,89 (t, 3H, J = 6,30 Hz, C*H*₃ chaîne aliphatique) ; 1,26-1,44 (m large, 18H, C*H*₂ chaîne aliphatique et N-CH₂-CH₂-C*H₂*) ; 2,02 (qui, 2H, J = 7,41 Hz, N-CH₂-C*H₂*) ; 4,02 (s, 2H, C*H*₂ fluorène) ; 4,64 (t, 2H, J = 7,41 Hz, N-C*H₂*) ; 7,37-7,46 (m, 3H, C*H* fluorène ou imidazolium) ; 7,59-7,61 (m, 2H, C*H* fluorène ou imidazolium) ; 7,73 et 7,76 (dd, 1H, J = 2,19 et 8,22 Hz, C*H* fluorène ou imidazolium) ; 7,83 (d, 1H, J = 7,14 Hz, C*H* fluorène ou imidazolium) ; 7,93 (d, 1H, J = 8,22 Hz, C*H* fluorène ou imidazolium) ; 8,05 (s, 1H, C*H* fluorène ou imidazolium) ; 11,29 (s, 1H, N-C*H-*N). RMN ¹³C (75 MHz, CDCl₃) : δ = 13,86 (C-*C*H₃ chaîne aliphatique) ; 22,42 ; 26,10 ; 28,87 ; 29,08 ; 29,19 ; 29,30 ; 29,35 ; 29,37 ; 30,23 ; 31,65 (*C*H₂ chaîne aliphatique) ; 36,79 (*C*H₂ fluorène) ; 50,22 (N-*C*H₂) ; 118,43 ; 120,28 ; 120,37 ; 120,91 ; 120,98; 122,94 ; 124,94 ; 126,91 ; 127,68 (*C*H fluorène ou imidazolium) ; 132,51 (*C* quaternaire) ; 135,29 (*C*H fluorène ou imidazolium) ; 139,40 ; 143,31 ; 143,34 ; 145,16 (*C* quaternaire). vₘₐₓ/cm⁻¹ 3041 (C-H aromatique) ; 2919 et 2851 (C-H aliphatique) ; 1556 (C=C aromatique). UV/Vis (CH₂Cl₂) : λₘₐₓ (ε L.mol⁻¹.cm⁻¹) = 304 nm (19700) ; 281 nm (22300) ; 227 nm (15600). Analyse élémentaire pour C₂₈H₃₇BrN₂ ; Calculée : C 69,84 ; H 7,75 ; N 5,82 %. Trouvée : C 69,87 ; H 7,82 ; N 5,89 %.

### Bromure 3-dodécyl-1-(9-méthylcarbazol-3-yl)-2H-imidazol-3-ium (54).

La procédure générale avec **30** (0,297 g ; 1,20 mmol) et du 1-bromododécane (1,708 g ; 6,85 mmol) permet d'obtenir **54** avec un rendement de 77 % (0,458 g ; 0,92 mmol). RMN ¹H (300 MHz, CDCl₃) : δ = 0,88 (t, 3H, J = 6,60 Hz, C*H*₃ chaîne aliphatique) ; 1,26-1,40 (m large, 18H, C*H*₂ chaîne aliphatique et N-CH₂-CH₂-C*H₂*) ; 2,02 (qui, 2H, J = 7,14 Hz, N-CH₂-C*H₂*) ; 3,88 (s, 3H, C*H*₃ carbazole) ; 4,62 (t, 2H, J = 7,14 Hz, N-C*H₂*) ; 7,31-7,33 (m, 1H, C*H* carbazole ou imidazolium) ; 7,39-7,67 (m, 5H, C*H* carbazole ou imidazolium) ; 7,88 (d, 1H, J = 9,06 Hz, C*H* carbazole ou imidazolium) ; 8,21 (d, 1H, J = 7,68 Hz, C*H* carbazole ou imidazolium) ; 8,47 (d, 1H, J = 2,22 Hz, C*H* carbazole ou imidazolium) ; 11,15 (s, 1H, N-CH-N). RMN ¹³C (75 MHz, CDCl₃) : δ = 13,92 (C-*C*H₃ chaîne aliphatique) ; 22,48 ; 26,16 ; 28,91 (*C*H₂ chaîne aliphatique) ; 29,11 (*C*H₃ carbazole) ; 29,14 ; 29,25 ; 29,36 ; 29,41 ; 29,43 ; 30,26 ; 31,71 (*C*H₂ chaîne aliphatique) ; 50,10 (N-*C*H₂) ; 108,74 ; 109,23 ; 113,56 ; 118,46 ; 119,45 ; 121,03 ; 121,23 (CH carbazole ou imidazolium); 121,72 (*C* quaternaire); 122,41 (*C*H carbazole ou imidazolium); 122,89; 125,98 (*C* quaternaire); 126,81 ; 135,02 (*C*H carbazole ou imidazolium); 140,28; 141,42 (*C* quaternaire). vₘₐₓ/cm⁻¹ 3073 (C-H aromatique) ; 2917 et 2851 (C-H aliphatique) ; 1561 (C=C aromatique). UV/Vis (CH₂Cl₂) : λₘₐₓ (ε L.mol⁻¹.cm⁻¹) = 352 nm (3000) ; 335 nm (4000) ; 280 nm (31900) ; 239 nm (31700) ; 229 nm (28600). Analyse élémentaire pour C₂₈H₃₈BrN₃ ; Calculée : C 67,73 ; H 7,71 ; N 8,46 %. Trouvée : C 67,54 ; H 7,77 ; N 8,54 %.

### Bromure de 1-(anthracèn-9-yl)-3-dodécyl-2H-imidazol-3-ium (55).

La procédure générale avec **31** (0.417 g, 1.71 mmol) et du 1-bromododécane (2,969 g , 11,91 mmol) permet d'obtenir **55** avec un rendement 95 % (0,796 g ; 1,61 mmol). RMN ¹H (300 MHz, CDCl₃) : δ = 0,89 (t, 3H, J = 6,87 Hz, C*H*₃ chaîne aliphatique) ; 1,28-1,53 (m large, 18H, C*H*₂ chaîne aliphatique et N-CH₂-CH₂-C*H₂*) ; 2,13 (qui, 2H, J = 7,41 Hz, N-CH₂-C*H₂*) ; 4,97 (t, 2H, J = 7,41 Hz, N-C*H₂*) ; 7,36-7,40 (m, 2H, C*H* anthracène ou imidazolium) ; 7,47 (t, 1H, J = 1,65 Hz, C*H* anthracène ou imidazolium) ; 7,58-7,68 (m, 4H, C*H* anthracène ou imidazolium) ; 7,92 (s, 1H, C*H* anthracène ou imidazolium) ; 8,14-8,17 (m, 2H, C*H* anthracène ou imidazolium) ; 8,77 (s, 1H, C*H* anthracène ou imidazolium) ; 10,43 (s, 1H, N-CH-N). RMN ¹³C (75 MHz, CDCl₃) : δ = 13,96 (C-*C*H₃ chaîne aliphatique) ; 22,52 ; 26,13 ; 28,95 ; 29,18 ; 29,33 ; 29,38 ; 29,46 ; 29,49 ; 30,40 ; 31,75 (*C*H₂ chaîne aliphatique) ; 50,63 (N-*C*H₂) ; 120,35 ; 123,87 (*C*H anthracène ou imidazolium) ; 124,55 (*C* quaternaire) ; 125,15 ; 126,17 (*C*H anthracène ou imidazolium) ; 127,50 (C quaternaire) ; 128,72 ; 129,15 (*C*H anthracène ou imidazolium); 130,77 (*C* quaternaire); 131,00; 138,62 (*C*H anthracène ou imidazolium). vₘₐₓ/cm⁻¹ 3043 (C-H aromatique); 2916 et 2849 (C-H aliphatique) ; 1466 (C=C aromatique). UV/Vis (CH₂Cl₂) : λₘₐₓ (ε L.mol⁻¹.cm⁻¹) = 387 nm (6500) ; 369 nm (7500) ; 352 nm (6000) ; 255 nm (161300). Analyse élémentaire pour C₂₉H₃₇BrN_{2.1,02}H₂O ; Calculée : C 68,04 ; H 7,69 ; N 5,47 %. Trouvée : C 68,04 ; H 7,59 ; N 5,51 %.

### Procédure générale pour la metathèse anionique - échange d'anion.

Un mélange de bromure d'imidazolium dissout dans du dichlorométhane et un mélange du sel correspondant dans de l'eau sont agités ensemble pendant 48 heures. La phase organique est extraite avec du dichlorométhane, séchée sur du chlorure de calcium puis filtrée. Le filtrat est ensuite purifié par chromatographie flash (gel de silice, dichlorométhane à dichlorométhane/méthanol: 95/5) pour obtenir un produit pur.

### Tétrafluoroborate de 3-dodécyl-1-(naphtalèn-1-yl)-2H-imidazol-3-ium (56).

La procédure générale avec **52** (0,819 g ; 1,85 mmol) et du tétrafluoroborate de potassium (0,630 g ; 5,00 mmol) permet d'obtenir **56** avec un rendement de 53 % (0,437 g ; 0,97 mmol). RMN ¹H (300 MHz, CDCl₃) : δ = 0,89 (t, 3H, J = 6,84 Hz, C*H*₃ chaîne aliphatique) ; 1,27-1,40 (m large, 18H, C*H*₂ chaîne aliphatique et N-CH₂-CH₂-C*H*₂) ; 2,02 (qui, 2H, J = 7,41 Hz, N-CH₂-C*H*₂) ; 4,49 (t, 2H, J = 7,41 Hz, N-C*H*₂) ; 7,47-7,51 (m, 2H, C*H* naphtalène ou imidazolium) ; 7,57-7,67 (m, 4H, C*H* naphtalène ou imidazolium) ; 7,80 (d, 1H, J = 7,41, C*H* naphtalène ou imidazolium) ; 8,01-8,04 (m, 1H, C*H* naphtalène ou imidazolium) ; 8,10 (d, 1H, J = 8,22 Hz, *CH* naphtalène ou imidazolium) ; 9,10 (s, 1H, N-*C*H-N). RMN ¹³C (75 MHz, CDCl₃) : δ = 14,00 (C-*C*H₃ chaîne aliphatique) ; 22,58 ; 26,18 ; 28,91 ; 29,24 ; 29,33 ; 29,44 ; 29,53 ; 29,54 ; 29,98 ; 31,81 (*C*H₂ chaîne aliphatique) ; 50,50 (N-CH₂) ; 120,54 ; 123,04 ; 124,38 ; 124,57 ; 125,19 ; 127,51 (*C*H naphtalène ou imidazolium) ; 127,56 (*C* quaternaire) ; 128,68 ; 128,73 (*C*H naphtalène ou imidazolium) ; 130,54 (*C* quaternaire); 131,47 (*C*H naphtalène ou imidazolium); 134,01 (*C* quaternaire) ; 136.46 (*C*H naphtalène or imidazolium). vₘₐₓ/cm⁻¹ 3055 (C-H aromatique) ; 2929 et 2857 (C-H aliphatique) ; 1422 (C=C aromatique) ; 1053 (BF₄⁻). UV/Vis (CH₂Cl₂) : λₘₐₓ (ε L.mol⁻¹.cm⁻¹) = 283 nm (7100); 229 nm (26700). Analyse élémentaire pour C₂₅H₃₅BF₄N₂ ; Calculée : C 66,67 ; H 7,83 ; N 6,22 %. Trouvée : C 66,50 ; H 7,98 ; N 6,28 %.

### Hexafluorophosphate de 3-dodécyl-1-(naphtalèn-1-yl)-2H-imidazol-3-ium (57).

La procédure générale avec **52** (0,833 g ; 1,88 mmol) et de l'hexafluorophosphate de potassium (0,812 g ; 4,41 mmol) permet d'obtenir **57** avec un rendement de 71 % (0,676 g ; 1,51 mmol). RMN ¹H (300 MHz, CDCl₃) : δ = 0,89 (t, 3H, J = 6,33 Hz, C*H*₃ chaîne aliphatique) ; 1,27-1,39 (m large, 18H, C*H*₂ chaîne aliphatique et N-CH₂-CH₂-C*H*₂) ; 2,01 (qui, 2H, J = 7,41 Hz, N-CH₂-C*H*₂) ; 4,42 (t, 2H, J = 7,41 Hz, N-C*H*₂) ; 7,45-7,50 (m, 2H, C*H* naphtalène ou imidazolium) ; 7,56-7,68 (m, 4H, C*H* naphtalène ou imidazolium) ; 7,74 (d, 1 H, J = 7,41 Hz, C*H* naphtalène ou imidazolium) ; 7,99-8,04 (m, 1 H, C*H* naphtalène ou imidazolium) ; 8,10 (d, 1H, J = 8,49 Hz, C*H* naphtalène ou imidazolium) ; 8,77 (s, 1H, N-CH-N). RMN ¹³C (75 MHz, CDCl₃) : δ = 14,05 (C-CH₃ chaîne aliphatique) ; 22,63 ; 26,22 ; 28,91 ; 29,28 ; 29,35 ; 29,47 ; 29,57 ; 29,81 ; 31,86 (*C*H₂ chaîne aliphatique) ; 50,63 (N-*C*H₂); 120,54; 122,91; 124,47; 124,61; 125,22; 127,63 (CH naphtalène ou imidazolium) ; 127,65 (*C* quaternaire) ; 128,70 ; 128,89 (*C*H naphtalène ou imidazolium) ; 130,46 (*C* quaternaire); 131,65 (*C*H naphtalène ou imidazolium); 134,05 (C quaternaire) ; 135,92 *(*CH naphtalène ou imidazolium). vₘₐₓ/cm⁻¹ 3163 (C-H aromatique) ; 2919 et 2852 (C-H aliphatique) ; 1514 (C=C aromatique) ; 820 (PF₆⁻). UV/Vis (CH₂Cl₂) : λₘₐₓ (ε L.mol⁻¹.cm⁻¹) = 283 nm (7100); 229 nm (27300). Analyse élémentaire pour C₂₅H₃₅F₆N₂P._{0,1} H₂O ; Calculée : C 58,84 ; H 6,95 ; N 5,49 %. Trouvée : C 58,83 ; H 7,07 ; N 5,54 %.

### Iodure de 3-dodécyl-1-(naphtalèn-1-yl)-2H-imidazol-3-ium (58).

La procédure générale avec **52** (1,056 g ; 2,38 mmol) et de l'iodure de potassium (0,878 g ; 5,29 mmol) permet d'obtenir **58** avec un rendement de 76 % (0,893 g ; 1,82 mmol). RMN ¹H (300 MHz, CDCl₃) : δ= 0,89 (t, 3H, J = 6,30 Hz, C*H*₃ chaîne aliphatique) ; 1,27-1,51 (m large, 18H, C*H*₂ chaîne aliphatique et N-CH₂-CH₂-C*H*₂) ; 2,09 (qui, 2H, J = 7,68 Hz, N-CH₂-C*H*₂) ; 4,73 (t, 2H, J = 7,68 Hz, N-C*H*₂) ; 7,50-7,53 (m, 2H, CH naphtalène ou imidazolium) ; 7,60-7,68 (m, 4H, C*H* naphtalène ou imidazolium) ; 7,89 (d, 1H, J = 7,41 Hz, C*H* naphtalène ou imidazolium) ; 8,02-8,05 (m, 1H, C*H* naphtalène ou imidazolium) ; 8,11 (d, 1H, J = 8,22 Hz, CH naphtalène ou imidazolium) ; 10,43 (s, 1H, N-CH-N). RMN ¹³C (75 MHz, CDCl₃) : δ = 13,92 (C-CH₃ chaîne aliphatique) ; 22,47; 26,11 ; 29,13; 29,25 ; 29,35 ; 29,41 ; 29,43 ; 30,13 ; 31,70 (CH₂ chaîne aliphatique) ; 50,72 (N-CH₂) ; 120,58 ; 123,25 ; 124,06 ; 124,76 ; 125,18 (CH naphtalène ou imidazolium) ; 127,42 (C quaternaire); 127,50; 128,70; 128,73 (CH naphtalène ou imidazolium); 130,34 (C quaternaire) ; 131,48 (CH naphtalène ou imidazolium) ; 133,98 (C quaternaire) ; 136,84 (CH naphtalène ou imidazolium). vₘₐₓ/cm⁻¹ 3054 (C-H aromatique) ; 2929 et 2856 (C-H aliphatique) ; 1422 (C=C aromatique). UV/Vis (CH₂Cl₂) : λₘₐₓ (ε L.mol⁻¹.cm⁻¹) = 283 nm (8100) ; 230 nm (25300). Analyse élémentaire pour C₂₅H₃₅IN₂._{0,3}H₂O ; Calculée : C 60,55 ; H 7,24 ; N 5,65 %. Trouvée : C 60,56 ; H 7,23 ; N 5,55 %.

### Bis(trifluorométhylsulfonyl)amidure de 3-dodécyl-1-(naphtalèn-1-yl)-2H-imidazol-3-ium (59).

La procédure générale avec **52** (1,185 g ; 2,67 mmol) et du bis(trifluorométhylsulfonyl)amidure de lithium (1,229 g ; 4,28 mmol) permet d'obtenir **59** avec un rendement de 74 % (1,274 g ; 1,98 mmol). RMN ¹H (300 MHz, CDCl₃) : δ= 0,89 (t, 3H, J = 6,30 Hz, C*H*₃ chaîne aliphatique); 1,27-1,42 (m large, 18H, C*H*₂ chaîne aliphatique et N-CH₂-CH₂-C*H*₂) ; 2,04 (qui, 2H, J = 7,95 Hz, N-CH₂-C*H*₂) ; 4,47 (t, 2H, J = 7,68 Hz, N-C*H*₂) ; 7,44-7,47 (m, 1H, *CH* naphtalène ou imidazolium) ; 7,57 (d, 1H, J = 8,22 Hz, C*H* naphtalène ou imidazolium); 7,62-7,71 (m, 1H, *CH* naphtalène ou imidazolium) ; 7,76 (d, 1H, J = 7,41 Hz, *CH* naphtalène ou imidazolium) ; 8,03-8,06 (m, 1H, *CH* naphtalène ou imidazolium) ; 8.13 (d, 1H, J = 8,49 Hz, *CH* naphtalène ou imidazolium) ; 9,01 (s, 1H, N-CH-N). RMN ¹³C (75 MHz, CDCl₃) : δ= 14,00 (C-CH₃ chaîne aliphatique) ; 22,59 ; 26,12 ; 28,82 ; 29,25 ; 29,27 ; 29,39 ; 29,51 ; 29,90 ; 31,82 (CH₂ chaîne aliphatique) ; 50,69 (N-CH₂) ; 119,72 (qua, J = 319,65 Hz, (CF₃SO₂)₂N⁻) ; 120,33 ; 123,02; 124,57; 124,63; 125,14 (CH naphtalène ou imidazolium) ; 127,63 (C quaternaire); 127,65; 128,77; 128,89 (CH naphtalène ou imidazolium); 130,35 (C quaternaire) ; 131,75 (CH naphtalène ou imidazolium) ; 134,11 (C quaternaire) ; 136,13 (CH naphtalène ou imidazolium). vₘₐₓ/cm⁻¹ 3055 (C-H aromatique) ; 2930 et 2857 (C-H aliphatique) ; 1422 (C=C aromatique) ; 1350 et 1196 ((CF₃SO₂)₂N⁻). UV/Vis (CH₂Cl₂) : λₘₐₓ (ε L.mol⁻¹.cm⁻¹) = 283 nm (7400) ; 228 nm (33800). Analyse élémentaire pour C₂₇H₃₅F₆N₃O₄S₂ ; Calculée : C 50,38 ; H 5,48 ; N 6,53 %. Trouvée : C 50,56 ; H 5,55 ; N 6,67%.

### Tétrafluoroborate de 3-dodécyl-1-(9H-fluorèn-2-yl)-2H-imidazol-3-ium (60).

La procédure générale avec **53** (18,948 g ; 39,35 mmol) et du tétrafluoroborate de potassium (7,634 g ; 60,63 mmol) permet d'obtenir **60** avec un rendement de 84 % (16,095 g ; 32,95 mmol). RMN ¹H (300 MHz, CDCl₃) : δ = 0,88 (t, 3H, J = 6,33 Hz, C*H*₃ chaîne aliphatique); 1,25-1,35 (m large, 18H, C*H*₂ chaîne aliphatique et N-CH₂-CH₂-CH₂) ; 1,95 (qui, 2H, J = 7,68 Hz, N-CH₂-C*H*₂) ; 3,99 (s, 2H, C*H*₂ fluorène) ; 4,38 (t, 2H, J = 7,68 Hz, N-C*H*₂) ; 7,36-7,45 (m, 3H, *CH* fluorène ou imidazolium) ; 7,56-7,61 (m, 3H, *CH* fluorène ou imidazolium) ; 7,78-7,90 (m, 3H, C*H* fluorène ou imidazolium) ; 9,34 (s, 1H, N-CH-N). RMN ¹³C (75 MHz, CDCl₃) : δ = 14,04 (C-CH₃ chaîne aliphatique) ; 22,62 ; 26,22 ; 28,95 ; 29,27 ; 29,35 ; 29,49 ; 29,55 ; 29,57 ; 30,08 ; 31,85 (CH₂ chaîne aliphatique) ; 36,87 (CH₂ fluorène); 50,48 (N-CH₂) ; 118,65; 120,47; 121,05; 121,39; 123,01; 125,13 ; 127,08 ; 127,89 (CH fluorène ou imidazolium) ; 132,61 (*C* quaternaire) ; 134,03 (CH fluorène ou imidazolium) ; 139,53 ; 143,59 ; 143,61 ; 145,48 (*C* quaternaire). vₘₐₓ/cm⁻¹ 3154 (C-H aromatique) ; 2924 et 2854 (C-H aliphatique) ; 1558 (C=C aromatique) ; 1067 (BF₄⁻). UV/Vis (CH₂Cl₂) : λₘₐₓ (ε L.mol⁻¹.cm⁻¹) = 304 nm (18800) ; 280 nm (21500) ; 229 nm (10200). Analyse élémentaire pour C₂₈H₃₇BF₄N₂ ; Calculée : C 68,86 ; H 7,64 ; N 5,74 %. Trouvée : C 68,67 ; H 7,77 ; N 5,82 %.

### Hexafluorophosphate de 3-dodécyl-1-(9H-fluorèn-2-yl)-2H-imidazol-3-ium (61).

La procédure générale avec **53** (0,898 g ; 1,86 mmol) et de l'hexafluorophosphate de potassium (0,869 g ; 4,72 mmol) permet d'obtenir **61** avec un rendement de 68 % (0,689 g ; 1,26 mmol). RMN ¹H (300 MHz, CDCl₃) : δ = 0,89 (t, 3H, J = 6,30 Hz, C*H*₃ chaîne aliphatique) ; 1,25-1,34 (m large, 18H, C*H*₂ chaîne aliphatique et N-CH₂-CH₂-C*H*₂) ; 1,94 (qui, 2H, J = 7,14 Hz, N-CH₂-C*H*₂) ; 3,97 (s, 2H, C*H*₂ fluorène) ; 4,31 (t, 2H, J = 7,11 Hz, N-C*H*₂) ; 7,36-7,45 (m, 3H, C*H* fluorène ou imidazolium) ; 7,51-7,57 (m, 3H, C*H* fluorène ou imidazolium) ; 7,77-7,89 (m, 3H, C*H* fluorène ou imidazolium) ; 8,98 (s, 1H, N-CH-N). RMN ¹³C (75 MHz, CDCl₃) : δ = 14.04 (C-CH₃ chaîne aliphatique) ; 22,62 ; 26,21 ; 28,91 ; 29,28; 29,33; 29,48; 29,57; 29,91 ; 31,86 (CH₂ chaîne aliphatique); 36,79 (*C*H₂ fluorène) ; 50,54 (N-*C*H₂) ; 118,78; 120,47; 120,53; 121,01 ; 121,53; 123,00; 125,11 ; 127,10 ; 127,93 (*C*H fluorène ou imidazolium) ; 132,53 (C quaternaire) ; 133,50 (CH fluorène ou imidazolium) ; 139,47 ; 143,57 ; 143,71 ; 145,47 (*C* quaternaire). vₘₐₓ/cm⁻¹ 3166 (C-H aromatique) ; 2919 et 2851 (C-H aliphatique) ; 1550 (C=C aromatique) ; 828 (PF₆-) .UV/Vis (CH₂Cl₂) : λₘₐₓ (ε L.mol⁻¹.cm⁻¹) = 304 nm (18700) ; 281 nm (20600) ; 229 nm (10300). Analyse élémentaire pour C₂₈H₃7F₆N₂P._{0,1}H₂O ; Calculée : C 61,33 ; H 6,84 ; N 5,11 %. Trouvée : C 61,30 ; H 6,94 ; N 5,19 %.

### Iodure de 3-dodécyl-1-(9H-fluorèn-2-yl)-2H-imidazol-3-ium (62).

La procédure générale avec 53 (0,750 g ; 1,56 mmol) et de l'iodure de potassium (0,443 g ; 2,67 mmol) permet d'obtenir 62 avec un rendement de 62 % (0,513 g ; 0,97 mmol). RMN ¹H (300 MHz, CDCl₃) : δ= 0,89 (t, 3H, J = 6,57 Hz, C*H*₃ chaîne aliphatique) ; 1,26-1,40 (m large, 18H, C*H*₂ chaîne aliphatique et N-CH₂-CH₂-C*H*₂) ; 2,03 (qui, 2H, J = 7,41 Hz, N-CH₂-C*H*₂) ; 4,04 (s, 2H, C*H*₂ fluorène) ; 4,60 (t, 2H, J = 7,41 Hz, N-C*H*₂) ; 7,37-7,46 (m, 3H, C*H* fluorène ou imidazolium) ; 7,59-7,62 (m, 2H, C*H* fluorène ou imidazolium) ; 7,73 et 7,75 (dd, 1H, J = 2,19 et 8,25 Hz, *C*H fluorène ou imidazolium) ; 7,83 (d, 1H, J = 6,57 Hz, *C*H fluorène ou imidazolium); 7,94 (d, 1H, J = 8,22 Hz, *C*H fluorène ou imidazolium) ; 8.06 (s large, 1H, C*H* fluorène ou imidazolium) ; 10.82 (s, 1H, N-CH-N). RMN ¹³C (75 MHz, CDCl₃) : δ = 13,96 (C-CH₃ chaîne aliphatique) ; 22,53 ; 26,15 ; 28,93 ; 29,18 ; 29,28 ; 29,40 ; 29,46 ; 29,47 ; 30,23 ; 31,75 (CH₂ chaîne aliphatique) ; 36,93 (*C*H₂ fluorène) ; 50,55 (N-CH₂) ; 118,87 ; 120,46 ; 120,68 ; 121,06 ; 121,11 ; 123,03 ; 125,10 ; 127,05; 127,87 (CH fluorène ou imidazolium); 132,40 (*C* quaternaire); 134,74 (CH fluorène ou imidazolium) ; 139,46 ; 143,50 ; 143,61 ; 145,32 (*C* quaternaire). vₘₐₓ/cm⁻¹ 3069 (C-H aromatique) ; 2930 et 2854 (C-H aliphatique) ; 1558 (C=C aromatique). UV/Vis (CH₂Cl₂) : λₘₐₓ (ε L.mol⁻¹.cm⁻¹) = 304 nm (20400) ; 282 nm (22500) ; 230 nm (19300). Analyse élémentaire pour C₂₈H₃₇IN₂ ; Calculée : C 63,63 ; H 7,06 ; N 5,30 %. Trouvée : C 63,64 ; H 7,09; N 5,14 %.

### Bis(trifluorométhylsulfonyl)amidure de 3-dodécyl-1-(9H-fluorèn-2-yl)-2H-imidazol-3-ium (63).

La procédure générale avec **53** (0,330 g ; 0,69 mmol) et du bis(trifluorométhylsulfonyl)amidure de lithium (0,492 g ; 1,71 mmol) permet d'obtenir **63** avec un rendement de 89 % (0,419 g ; 0,61 mmol). RMN ¹H (300 MHz, CDCl₃) : δ = 0,88 (t, 3H, J = 6,33 Hz, C*H*₃ chaîne aliphatique) ; 1,27-1,38 (m large, 18H, C*H*₂ chaîne aliphatique et N-CH₂-CH₂-C*H*₂) ; 1,98 (qui, 2H, J = 7,95 Hz, N-CH₂-C*H*₂) ; 4,04 (s, 2H, C*H*₂ fluorène) ; 4,37 (t, 2H, J = 7,68 Hz, N-C*H*₂) ; 7,39-7,48 (m, 3H, *CH* fluorène ou imidazolium) ; 7,53-7,62 (m, 3H, C*H* fluorène ou imidazolium) ; 7,83 et 7,86 (dd, 2H, J = 1,65 et 9,87 Hz, *CH* fluorène ou imidazolium) ; 7,94 (d, 1H, J = 8,22 Hz, *CH* fluorène ou imidazolium) ; 9,26 (s, 1H, N-CH-N). RMN ¹³C (75 MHz, CDCl₃) : δ= 14,03 (C-CH₃ chaîne aliphatique) ; 22,62 ; 26,13 ; 28,85 ; 29,27 ; 29,42 ; 29,54 ; 30,05 ; 31,85 (CH₂ chaîne aliphatique); 36,88 (CH₂ fluorène); 50,66 (N-*C*H₂) ; 119,87 (qua, J = 319,65 Hz, (CF₃SO₂)₂N⁻) ; 118,90; 120,57; 120,72; 121,21 ; 121,80; 123,07; 125,24; 127,18; 128,07 (CH fluorène ou imidazolium) ; 132,50 (C quaternaire) ; 133,81 (CH fluorène ou imidazolium); 139,56; 143,69; 144,06; 145,67 (C quaternaire). vₘₐₓ/cm⁻¹ 3139 (C-H aromatique) ; 2922 et 2855 (C-H aliphatique) ; 1464 (C=C aromatique) ; 1349 et 1121 ((CF₃SO₂)₂N⁻). UV/Vis (CH₂Cl₂) : λₘₐₓ (ε L.mol⁻¹.cm⁻¹) = 304 nm (19900) ; 281 nm (21800) ; 227 nm (13200). Analyse élémentaire pour C₃₀H₃7FₑN₃O₄S₂ ; Calculée : C 52,85 ; H 5,47 ; N 6,16 %. Trouvée : C 53,01 ; H 5,50 ; N 6,37 %.

### Tétrafluoroborate de 3-dodécyl-1-(9-méthylcarbazol-3-yl)-2H-imidazol-3-ium (64).

La procédure générale avec **54** (0,189 g ; 0,38 mmol) et du tétrafluoroborate de potassium (0,218 g ; 1,73 mmol) permet d'obtenir **64** avec un rendement de 65 % (0,125 g ; 0,25 mmol). RMN ¹H (300 MHz, CDCl₃) : δ = 0,88 (t, 3H, J = 6,30 Hz, C*H*₃ chaîne aliphatique) ; 1,26-1,36 (m large, 18H, C*H*₂ chaîne aliphatique et N-CH₂-CH₂-C*H*₂) ; 1,95 (qui, 2H, J = 7,14 Hz, N-CH₂-C*H*₂) ; 3,82 (s, 3H, C*H*₃ carbazole) ; 4,37 (t, 2H, J = 7,14 Hz, N-C*H*₂) ; 7,30-7,32 (m, 1H, C*H* carbazole ou imidazolium); 7,39-7,48 (m, 3H, C*H* carbazole ou imidazolium) ; 7,54-7,65 (m, 3H, C*H* carbazole ou imidazolium) ; 8,17 (d, 1H, J = 7,68 Hz, C*H* carbazole ou imidazolium) ; 8,27 (s large, 1H, C*H* carbazole ou imidazolium) ; 9,32 (s, 1H, N-C*H*-N). RMN ¹³C (75 MHz, CDCl₃) : δ= 14,04 (C-CH₃ chaîne aliphatique) ; 22,62 ; 26,24 (*C*H₂ chaîne aliphatique) ; 28,91 (*C*H₃ carbazole) ; 28,96 ; 29,28 ; 29,37 ; 29,49 ; 29,56 ; 29,57 ; 30,10 ; 31,85 (*C*H₂ chaîne aliphatique) ; 50,29 (N-*C*H₂); 108,82; 109,42; 113,41 ; 118,54; 119,65; 120,90; 121,60 (*C*H carbazole ou imidazolium) ; 121,73 (*C* quaternaire) ; 122,59 (*C*H carbazole ou imidazolium) ; 122,99 ; 125,99 (*C* quaternaire) ; 126,94 ; 133,78 *(*CH carbazole ou imidazolium) ; 140,49 ; 141,52 (*C* quaternaire). vₘₐₓ/cm⁻¹ 3164 (C-H aromatique) ; 2919 et 2852 (CH aliphatique) ; 1589 (C=C aromatique) ; 1056 (BF₄⁻). UV/Vis (CH₂Cl₂) : λₘₐₓ (ε L.mol⁻¹.cm⁻¹) = 351 nm (2500) ; 334 nm (3500) ; 279 nm (29600) ; 239 nm (29700). Analyse élémentaire pour C₂₈H₃₈BF₄N₃ ; Calculée : C 66,80 ; H 7,61 ; N 8,35 %. TRouvée : C 66,65 ; H 7,78 ; N 8,39 %.

### Hexafluorophosphate de 3-dodécyl-1-(9-méthylcarbazol-3-yl)-2H-imidazol-3-ium (65).

La procédure générale avec **54** (0,185 g ; 0,37 mmol) et de l'hexafluorophosphate de potassium (0,288 g ; 1,56 mmol) permet d'obtenir **65** avec un rendement de 72 % (0,150 g ; 0,27 mmol). RMN ¹H (300 MHz, CDCl₃) : δ = 0,88 (t, 3H, J = 6,33 Hz, C*H*₃ chaîne aliphatique) ; 1,26-1,35 (m large, 18H, C*H*₂ chaîne aliphatique et N-CH₂-CH₂-C*H*₂) ; 1,94 (qui, 2H, J = 6,87 Hz, N-CH₂-C*H*₂) ; 3,80 (s, 3H, C*H*₃ carbazole) ; 4,31 (t, 2H, J = 6,87 Hz, N-C*H*₂) ; 7,39-7,34 (m, 1H, C*H* carbazole ou imidazolium) ; 7,39-7,45 (m, 3H, C*H* carbazole ou imidazolium) ; 7,53-7,58 (m, 3H, C*H* carbazole ou imidazolium) ; 8,14 (d, 1H, J = 7,95 Hz, C*H* carbazole ou imidazolium) ; 8,21 (s large, 1 H, C*H* carbazole ou imidazolium) ; 8,95 (s large, 1H, N-C*H*-N). RMN ¹³C (75 MHz, CDCl₃) : δ = 14,06 (C-*C*H₃ chaîne aliphatique) ; 22,65 ; 26,23 ; 28,93 (*C*H₂ chaîne aliphatique) ; 28,99 (*C*H₃ carbazole) ; 29,30 ; 29,35 ; 29,49 ; 29,59 ; 30,04 (*C*H₂ chaîne aliphatique) ; 50,44 (N-*C*H₂); 108,94; 109,60; 113,58; 118,81; 119,81; 120,81 (*C*H carbazole ou imidazolium) ; 121,73 (*C* quaternaire) ; 121,84 ; 122,54 (*C*H carbazole ou imidazolium) ; 123,13; 125,97 (*C* quaternaire); 127,10; 133,86 (*C*H carbazole ou imidazolium); 140,71; 141,65 (*C* quaternaire). vₘₐₓ/cm⁻¹ 3053 (C-H aromatique) ; 2922 et 2853 (C-H aliphatique) ; 1560 (C=C aromatique) ; 816 (PF₆⁻). UV/Vis (CH₂Cl₂) : λₘₐₓ (ε L.mol⁻¹.cm⁻¹) = 350 nm (2200) ; 334 nm (3200) ; 280 nm (28600) ; 238 nm (29300). Analyse élémentaire pour C₂₈H₃₈F₆N₃P ; Calculée : C 59,88 ; H 6,82 ; N 7,48 %. Trouvée : C 59,83 ; H 6,96 ; N 7,45 %.

### Iodure de 3-dodécyl-1-(9-méthylcarbazol-3-yl)-2H-imidazol-3-ium (66).

La procédure générale avec **54** (0,415 g ; 0,84 mmol) et de l'iodure de potassium (0,416 g ; 2,50 mmol) permet d'obtenir **66** avec un rendement de 56 % (0,256 g ; 0,47 mmol). RMN ¹H (300 MHz, CDCl₃) : δ= 0,88 (t, 3H, J = 6,30 Hz, C*H*₃ chaîne aliphatique) ; 1,26-1,45 (m large, 18H, C*H*₂ chaîne aliphatique et N-CH₂-CH₂-C*H*₂) ; 2,03 (qui, 2H, J = 7,41 Hz, N-CH₂-C*H*₂) ; 3,87 (s, 3H, C*H*₃ carbazole) ; 4,56 (t, 2H, J = 7,41 Hz, N-C*H*₂) ; 7,30-7,34 (m, 1H, C*H* carbazole ou imidazolium); 7,41-7,62 (m, 4H, C*H* carbazole ou imidazolium) ; 7,67 (t, 1H, J = 1,92 Hz, C*H* carbazole ou imidazolium) ; 7,81 et 7,84 (dd, 1H, J = 2,19 et 8,52 Hz, C*H* carbazole ou imidazolium) ; 8,20 (d, 1H, J = 7,68 Hz, C*H* carbazole ou imidazolium) ; 8,43 (d, 1H, J = 2,19 Hz, C*H* carbazole ou imidazolium) ; 10,77 (s, 1H, N-C*H*-N). RMN ¹³C (75 MHz, CDCl₃) : δ = 13,98 (C-CH₃ chaîne aliphatique) ; 22,55 ; 26,19 ; 28,95 ; 29,20 ; 29.30 (*C*H₂ chaîne aliphatique) ; 29,35 (*C*H₃ carbazole) ; 29,42 ; 29,48 ; 29,49 ; 30,26 ; 31,78 (*C*H₂ chaîne aliphatique) ; 50,36 (N-*C*H₂) ; 108,85 ; 109,45; 113,90; 118,73; 119,64; 121,31 ; 121,40 (CH carbazole ou imidazolium); 121,77 (*C* quaternaire) ; 122,54 (*C*H carbazole ou imidazolium); 123,00; 125,90 (*C* quaternaire); 126,96; 134,49 (*C*H carbazole ou imidazolium); 140,45; 141,51 (*C* quaternaire). vₘₐₓ/cm⁻¹ 3040 (C-H aromatique) ; 2919 et 2851 (C-H aliphatique) ; 1559 (C=C aromatique). UV/Vis (CH₂Cl₂) : λₘₐₓ (ε L.mol⁻¹.cm⁻¹) = 352 nm (3700) ; 336 nm (4500) ; 281 nm (32200) ; 239 nm (42900). Analyse élémentaire pour C₂₈H₃₈IN₃ ; Calculée : C 61,87 ; H 7,05; N 7,73 %. Trouvée : C 61,93 ; H 7,12; N 7,66 %.

### Bis(trifluorométhylsulfonyl)amidure de 3-dodécyl-1-(9-méthylcarbazol-3-yl)-2H-imidazol-3-ium (67).

La procédure générale avec **54** (0,367 g ; 0,74 mmol) et du bis(trifluorométhylsulfonyl)amidure de lithium (0,652 g ; 2,27 mmol) permet d'obtenir **67** avec un rendement de 90 % (0,466 g ; 0,67 mmol). RMN ¹H (300 MHz, CDCl₃) : δ = 0,88 (t, 3H, J = 6,84 Hz, C*H*₃ chaîne aliphatique); 1,27-1,38 (m large, 18H, C*H*₂ chaîne aliphatique et N-CH₂-CH₂-C*H*₂) ; 1,98 (qui, 2H, J = 7,68 Hz, N-CH₂-C*H*₂) ; 3,91 (s, 3H, C*H*₃ carbazole) ; 4,38 (t, 2H, J = 7,68 Hz, N-C*H*₂) ; 7,31-7,36 (m, 1H, C*H* carbazole ou imidazolium) ; 7,44-7,49 (m, 2H, C*H* carbazole ou imidazolium) ; 7,53-7,66 (m, 4H, C*H* carbazole ou imidazolium) ; 8,15 (d, 1H, J = 7.68 Hz, C*H* carbazole ou imidazolium) ; 8,25 (s, 1H, C*H* carbazole ou imidazolium) ; 9,16 (s, 1H, N-C*H*-N). RMN ¹³C (75 MHz, CDCl₃) : δ = 14,02 (C-*C*H₃ chaîne aliphatique) ; 22,61 ; 26,15 ; 28,86 (*C*H₂ chaîne aliphatique) ; 29,10 (*C*H₃ carbazole); 29,27; 29,29; 29,43; 29,54; 30,07; 31,84 (*C*H₂ chaîne aliphatique) ; 50,47 (N-*C*H₂) ; 109,02 ; 109,66 ; 113,79 ; 118,98 ; 119,84 (*C*H carbazole ou imidazolium); 119,92 (qua, J = 319,65 Hz, (*C*F₃SO₂)₂N⁻) ; 120,84 (*C*H carbazole ou imidazolium) ; 121,76 (C quaternaire) ; 122,16 ; 122,74 (*C*H carbazole ou imidazolium) ; 123,25; 125,95 (*C* quaternaire); 127,16; 133,51 (*C*H carbazole ou imidazolium); 140,84; 141,73 (*C* quaternaire). vₘₐₓ/cm⁻¹ 3055 (*C*-H aromatique) ; 2930 et 2857 (C-H aliphatique) ; 1423 (C=C aromatique) ; 1350 and 1197 ((CF₃SO₂)₂N⁻). UV/Vis (CH₂Cl₂) : λₘₐₓ (ε L.mol⁻¹.cm⁻¹) = 351 nm (3000) ; 337 nm (4000) ; 281 nm (28100) ; 238 nm (28900). Analyse élémentaire pour C₃₀H₃₈F₆N₄O₄S₂ ; Calculée : C 51,71 ; H 5,50 ; N 8,04 %. Trouvée : C 51,97 ; H 5,60 ; N 8,21 %.

### Tétrafluoroborate de 1-(anthracèn-9-yl)-3-dodécyl-2H-imidazol-3-ium (68).

La procédure générale avec **55** (0,165 g ; 0,33 mmol) et du tétrafluoroborate de potassium (0,253 g ; 2,01 mmol) permet d'obtenir **68** avec un rendement de 88 % (0,147 g ; 0,29 mmol). RMN ¹H (300 MHz, CDCl₃) : δ = 0,89 (t, 3H, J = 6,84 Hz, C*H*₃ chaîne aliphatique) ; 1,27-1,43 (m large, 18H, C*H*₂ chaîne aliphatique et N-CH₂-CH₂-C*H*₂) ; 2,08 (qui, 2H, J = 7,68 Hz, N-CH₂-C*H*₂) ; 4,62 (t, 2H, J = 7,41 Hz, N-C*H*₂) ; 7,37 (d, 2H, J = 8,79 Hz, C*H* anthracène ou imidazolium) ; 7,49 (t, 1H, J = 1,65 Hz, C*H* anthracène ou imidazolium) ; 7,57-7,68 (m, 4H, C*H* anthracène ou imidazolium) ; 7,83 (t, 1H, J = 1,65 Hz, C*H* anthracène ou imidazolium) ; 8,15 (d, 2H, J = 7,68 Hz, C*H* anthracène ou imidazolium) ; 8,75 (s, 1H, C*H* anthracène ou imidazolium) ; 9,02 (s, 1H, N-C*H*-N). RMN ¹³C (75 MHz, CDCl₃) : δ = 14,04 (C-*C*H₃ chaîne aliphatique) ; 22,61 ; 26,15 ; 28,93 ; 29,27 ; 29,40 ; 29,45 ; 29,55 ; 29,57 ; 30,07 ; 31,83 (*C*H₂ chaîne aliphatique) ; 50,66 (N-*C*H₂) ; 120,30 ; 123,74 (*C*H anthracène ou imidazolium) ; 124,49 (C quaternaire) ; 125,49 ; 126,22 (*C*H anthracène ou imidazolium) ; 127,50 (*C* quaternaire) ; 128,70 ; 129,23 (*C*H anthracène ou imidazolium) ; 130,75 (*C* quaternaire) ; 131,07 ; 137,63 (*C*H anthracène ou imidazolium). vₘₐₓ/cm⁻¹ 3061 (C-H aromatique) ; 2921 et 2852 (C-H aliphatique) ; 1545 (C=C aromatique) ; 1140 (BF₄⁻). UV/Vis (CH₂Cl₂) : λₘₐₓ (ε L.mol⁻¹.cm⁻¹) = 387 nm (5400) ; 371 nm (6700) ; 351 nm (5300) ; 308 nm (8500) ; 254 nm (185400). Analyse élémentaire pour C₂₉H₃7BF₄N₂ ; Calculée : C 69,60 ; H 7,45 ; N 5,60 %. Trouvée : C 69,53 ; H 7,43 ; N 5,38 %.

### Hexafluorophosphate de 1-(anthracèn-9-yl)-3-dodécyl-2H-imidazol-3-ium (69).

La procédure générale avec **55** (0,165 g ; 0,33 mmol) et de l'hexafluorophosphate de potassium (0,151 g ; 0,82 mmol) permet d'obtenir **69** avec un rendement de 91 % (0,171 g ; 0,31 mmol). RMN ¹H (300 MHz, CDCl₃) : δ = 0,89 (t, 3H, J = 6,87 Hz, C*H*₃ chaîne aliphatique) ; 1,27-1,42 (m large, 18H, C*H*₂ chaîne aliphatique et N-CH₂-CH₂-C*H*₂) ; 2,07 (qui, 2H, J = 7,41 Hz, N-CH₂-C*H*₂) ; 4,57 (t, 2H, J = 7,41 Hz, N-C*H*₂) ; 7,37 (d, 2H, J = 8,76 Hz, C*H* anthracène ou imidazolium); 7,49 (t, 1H, J = 1,65 Hz, C*H* anthracène ou imidazolium) ; 7,57-7,68 (m, 4H, C*H* anthracène ou imidazolium) ; 7,80 (t, 1H, J = 1,65 Hz, C*H* anthracène ou imidazolium) ; 8,14 (d, 2H, J = 8,22 Hz, C*H* anthracène ou imidazolium) ; 8,69 (s, 1H, N-C*H*-N) ; 8,75 (s, 1H, C*H* anthracène ou imidazolium). RMN ¹³C (75 MHz, CDCl₃) : δ = 14.07 (C-CH₃ chaîne aliphatique) ; 22,63 ; 26,20 ; 28,91 ; 29,30 ; 29,40 ; 29,46 ; 29,58 ; 29,59 ; 29,85 ; 31,86 (*C*H₂ chaîne aliphatique) ; 50,79 (N-*C*H₂) ; 120,25 ; 123,66 (*C*H anthracène ou imidazolium) ; 124,32 (*C* quaternaire) ; 125,61 ; 126,30 (*C*H anthracène ou imidazolium) ; 127,53 (*C* quaternaire) ; 128,71 ; 129,39 (*C*H anthracène ou imidazolium) ; 130,76 (*C* quaternaire) ; 131,21 ; 136,92 (*C*H anthracène ou imidazolium). vₘₐₓ/cm⁻¹ 3061 (C-H aromatique) ; 2925 et 2854 (C-H aliphatique) ; 1549 (C=C aromatique), 845 (PF₆⁻). UV/Vis (CH₂Cl₂) : λₘₐₓ (ε L.mol⁻¹.cm⁻¹) = 387 nm (5500) ; 370 nm (6800) ; 354 nm (5100) ; 254 nm (160900). Analyse élémentaire pour C₂₉H₃₇FₑN₂P ; Calculée : C 62,36 ; H 6,68 ; N 5,02 %. Trouvée : C 62,33 ; H 6,72 ; N 5,01 %.

### Iodure de 1-(anthracèn-9-yl)-3-dodécyl-2H-imidazol-3-ium (70).

La procédure générale avec **55** (0,165 g ; 0,33 mmol) et de l'iodure de potassium (0,225 g ; 1,35 mmol) permet d'obtenir **70** avec un rendement de 91 % (0,164 g ; 0,30 mmol). RMN ¹H (300 MHz, CDCl₃) : δ = 0,89 (t, 3H, J = 6,87 Hz, C*H*₃ chaîne aliphatique) ; 1,28-1,53 (m large, 18H, C*H*₂ chaîne aliphatique et N-CH₂-CH₂-C*H*₂) ; 2,14 (qui, 2H, J = 7,68 Hz, N-CH₂-C*H*₂) ; 4,92 (t, 2H, J = 7,41 Hz, N-C*H*₂) ; 7,42 (d, 2H, J = 8,76 Hz, C*H* anthracène ou imidazolium) ; 7,50 (t, 1H, J = 1,65 Hz, C*H* anthracène ou imidazolium) ; 7,59-7,69 (m, 4H, C*H* anthracène ou imidazolium); 7,95 (t, 1H, J = 1,65 Hz, C*H* anthracène ou imidazolium) ; 8,15-8,18 (m, 2H, C*H* anthracène ou imidazolium) ; 8,77 (s, 1H, C*H* anthracène ou imidazolium) ; 10,05 (s, 1H, N-C*H*-N). RMN ¹³C (75 MHz, CDCl₃) : δ = 13,94 (C-*C*H₃ chaîne aliphatique) ; 22,50; 26,05; 28,92; 29,16; 29,30; 29,35; 29,43 ; 29,46 ; 30,31 ; 31,73 (*C*H₂ chaîne aliphatique) ; 50,89 (N-*C*H₂) ; 120,43 ; 124,19 (*C*H anthracène ou imidazolium); 124,31 (*C* quaternaire) ; 125,21 ; 126,20 (*C*H anthracène ou imidazolium) ; 127,41 (*C* quaternaire) ; 128,70 ; 129,26 (*C*H anthracène ou imidazolium) ; 130,73 (*C* quaternaire) ; 131,07 ; 137,83 (*C*H anthracène ou imidazolium). vₘₐₓ/cm⁻¹ 3044 (C-H aromatique) ; 2920 et 2850 (C-H aliphatique) ; 1544 (C=C aromatique). UV/Vis (CH₂Cl₂) : λₘₐₓ (ε L.mol⁻¹.cm⁻¹) = 387 nm (6700) ; 369 nm (8300) ; 354 nm (6700) ; 289 nm (3500) ; 254 nm (165900). Analyse élémentaire pour C₂₉H₃7IN₂ ; Calculée : C 64,44 ; H 6,90 ; N 5,18 %. Trouvée : C 64,34 ; H 6,89 ; N 5,20 %.

**Bis(trifluorométhylsulfonyl)amidure 1-(anthracèn-9-yl)-3-dodécyl-2H-imidazol-3-ium (71).**

La procédure générale avec **55** (0,171 g ; 0,35 mmol) et du bis(trifluorométhylsulfonyl)amidure de lithium (0,222 g , 0,77 mmol) permet d'obtenir **71** avec un rendement de 91 % (0,219 g ; 0,32 mmol). RMN ¹H (300 MHz, CDCl₃) : δ = 0,89 (t, 3H, J = 6,33 Hz, C*H*₃ chaîne aliphatique) ; 1,28-1,45 (m large, 18H, C*H*₂ chaîne aliphatique et N-CH₂-CH₂-C*H*₂) ; 2,10 (qui, 2H, J = 7,41 Hz, N-CH₂-C*H*₂) ; 4,58 (t, 2H, J = 7,41 Hz, N-C*H*₂) ; 7,36 (d, 2H, J = 8,76 Hz, C*H* anthracène ou imidazolium) ; 7,54 (t, 1H, J = 1,62 Hz, C*H* anthracène ou imidazolium); 7,60-7,70 (m, 4H, C*H* anthracène ou imidazolium) ; 7,83 (t, 1H, J= 1,65 Hz, C*H* anthracène ou imidazolium) ; 8,16-8,19 (m, 2H, C*H* anthracène ou imidazolium) ; 8,78 (s, 1H, C*H* anthracène ou imidazolium) ; 8,90 (s, 1H, N-C*H*-N). RMN ¹³C (75 MHz, CDCl₃) : δ = 14,04 (C-*C*H₃ chaîne aliphatique) ; 22,62 ; 26,15 ; 28,85 ; 29,28 ; 29,35 ; 29,41 ; 29,55 ; 29,97 ; 31,86 (*C*H₂ chaîne aliphatique) ; 50,92 (N-CH₂) ; 119,67 (qua, J = 319,65 Hz, (*C*F₃SO₂)₂N⁻) ; 120,09; 123,70 (*C*H anthracène ou imidazolium) ; 124,27 (*C* quaternaire) ; 125,72 ; 126,37 (*C*H anthracène ou imidazolium) ; 127,61 (*C* quaternaire) ; 128,83 ; 129,47 (*C*H anthracène ou imidazolium) ; 130,87 (*C* quaternaire) ; 131,33 ; 137,39 (*C*H anthracène ou imidazolium). vₘₐₓ/cm⁻¹ 3055 (C-H aromatique) ; 1423 (C=C aromatique) ; 1350 et 1138 (N(SO₂CF₃)₂⁻). UV/Vis (CH₂Cl₂) : λₘₐₓ (ε L.mol⁻¹.cm⁻¹) = 387 nm (4900); 370 nm (6000); 354 nm (4900); 254 nm (156800). Analyse élémentaire pour C₃₁H₃7F₆N₃O₄S₂ ; Calculée : C 53,67 ; H 5,38 ; N 6,06 %. Trouvée : C 53,88 ; H 5,48 ; N 6,09 %.

### Bromure de 1-(9H-fluorèn-2-yl)-3-Hexyl-1H-imidazol-3-ium (72)

Dans un ballon, on introduit le 1-(9*H*-fluorèn-2-yl)-1*H*-imidazole (2,054 g, 8,843 mmol) et le 1-bromohexane (8 mL). Le mélange réactionnel est chauffé à 156 °C pendant 48 heures. On récupère le produit par précipitation dans l'éther, suivi d'un séchage sous pression réduite à température ambiante pendant 24 heures. C'est une poudre blanche légèrement colorée (2g, 57%).

Point de fusion (Pf) : Pf = 206.4 ± 1 °C.

Les expériences de RMN ont été réalisées dans le CDCI₃. (¹H RMN: 6 mg/0.3 mL CDCl₃): ¹H RMN (300 MHz, CDCl₃, 20°C): δ 11.10 ppm (s, 1H, =CH- aromatique), 8.01 (s, 1H, =CH- aromatique), 7.87 (d, 1H, =CH- aromatique, ³J = 8.07 Hz), 7.76 (m, 3H, =CH-aromatique), 7.55 (m, 2H, =CH- aromatique), 7.39(m, 2H, =CH- aromatique), 4.59 (t, 2H, N-CH₂-CH₂-(CH₂)₃-CH₃, ³J = 7.35 Hz), 3.97 (s, 2H, N-CH₂-), 1.99 (m, 2H, N-CH₂-CH₂-(CH₂)₃-CH₃), 1.35 (m, 6H, N-CH₂-CH₂-(CH₂)₃-CH₃), 0.88 (t, 3H, N-CH₂-CH₂-(CH₂)₃-CH₃, ³J = 6.78 Hz).

### Tétrafluoroborate de 1-(9H-fluorèn-2-yl)-3-Hexyl-1H-imidazol-3-ium (73)

Dans un ballon, on introduit du bromure de 1-(9H-fluorèn-2-yl)-3-Hexyl-1H-imidazol-3-ium (102 mg, 0,257 mmol) qu'on dissout dans un mélange eau/éthanol (5 mL/4 mL) et on lui rajoute du tétrafluoroborate de sodium (51 mg , 0,465 mmol). Le mélange est agité à température ambiante pendant 2 jours, puis le mélange réactionnel est extrait au dichlorométhane, séché et évaporé sous vide à pression réduite à température ambiante pendant 12 heures. On récupère un solide légèrement jaunâtre (93 mg, rendement: 90%).

Point de fusion (Pf) : Pf = 112.6 ± 1 °C.

Le produit est caractérisé par spectroscopie RMN.

¹H RMN (300 MHz, CDCl₃, 20°C): δ 9.28 ppm (s, 1H, =CH- aromatique), 7.77 (m, 3H, =CH- aromatique), 7.63 (s, 1H), 7.53 (m, 2H, =CH- aromatique), 7.38 (m, 3H, =CH-aromatique), 4.32 (t, 2H, N-CH₂-CH₂-(CH₂)₃-CH₃, ³J = 7.35 Hz), 3.91 (s, 2H, N-CH₂-), 1.91 (m, 2H, N-CH₂-CH₂-(CH₂)₃-CH₃), 1.31 (m, 6H, N-CH₂-CH₂-(CH₂)₃-CH₃), 0.87 (t, 3H, N-CH₂-CH₂-(CH₂)₃-CHₛ, ³J = 6.8 Hz).

13C {1H} RMN (75 MHz, CDCl₃, 20 °C): δ 13.84 (N-(CH2)5-CH3) ppm, 22.31 (N-(CH2)4-CH2-CH3), 25.82 (N-(CH2)3-CH2-CH2-CH3), 30.02 (N-(CH2)2-CH2-(CH2)2-CH3), 30.98 (N-CH2-CH2-(CH2)3-CH3), 36.88 (-CH2-Fluorene), 50.46 (N-CH2-CH2-(CH2)3-CH3), 118.66 (C aromatique), 120.48 (C aromatique), 121.07 (C aromatique), 121.36 (C aromatique), 123.00 (C aromatique), 125.13 (C aromatique), 127.09 (C aromatique), 127.90 (C aromatique), 132.60 (C aromatique), 134.08 (C aromatique), 139.52 (C aromatique), 143.59 (C aromatique), 145.47 (C aromatique).

## Revendications

1. Composé imidazolium de formule suivante : 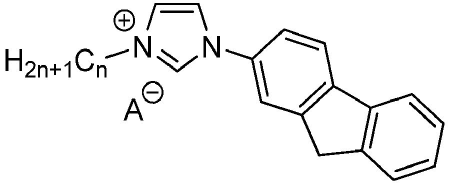
dans lequel n est un nombre allant de 5 à 20 ;dans lequel ledit composé est associé à un contre-anion « A⁻», A⁻ étant choisi parmi un halogénure, P(R ⁴)₆⁻, B(R⁴)₄⁻, SCN⁻, (R⁵SO₂)₂N⁻, R⁵OSO₃, R⁵SO₃⁻ , carborane, carbonate, hydrogénocarbonate, alcoolate, carboxylate, amidure, phosphate, SiF₆⁻, SbF₆⁻, I₃-, nitrate, oxyde d'halogénure, silicate, sulfate, sulfonate, cyanure, carbanions, ou métallate,
dans laquelle :
R⁴ est, à chaque occurrence, un groupe indépendamment choisi parmi un atome d'halogène, un groupe alkyle en C₁-C₆, un groupe aryle en C₆-C₁₀, un groupe aralkyle ;
R⁵ est, à chaque occurrence, un groupe indépendamment choisi parmi alkyle en C₁-C₂₀, halogénoalkyle en C₁-C₂₀, aryle en C₆-C₁₀, aralkyle.

2. Composé selon la revendication 1, **caractérisé en ce que** A⁻ est choisi parmi Br⁻, I⁻, PF₆⁻, BF₄⁻, (CF₃SO₂)₂N⁻, C₁₂H₂₅OSO₃⁻, C₁₆H₃₃OSO₃⁻, CF₃SO₃⁻.

3. Composé selon la revendication 1, **caractérisé en ce que** le composé est le tétrafluoroborate de 3-dodécyl-1-(9*H*-fluorèn-2-yl)-2*H*-imidazol-3-ium: 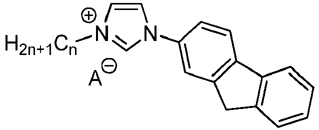 où n vaut 12 et A⁻ représente le tétrafluoroborate.

4. Composé selon la revendication 1, **caractérisé en ce que** le composé est le tétrafluoroborate de 1-(9H-fluorèn-2-yl)-3-octyl-2H-imidazol-3-ium: 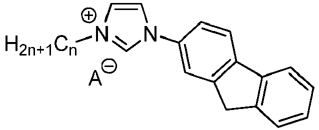 où n vaut 8 et A⁻ représente le tétrafluoroborate.

5. Matériau solide fluorophore, comprenant ou constitué d'un composé fluorophore, ledit composé fluorophore étant tel que défini selon l'une quelconque des revendications 1 à 4.

6. Matériau liquide fluorophore, comprenant ou constitué d'un composé fluorophore, ledit composé fluorophore étant tel que défini selon l'une quelconque des revendications 1 à 5.

7. Matériau selon la revendication 6, **caractérisé en ce que** le matériau est un matériau plastique transparent.

8. Utilisation d'un composé fluorophore tel que défini selon l'une quelconque des revendications 1 à 4, ou d'un matériau tel que défini selon l'une quelconque des revendications 5 à 7, pour la détection des rayonnements gamma, X, neutron, proton.

9. Utilisation selon la revendication 8, pour la discrimination des rayonnements proton/gamma, proton/X, neutron/gamma, neutron/X, alpha/gamma, alpha/X.

10. Utilisation selon la revendication 8, pour la détection et/ou l'identification des neutrons rapides et/ou lents.

11. Utilisation d'un composé fluorophore tel que défini selon l'une quelconque des revendications 1 à 4, ou d'un matériau tel que défini selon l'une quelconque des revendications 5 à 7, pour la fabrication d'un instrument de radiodétection, de dosimétrie industrielle ou médicale.

12. Procédé de préparation d'un composé tel que défini selon l'une quelconque des revendications 1 à 4, ledit procédé comprenant une réaction de couplage d'un groupe aromatique à un groupe imidazole par création d'une liaison covalente entre un atome de carbone sp² du groupe aromatique et un atome d'azote du groupe imidazole, en présence d'une zéolite NaY comportant du cuivre (II) et une base, de préférence par couplage de type Ullmann.

13. Procédé selon la revendication 12, **caractérisé en ce que** ledit procédé comprend :
(i) une réaction de couplage d'un groupe fluorène à un groupe imidazole par création d'une liaison covalente entre un atome de carbone sp² du groupe aromatique et un atome d'azote du groupe imidazole, en présence d'une zéolite NaY comportant du cuivre (II) et une base, de préférence par couplage de type Ullmann ; et
(ii) une réaction de couplage d'un groupe alkyle CnH2n+1, où n est un nombre allant de 5 à 20, avec un groupe imidazole par création d'une liaison covalente entre l'atome de carbone sp³ du groupe alkyle CnH2n+1 où n est un nombre allant de 5 à 20 et un atome d'azote du groupe imidazole, de préférence par substitution nucléophile, de préférence en utilisant un groupe alkyle CnH2n+1 où n est un nombre allant de 5 à 20 réactif halogéné.

## Patentansprüche

1. Imidazolium-Zusammensetzung von folgender Formel: 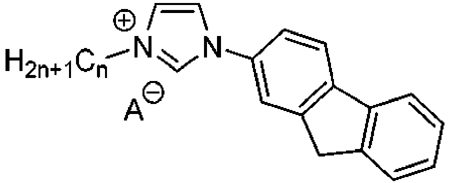
wobei n eine Zahl von 5 bis 20 ist; wobei die Zusammensetzung mit einem Gegenanion "A" assoziiert ist, wobei A ausgewählt ist aus Halogenid, P(R⁴)₆⁻, B(R⁴)₄⁻, SCN⁻, (R⁵SO²)₂N⁻, R⁵OSO₃, R⁵SO₃⁻, Carboran, Carbonat, Hydrogencarbonat, Alkoholat, Carboxylat, Amid, Phosphat, SiF₆⁻, SbF₆⁻, l₃-, Nitrat, Halogenidoxid, Silikat, Sulfat, Sulfonat, Cyanid, Carbanionen oder Metallat,
wobei:
R⁴ bei jedem Vorkommen eine Gruppe ist, unabhängig ausgewählt aus einem Halogenatom, einer C₁-C₆-Alkylgruppe, einer C₆-C₁₀-Arylgruppe, einer Aralkylgruppe;
R⁵ bei jedem Vorkommen eine Gruppe ist, unabhängig ausgewählt aus C₁-C₂₀-Alkyl, C₁-C₂₀-Halogenalkyl, C₆-C₁₀-Aryl, Aralkyl.

2. Zusammensetzung nach Anspruch 1, **dadurch gekennzeichnet, dass** A⁻ausgewählt ist aus Br, I⁻, PF₆⁻, BF₄⁻, (CF₃SO₂)₂N⁻, C₁₂H₂₅OSO₃⁻, C₁₆H₃₃OSO₃, CF₃SO₃⁻.

3. Zusammensetzung nach Anspruch 1, **dadurch gekennzeichnet, dass** die Zusammensetzung 3-Dodecyl-1-(9H-fluoren-2-yl)-2H-imidazol-3-ium ist: 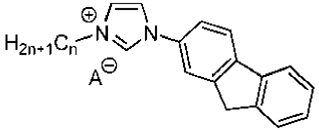 wobei n 12 ist und A⁻ das Tetrafluorborat darstellt.

4. Zusammensetzung nach Anspruch 1, **dadurch gekennzeichnet, dass** die Zusammensetzung 1 -(9H-Fluoren-2-yl)-3-octyl-2H-imidazol-3-ium ist: 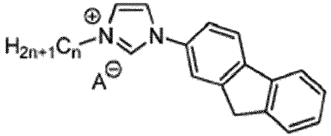 wobei n 8 ist und A⁻ das Tetrafluorborat darstellt.

5. Festes fluorophores Material, umfassend oder bestehend aus einer fluorophoren Zusammensetzung, wobei die fluorophore Zusammensetzung wie definiert nach einem der Ansprüche 1 bis 4 ist.

6. Flüssiges Fluorophormaterial, umfassend oder bestehend aus einer Fluorophorverbindung, wobei die Fluorophorverbindung wie definiert nach einem der Ansprüche 1 bis 5 ist.

7. Material nach Anspruch 6, **dadurch gekennzeichnet, dass** das Material ein transparentes Kunststoffmaterial ist.

8. Verwendung einer fluorophoren Zusammensetzung, wie definiert nach einem der Ansprüche 1 bis 4 ist, oder eines Materials, wie definiert nach einem der Ansprüche 5 bis 7, zur Detektion von Gamma-, X-, Neutronen- und Protonenstrahlung.

9. Verwendung nach Anspruch 8 zur Unterscheidung von Proton/Gamma-, Proton/X-, Neutron/Gamma-, Neutron/X-, Alpha/Gamma-, Alpha/X-Strahlung.

10. Verwendung nach Anspruch 8 zur Detektion und/oder zur Identifizierung von schnellen und/oder langsamen Neutronen.

11. Verwendung einer fluorophoren Zusammensetzung, wie definiert nach einem der Ansprüche 1 bis 4, oder eines Materials, wie definiert nach einem der Ansprüche 5 bis 7, zur Herstellung eines Instruments für Srahlendetektion, industrielle oder medizinische Dosimetrie.

12. Verfahren zur Herstellung einer Zusammensetzung wie definiert in einem der Ansprüche 1 bis 4, das Verfahren umfassend eine Kupplungsreaktion einer aromatischen Gruppe an eine Imidazolgruppe durch Bilden einer kovalenten Bindung zwischen einem sp²-Kohlenstoffatom der aromatischen Gruppe und einem Stickstoffatom der Imidazolgruppe in Anwesenheit eines NaY-Zeoliths mit Kupfer (II) und einer Base, vorzugsweise durch eine Kupplung vom Typ Ullmann.

13. Verfahren gemäß Anspruch 12, **dadurch gekennzeichnet, dass** das Verfahren Folgendes umfasst:
(i) eine Kupplungsreaktion einer Fluorengruppe an eine Imidazolgruppe durch Bilden einer kovalenten Bindung zwischen einem sp²-Kohlenstoffatom der aromatischen Gruppe und einem Stickstoffatom der Imidazolgruppe in Anwesenheit eines NaY-Zeoliths, umfassend Kupfer (II) und eine Base, vorzugsweise durch eine Kupplung vom Typ Ullmann; und
(ii) eine Kupplungsreaktion einer CnH2n+1-Alkylgruppe, wobei n eine Zahl von 5 bis 20 ist, mit einer Imidazolgruppe durch Erzeugen einer kovalenten Bindung zwischen dem sp³-Kohlenstoffatom der CnH2n+1-Alkylgruppe, wobei n eine Zahl von 5 bis 20 ist, und einem Stickstoffatom der Imidazolgruppe, vorzugsweise durch nukleophile Substitution, vorzugsweise unter Verwendung einer CnH2n+1 -Alkylgruppe, wobei n eine Zahl von 5 bis 20 ist, halogenierte Reaktanten, durchgeführt wird.

## Claims

1. Imidazolium compound of formula : 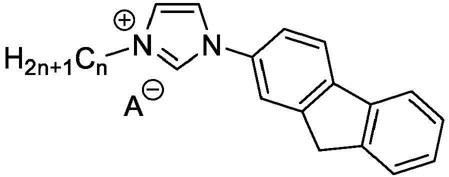
wherein said compound is associated with an « A-» counter-ion, A being chosen from a halide, P(R₄)₆⁻, B(R⁴)₄⁻, SCN⁻, (R₅SO₂)₂N⁻, R⁵OSO₃, R⁵SO₃⁻, carborane, carbonate, hydrogen carbonate, alcoholate, carboxylate, amide, phosphate, SiF₆⁻, SbF₆⁻, I₃-, nitrate, halide oxide, silicate, sulfate, sulfonate, cyanide, carbanions or metallate,
wherein:
R⁴ on each occurrence is a group independently selected from among a halogen atom, C₁-C₆ alkyl group, C₆-C₁₀ aryl group, an aralkyl group;
R⁵ on each occurrence is a group independently selected from among C₁-C₂₀ alkyl, C₁-C₂₀ haloalkyl, C₆-C₁₀ aryl, aralkyl.

2. The compound according to claim 1, wherein A⁻ is selected from among Br⁻, I⁻, PF₆⁻, BF₄⁻, (CF₃SO₂)₂N⁻, C₁₂H₂₅OSO₃⁻, C₁₆H₃₃OSO₃⁻, CF₃SO₃⁻.

3. The compound according to claim 1, **characterized in that** the coumpound is 3-dodecyl-1-(9*H*-fluoren-2-yl)-2*H*-imidazol-3-ium tetrafluoroborate: 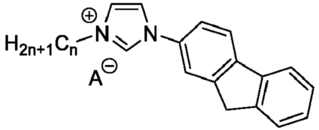 wherein n is equal to 12 and A⁻ is tetrafluoroborate.

4. The compound according to claim 1, **characterized in that** the coumpound is 1-(9*H*-fluoren-2-yl)-3-octyl-2*H*-imidazol-3-ium tetrafluoroborate: 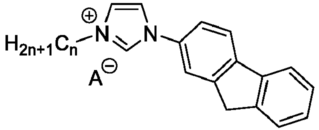 wherein n is equal to 8 and A⁻ is tetrafluoroborate.

5. A fluorophore solid material comprising or consisting of a fluorophore compound, the said fluorophore compound such as defined according to any of claims 1 to 4.

6. A fluorophore liquid material comprising or consisting of a fluorophore compound, the said fluorophore compound such as defined according to any of claims 1 to 5.

7. The material according to claim6, **characterized in that** the material is a transparent plastic material.

8. The use of a fluorophore compound as defined in according to any of claims 1 to 4, or of a material as defined in any of claims 5 to 7, for the detection of gamma, X, neutron, proton radiation.

9. The use according to claim 8 for discrimination between proton/gamma, proton/X, neutron/gamma, neutron/X, alpha/gamma, alpha/X radiation.

10. The use according to claim 8, for the detection and/or identification of fast and/or slow neutrons

11. The use of a fluorophore compound as defined according to any of claims 1 to 4, or of a material such as defined according to any of claims 5 to 7, to manufacture an instrument for radiation detection, industrial or medical dosimetry.

12. A method to prepare a compound as defined according to any of claims 1 to 4, the said method comprising a coupling reaction of an aromatic group with an imidazole group by creating a covalent bond between a sp² carbon atom of the aromatic group and a nitrogen atom of the imidazole group in the presence of a zeolite NaY containing copper(ll) and a base, preferably via coupling of Ullmann-type.

13. The method according to claim 12, **characterized in that** said method comprises:
(i) a coupling reaction between a fluorene group and an imidazole group by creating a covalent bond between a sp² carbon atom of the aromatic group and a nitrogen atom of the imidazole group, in the presence of a zeolite NaY containing copper(II) and a base, preferably coupling of Ullmann type; and
(ii) a coupling reaction of a CnH2n+1 alkyl group, wherein n is a number ranging from 5 to 20, with an imidazole group by creating a covalent bond between the sp³ carbon atom of the CnH2n+1 alkyl group, wherein n is a number ranging from 5 to 20, and a nitrogen atom of the imidazole group, preferably via nucleophilic substitution, preferably using a reactive halogen-containing CnH2n+1 alkyl group, wherein n is a number ranging from 5 to 20.
